(19) Europäisches Patentamt
European Patent Office
Office européen des brevets

(11) Veröffentlichungsnummer: **0 289 913 B1**

(12) **EUROPÄISCHE PATENTSCHRIFT**

(45) Veröffentlichungstag der Patentschrift: **13.01.93**

(51) Int. Cl.5: **C07D 317/28**, C07D 307/14, C07D 327/04, A01N 43/08, A01N 43/28

(21) Anmeldenummer: **88106661.7**

(22) Anmeldetag: **26.04.88**

(54) **Schädlingsbekämpfungsmittel auf Basis von substituierten Aminomethylheterocyclen.**

(30) Priorität: **08.05.87 DE 3715482**

(43) Veröffentlichungstag der Anmeldung:
**09.11.88 Patentblatt 88/45**

(45) Bekanntmachung des Hinweises auf die Patenterteilung:
**13.01.93 Patentblatt 93/02**

(84) Benannte Vertragsstaaten:
**BE CH DE FR GB IT LI NL SE**

(56) Entgegenhaltungen:
**EP-A- 0 005 142**
**EP-A- 0 097 822**
**EP-A- 0 131 793**
**EP-A- 0 158 922**

**TETRAHEDRON, Band 33, November 1977, Seiten 1309-1319, Oxford, GB; M. BARRELLE et al.: "Aminomercuration intramoleculaire d'aziridines cyclooctheniques et de leurs aminoalcools precurseurs"**

(73) Patentinhaber: **BAYER AG**

**W-5090 Leverkusen 1 Bayerwerk(DE)**

(72) Erfinder: **Krämer, Wolfgang, Dr.**
**Rosenkranz 25**
**W-5093 Burscheid 2(DE)**
Erfinder: **Weissmüller, Joachim, Dr.**
**Carl-Langhans-Strasse 53**
**W-4019 Monheim(DE)**
Erfinder: **Holmwood, Graham, Dr.**
**Krutscheider Weg 105**
**W-5600 Wuppertal 1(DE)**
Erfinder: **Berg, Dieter, Dr.**
**Gellertweg 27**
**W-5600 Wuppertal 1(DE)**
Erfinder: **Dutzmann, Stefan, Dr.**
**Leinenweberweg 33**
**W-4000 Düsseldorf 31(DE)**
Erfinder: **Brandes, Wilhelm, Dr.**
**Eichendorfstrasse 3**
**W-5653 Leichlingen 1(DE)**

**EP 0 289 913 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EUROPEAN JOURNAL OF MEDICINAL CHEMISTRY-CHIMIE THERAPEUTIOUE, Band 19, Juni 1984, Seiten 495-500, Châtenay-Malabry, FR; P. ANGELI et al.: "Size of muscarinic receptor anionic binding site related to onium group of ligands with a 1,3-oxathiolane nucleus"**

**CANADIAN JOURNAL OF CHEMISTRY, Band 61, Juli 1983, Seiten 1383-1386, Ottawa, CA; P.C. BELANGER et al.: "Synthesis of optically active 2-tetrahydrofuran derivatives"**

Erfinder: **Hänssler, Gerd, Dr.**
**Am Arenzberg 58a**
**W-5090 Leverkusen 3(DE)**
Erfinder: **Reinecke, Paul, Dr.**
**Steinstrasse 8**
**W-5090 Leverkusen 3(DE)**

## Beschreibung

Die Erfindung betrifft die Verwendung von teilweise bekannten substituierten Aminomethylheterocyclen als Schädlingsbekämpfungsmittel.

Es ist bereits bekannt, daß bestimmte Aminomethylheterocyclen, wie unter anderen beispielsweise das TetrahydroN,5-dimethyl-furfurylamin oder das 2,2-Dimethyl-N-isopropyl-1,3-dioxolan-4-methanamin oder das N,2-Dimethyl-1,3-oxathiolan-5-methanamin bzw. dessen Hydrochlorid oder das cis-N-t-Butyl-5-methylfurfurylamin oder das N-Cyclohexyl-2-methyl-2-phenyl-1,3-dioxolan-4-methanamin bestimmte pharmakologische Eigenschaften, wie beispielsweise antihypertensive, saluretische, parasympatholytische oder muscarinerge Eigenschaften besitzen oder als Zwischenprodukte zur Herstellung von pharmakologisch aktiven Verbindungen verwendet werden (vgl. unter anderem beispielsweise GB 1 031 916; DE-OS 2 810 732, DE-OS 2 757 922; Tetrahedron 33, 1309-1319 [1977]; Acta Pol. Pharm. 39, 33-39 [1982] oder Europ. J. Med. Chem./Chim. Ther. 19, 495-500 [1984]).

Über eine Wirksamkeit dieser vorbekannten Verbindungen als Schädlingsbekämpfungsmittel ist bisher nichts bekannt.

Weiterhin ist bekannt, daß bestimmte Aminoketale, wie beispielsweise das 2-[3-(4-Chlorphenoxy)-2-methyl-prop-2-yl)-2-methyl-4-[N,N-(di-n-butyl)-aminomethyl]-dioxolan oder das 2-[3-(4-Methyl-phenoxy)-2-methyl-prop-2-yl]-2-methyl-4-[N,N-(di-n-butyl)-aminomethyl]-dioxolan oder das 2-[3-(4-Chlorphenoxy)-2-methyl-prop-2-yl]-2-methyl-4-[N-methyl-N-(3-methyl-but-2-en-1-yl)-aminomethyl]-dioxolan gute fungizide Eigenschaften besitzen (vgl. EP 97 822).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurde gefunden, daß die teilweise bekannten substituierten Aminomethylheterocyclen der allgemeinen Formel (I),

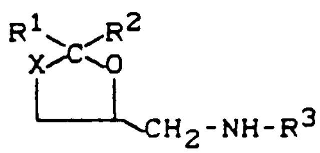

(I)

in welcher

- $R^1$ für Wasserstoff, für Alkyl, für Alkenyl, für jeweils gegebenenfalls substituiertes Tetrahydronaphthyl, Decahydronaphthyl, Cycloalkyl oder Cycloalkenyl steht, ferner für durch Cycloalkyl, Cycloalkenyl, Cycloalkyloxy oder Cycloalkylthio substituiertes Alkyl steht, wobei die cyclischen Reste gegebenenfalls substituiert sein können, ferner für gegebenenfalls substituiertes Aryl steht, weiterhin für durch Aryl, Aryloxy, Arylthio, Arylsulfinyl oder Arylsulfonyl substituiertes Alkyl oder für durch Aryl substituiertes Alkenyl steht, wobei jeweils die Arylreste gegebenenfalls substituiert sein können;
- $R^2$ für Wasserstoff oder Methyl steht,
- $R^3$ für Alkyl, Alkenyl, Alkinyl, Alkoxyalkyl, Hydroxyalkyl, Hydroxyalkoxyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und
- X für Sauerstoff, Schwefel oder eine $CH_2$-Gruppe steht,

sowie deren Säureadditionssalze eine gute Wirkung gegen Schädlinge, insbesondere gegen pilzliche Schädlinge besitzen.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Überraschenderweise zeigen die erfindungsgemäß verwendbaren substituierten Aminomethylheterocyclen der allgemeinen Formel (I) eine bessere Wirksamkeit gegen pilzliche Schädlinge als die aus dem Stand der Technik bekannten Aminoketale, wie beispielsweise das 2-[3-(4-Chlorphenoxy)-2-methyl-prop-2-yl)-2-methyl-4-[N,N-(di-n-butyl)-aminomethyl)-dioxolan oder das 2-[3-(4-Methylphenoxy)-2-methyl-prop-2-yl)-2-methyl-4-[N,N-(di-n-butyl)-aminomethyl]-dioxolan oder das 2-[3-(4-Chlorphenoxy)-2-methyl-prop-2-yl]-2-methyl-4-[N-methyl-N-(3-methyl-but-2-en-1-yl)-aminomethyl]-dioxolan, welches chemisch und wirkungsmäßig naheliegende Verbindungen sind.

Die erfindungsgemäß verwendbaren substituierten Aminomethylheterocyclen sind durch die Formel (I) allgemein definiert. Bevorzugt verwendbar sind Verbindungen der Formel (I), bei welchen

$R^1$ für Wasserstoff; für jeweils geradkettiges oder verzweigtes Alkyl mit 1 bis 18 Kohlenstoffatomen oder Alkenyl mit 3 bis 12 Kohlenstoffatomen steht; außerdem für jeweils gegebenenfalls einfach bis mehrfach, gleich oder verschieden substituiertes Tetrahydronaphthyl, Decahydronaphthyl, Cycloalkyl, Cycloalkenyl, Cycloalkylalkyl, Cycloalkenylalkyl, Cycloalkyloxyalkyl oder Cycloalkylthioalkyl mit jeweils gegebenenfalls 5 bis 7 Kohlenstoffatomen in den Cycloalkyl- bzw. Cycloalkenylteilen und gegebenenfalls 1 bis 6 Kohlenstoffatomen in den geradkettigen oder verzweigten Alkylteilen steht, wobei als Substituenten jeweils infrage kommen:
jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen; und schließlich für jeweils gegebenenfalls einfach bis mehrfach, gleich oder verschieden substituiertes Aryl, Aralkyl, Aryloxyalkyl, Arylthioalkyl, Arylsulfinylalkyl, Arylsulfonylalkyl oder Arylalkenyl mit jeweils 6 bis 10 Kohlenstoffatomen im Arylteil und gegebenenfalls bis zu 6 Kohlenstoffatomen in den geradkettigen oder verzweigten Alkyl- bzw. Alkenylteilen steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Cyclohexyl oder Phenyl;

$R^2$ für Wasserstoff oder Methyl steht,

$R^3$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen; oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
und außerdem für jeweils gegebenenfalls einfach bis mehrfach, gleich oder verschieden im Arylteil substituiertes Aryl, Arylalkyl oder Arylalkenyl mit jeweils 6 bis 10 Kohlenstoffatomen im Arylteil und gegebenenfalls bis zu 6 Kohlenstoffatomen im jeweils geradkettigen oder verzweigten Alkyl- bzw. Alkenylteil steht, wobei als Arylsubstituenten jeweils infrage kommen:
Halogen, Cyano, Nitro sowie jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder für jeweils geradkettiges oder verzweigtes Furanylalkyl, Tetrahydrofuranylalkyl, Tetrahydropyranylalkyl, Dioxolanylalkyl oder Dioxanylalkyl mit jeweils 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen und

X für Sauerstoff, Schwefel oder eine $CH_2$-Gruppe steht.

Besonders bevorzugt erfindungsgemäß verwendbar sind Verbindungen der Formel (I), bei welchen

$R^1$ für Wasserstoff; für jeweils geradkettiges oder verzweigtes Alkyl mit 1 bis 12 Kohlenstoffatomen oder Alkenyl mit 3 bis 8 Kohlenstoffatomen oder für jeweils gegebenenfalls ein- bis dreifach, gleich oder verschieden durch geradkettiges oder verzweigtes Alkyl mit 1 bis 4 Kohlenstoffatomen substituiertes Tetrahydronaphthyl, Decahydronaphthyl, Cyclohexyl oder Cyclohexenyl steht; außerdem für einen Rest

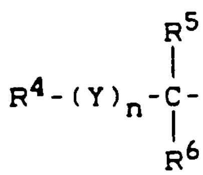

oder für einen Rest

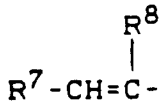

steht, wobei

$R^4$ für jeweils gegebenenfalls ein- bis dreifach, gleich oder verschieden substituiertes Cyclohexyl, Cyclohexenyl oder Phenyl steht, wobei als Cyclohexyl- bzw. Cyclohexenylsubstituenten infrage kommen: jeweils geradkettiges oder verzweigtes Alkyl, Alkoxy oder Halogenalkyl mit jeweils 1 bis 4 Kohlenstoffatomen und gegebenenfalls 1 bis 9 gleichen oder verschiedenen Halogenatomen und wobei als Phenylsubstituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, Cyclohexyl oder Phenyl,

Y für Sauerstoff, Schwefel, eine $-CH_2$-Gruppe, eine $-O-CH_2$-Gruppe, eine $-S-CH_2$-Gruppe, eine Sulfinylgruppe, eine Sulfonylgruppe, eine

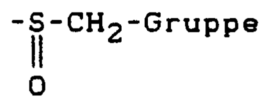

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff oder Methyl steht,

$R^7$ für gegebenenfalls ein- bis dreifach, gleich oder verschieden substituiertes Phenyl steht, wobei als Phenylsubstituenten die bei $R^4$ genannten infrage kommen und

$R^8$ für Methyl oder Ethyl steht,

$R^1$ außerdem für jeweils gegebenenfalls ein- bis dreifach, gleich oder verschieden substituiertes Phenyl oder Naphthyl steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, Cyclohexyl oder Phenyl;

$R^2$ für Wasserstoff oder Methyl steht,

$R^3$ für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für jeweils gegebenenfalls ein- bis fünffach, gleich oder verschieden durch Fluor, Chlor, Brom, Methyl, Ethyl, n- oder i-Propyl, n-, i-, s- oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl steht oder für jeweils gegebenenfalls ein- bis dreifach, gleich oder verschieden substituiertes Phenyl, Benzyl oder Phenylethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl; oder für Furanylmethyl, Tetrahydrofuranylmethyl, Tetrahydropyranylmethyl, Dioxolanylmethyl, Dioxolanylethyl, Dioxanylmethyl oder Dioxanylethyl steht und

X für Sauerstoff, Schwefel oder eine $CH_2$-Gruppe steht.

Ganz besonders bevorzugt erfindungsgemäß verwendbar sind Verbindungen der Formel (I), bei welcher

$R^1$ für jeweils gegebenenfalls ein- bis dreifach, gleich oder verschieden durch Methyl oder t-Butyl substituiertes Tetrahydronaphthyl, Decahydronaphthyl, Cyclohexyl oder Cyclohexenyl steht, oder für einen Rest

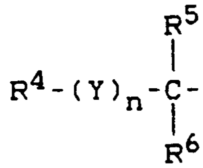

oder einen Rest

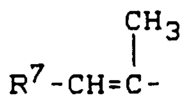

steht, wobei

- $R^4$ für jeweils gegebenenfalls ein- bis dreifach, gleich oder verschieden durch Methyl, i-Propyl, t-Butyl, Methoxy, Propoxy, Butoxy oder Trifluormethyl substituiertes Cyclohexyl oder Cyclohexenyl steht oder für gegebenenfalls ein- bis dreifach, gleich oder verschieden durch Fluor, Chlor, Brom, Methyl, i-Propyl, t-Butyl, Trifluormethyl, Trifluormethoxy, Trifluormethylthio, Methoxy, Phenyl oder Cyclohexyl substituiertes Phenyl steht,
- Y für Sauerstoff, Schwefel, eine $-CH_2$-Gruppe, eine $-O-CH_2$-Gruppe oder eine $-S-CH_2$-Gruppe steht,
- n für eine Zahl 0 oder 1 steht,
- $R^5$ für Methyl oder Ethyl steht,
- $R^6$ für Wasserstoff oder Methyl steht und
- $R^7$ für gegebenenfalls ein- bis dreifach, gleich oder verschieden durch Fluor, Chlor, Methyl, Isopropyl, t-Butyl, Trifluormethyl, Trifluormethoxy, Trifluormethylthio, Methoxy, Phenyl oder Cyclohexyl substituiertes Phenyl steht,
- $R^1$ außerdem für jeweils gegebenenfalls ein- bis dreifach, gleich oder verschieden durch Fluor, Chlor, Brom, Methyl, i-Propyl, t-Butyl, Trifluormethyl, Trifluormethoxy, Trifluormethylthio, Methoxy, Phenyl oder Cyclohexyl substituiertes Phenyl oder Naphthyl steht,
- $R^2$ für Wasserstoff oder Methyl steht,
- $R^3$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Dichlorcyclopropylmethyl, Dimethylcyclopropylmethyl, Dichlordimethylcyclopropylmethyl, Cyclopentyl oder für jeweils gegebenenfalls ein- bis dreifach durch Methyl substituiertes Cyclohexyl oder Cyclohexylmethyl oder für Furanylmethyl, Tetrahydrofuranylmethyl, Tetrahydropyranylmethyl, Dioxolanylmethyl, Dioxolanylethyl oder Dioxanylmethyl steht und
- X für Sauerstoff, Schwefel oder eine $CH_2$-Gruppe steht.

Bevorzugte erfindungsgemäß verwendbare Verbindungen sind auch pflanzenverträgliche Additionsprodukte aus Säuren und denjenigen substituierten Aminomethylheterocyclen der Formel (I), in denen die Substituenten X, $R^1$, $R^2$ und $R^3$ die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure; Sulfonsäuren, wie p-Toluolsulfonsäure und 1,5-Naphthalindisulfonsäure sowie Saccharin.

Im einzelnen sei auf die bei den Herstellungsbeispielen genannten Verbindungen verwiesen.

Die erfindungsgemäß verwendbaren substituierten Aminomethylheterocyclen sind teilweise bekannt (vgl. z.B. GB 1 031 916; DE-OS 2 810 732; DE-OS 2 757 922; Tetrahedron 33, 1309-1319 [1977]; Acta Pol. Pharm. 39, 33-39 [1982] oder Europ. J. Med. Chem./Chim. Ther. 19, 495-500 [1984]) oder können nach Analogieverfahren hergestellt werden, so z. B. nach den für die Verbindungen der Formel (Ia) im folgenden angegebenen Verfahren.

Noch nicht bekannt sind substituierte Aminomethylheterocyclen der Formel (Ia),

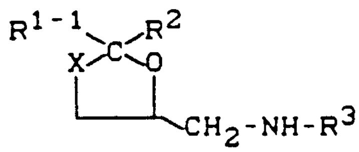

(Ia)

in welcher

$R^{1-1}$ für substituiertes Phenyl, für jeweils gegebenenfalls substituiertes Cycloalkyl, Cycloalkenyl, Tetrahydronaphthyl, Decahydronaphthyl oder Naphthyl, für einen Rest

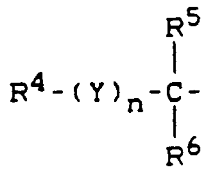

oder für einen Rest

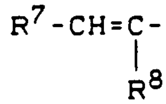

steht,

wobei

$R^4$ für jeweils gegebenenfalls substituiertes Cyclohexyl, Cyclohexenyl oder Phenyl steht,

Y für Sauerstoff, Schwefel, eine $-CH_2$-Gruppe eine $-O-CH_2$-Gruppe, eine $-S-CH_2$-Gruppe, eine Sulfinylgruppe, eine Sulfonylgruppe, eine

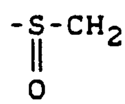-Gruppe

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff, Methyl oder Ethyl steht,

$R^7$ für gegebenenfalls ein- bis dreifach, gleich oder verschieden substituiertes Phenyl steht und

$R^8$ für Methyl oder Ethyl steht;

$R^2$ für Wasserstoff oder Methyl steht,

$R^3$ für Alkyl, Alkenyl, Alkinyl, Alkoxyalkyl, Hydroxyalkyl, Hydroxyalkoxyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und

X für Sauerstoff, Schwefel oder eine $-CH_2$-Gruppe steht,

deren Säureadditionssalze sowie deren geometrische und/oder optische Isomere oder Isomerengemische.

Man erhält die noch nicht bekannten substituierten Aminomethylheterocyclen der Formel (Ia),

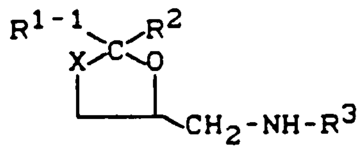

(Ia)

in welcher

$R^{1-1}$ für substituiertes Phenyl, für jeweils gegebenenfalls substituiertes Napthyl, Cycloalkyl, Cycloalkenyl, Tetrahydronaphthyl oder Decahydronaphthyl, für einen Rest

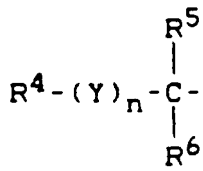

oder für einen Rest

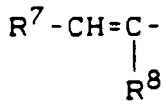

steht,
wobei

$R^4$ für jeweils gegebenenfalls substituiertes Cyclohexyl, Cyclohexenyl oder Phenyl steht,

Y für Sauerstoff, Schwefel, eine $-CH_2$-Gruppe eine $-O-CH_2$-Gruppe, eine $-S-CH_2$-Gruppe, eine Sulfinylgruppe, eine Sulfonylgruppe, eine

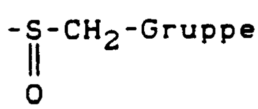

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff, Methyl oder Ethyl steht,

$R^7$ für gegebenenfalls ein- bis dreifach, gleich oder verschieden substituiertes Phenyl steht,

$R^2$ für Wasserstoff oder Methyl steht,

$R^3$ für Alkyl, Alkenyl, Alkinyl, Alkoxyalkyl, Hydroxyalkyl, Hydroxyalkoxyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und

X für Sauerstoff, Schwefel oder eine $CH_2$-Gruppe steht,

deren Säureadditionssalze sowie deren geometrische und/oder optische Isomere oder Isomerengemische in Analogie zu bekannten Verfahren, wenn man

(a) heterocyclische Verbindungen der Formel (II),

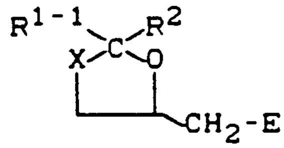

(II)

in welcher

$R^{1-1}$ und $R^2$ die oben angegebene Bedeutung haben und

E für eine elektronenanziehende Abgangsgruppe steht,

mit Aminen der Formel (III),

$H_2N-R^3$ (III)

in welcher

$R^3$ die oben angegebene Bedeutung hat,

gegebenenfalls in Gegenwart eines Verdünnungsmittels und gegebenenfalls in Gegenwart eines Säurebindemittels umsetzt oder wenn man

(b) die mit Hilfe des Verfahrens (a) erhältichen substituierten Aminomethylheterocyclen der Formel (Ia-1),

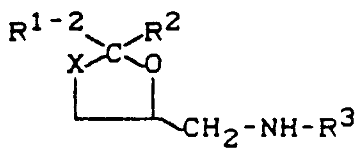

(Ia-1)

in welcher

$R^{1-2}$ für substituiertes Phenyl, für gegebenenfalls substituiertes Naphthyl, für einen Rest

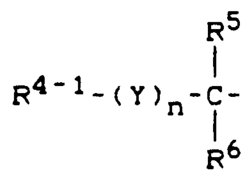

oder für einen Rest

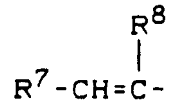

steht,

X, $R^2$, $R^3$, $R^5$, $R^6$, $R^7$ und $R^8$ die oben angegebene Bedeutung haben und

$R^{4-1}$ für gegebenenfalls substituiertes Phenyl steht,

mit Wasserstoff in Gegenwart eines Katalysators sowie gegebenenfalls in Gegenwart eines Verdünnungsmittels hydriert.

Die neuen substituierten Aminomethylheterocyclen sind durch die Formel (Ia) allgemein definiert. Bevorzugt sind Verbindungen der Formel (Ia), bei welchen

$R^{1-1}$ für einfach bis mehrfach, gleich oder verschieden substituiertes Phenyl oder für gegebenenfalls einfach bis mehrfach, gleich oder verschieden substituiertes Naphthyl steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio mit jeweils 1 bis 6 Kohlenstoffatomen und gegebenenfalls 1 bis 9 gleichen oder verschiedenen Halogenatomen, Cyclohexyl oder Phenyl; außerdem für jeweils gegebenenfalls einfach bis mehrfach, gleich oder verschieden substituiertes Cycloalkyl oder Cycloalkenyl mit jeweils 5 bis 7 Kohlenstoffatomen, Tetrahydronaphthyl oder Decahydronaphthyl steht, wobei als Substituenten jeweils infrage kommen: jeweils geradkettiges oder verzweigtes Alkyl, Halogenalkyl oder Halogenalkoxy mit jeweils 1 bis 6 Kohlenstoffatomen und gegebenenfalls 1 bis 9 gleichen oder verschiedenen Halogenatomen; außerdem für einen Rest

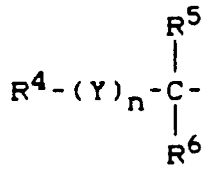

oder für einen Rest

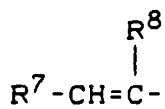

steht, wobei

$R^4$ für jeweils gegebenenfalls ein- bis dreifach, gleich oder verschieden substituiertes Cyclohexyl, Cyclohexenyl oder Phenyl steht, wobei als Cyclohexyl- bzw. Cyclohexenylsubstituenten infrage kommen: jeweils geradkettiges oder verzweigtes Alkyl, Alkoxy oder Halogenalkyl mit jeweils 1 bis 4 Kohlenstoffatomen und gegebenenfalls 1 bis 9 Halogenatomen und wobei als Phenylsubstituenten infrage kommen: Halogen, Cyano, Nitro, jeweils geradkettiges oder verzweigtes Alkyl, Alkoxy, Alkylthio, Halogenalkyl, Halogenalkoxy, oder Halogenalkylthio mit jeweils 1 bis 4 Kohlenstoffatomen und gegebenenfalls 1 bis 9 gleichen oder verschiedenen Halogenatomen, Cyclohexyl oder Phenyl,

Y für Sauerstoff, Schwefel, eine $-CH_2$-Gruppe, eine $-O-CH_2$-Gruppe, eine $-S-CH_2$-Gruppe, eine Sulfinylgruppe, eine Sulfonylgruppe, eine

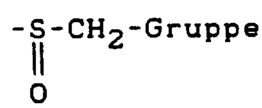

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff oder Methyl steht,

$R^7$ für gegebenenfalls ein- bis dreifach, gleich oder verschieden substituiertes Phenyl steht, wobei als Phenylsubstituenten die bei $R^4$ genannten infrage kommen und

$R^8$ für Methyl oder Ethyl steht,

$R^2$ für Wasserstoff oder Methyl steht,

$R^3$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, oder für jeweils gegebenenfalls im Cycloalkylteil einfach bis mehrfach, gleich oder verschieden substituiertes

Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
außerdem für jeweils gegebenenfalls einfach bis mehrfach, gleich oder verschieden im Arylteil substituiertes Aryl, Arylalkyl oder Arylalkenyl mit jeweils 6 bis 10 Kohlenstoffatomen im Arylteil und gegebenenfalls bis zu 6 Kohlenstoffatomen im jeweils geradkettigen oder verzweigten Alkyl- bzw. Alkenylteil steht, wobei als Arylsubstituenten jeweils infrage kommen:
Halogen, Cyano, Nitro sowie jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder für jeweils geradkettiges oder verzweigtes Furanylalkyl, Tetrahydrofuranylalkyl, Tetrahydropyranylalkyl, Dioxolanylalkyl oder Dioxanylalkyl mit jeweils 1 bis 4 Kohlenstoffatomen im Alkylteil steht und

X für Sauerstoff, Schwefel oder eine $CH_2$-Gruppe steht,

Besonders bevorzugt sind erfindungsgemäße Verbindungen, wobei in der Formel (Ia), bei welchen

$R^{1-1}$ für ein- bis dreifach, gleich oder verschieden substituiertes Phenyl oder für gegebenenfalls ein- bis dreifach, gleich oder verschieden substituiertes Naphthyl steht, wobei als Substituenten jeweils infrage kommen: Fluor, Chlor, Brom, Methyl, i-Propyl, t-Butyl, Trifluormethyl, Trifluormethoxy, Trifluormethylthio, Methoxy, Phenyl oder Cyclohexyl; außerdem für jeweils gegebenenfalls ein- bis dreifach, gleich oder verschieden substituiertes Cyclohexyl, Cyclohexenyl, Tetrahydronaphthyl oder Decahydronaphthyl steht, wobei als Substituenten jeweils infrage kommen: geradkettiges oder verzweigtes Alkyl mit 1 bis 4 Kohlenstoffatomen; außerdem für einen Rest

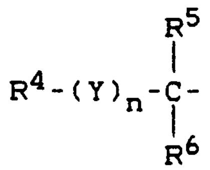

oder für einen Rest

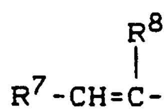

steht, wobei

$R^4$ für jeweils gegebenenfalls ein- bis dreifach, gleich oder verschieden durch Methyl, i-Propyl, t-Butyl, Methoxy, Propoxy, Butoxy oder Trifluormethyl substituiertes Cyclohexyl oder Cyclohexenyl steht oder für gegebenenfalls ein- bis dreifach, gleich oder verschieden durch Fluor, Chlor, Brom, Methyl, i-Propyl, t-Butyl, Trifluormethyl, Trifluormethoxy, Trifluormethylthio, Methoxy, Phenyl oder Cyclohexyl substituiertes Phenyl steht,

Y für Sauerstoff, Schwefel, eine $-CH_2$-Gruppe, eine $-O-CH_2$-Gruppe oder eine $-S-CH_2$-Gruppe steht,

n für eine Zahl 0 oder 1 steht,

$R^5$ für Methyl oder Ethyl steht,

$R^6$ für Wasserstoff oder Methyl steht und

$R^7$ für gegebenenfalls ein- bis dreifach gleich oder verschieden durch Fluor, Chlor, Methyl, Isopropyl, t-Butyl, Trifluormethyl, Trifluormethoxy, Trifluormethylthio, Methoxy, Phenyl oder Cyclohexyl substituiertes Phenyl steht und

$R^8$ für Methyl oder Ethyl steht,

$R^2$ für Wasserstoff oder Methyl steht,

$R^3$ für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für jeweils gegebenenfalls ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethoxy, Trifluormethyl, Trifluormethylthio, Methoxycarbonyl, Ethoxycarbonyl oder Methoximinomethyl; oder für Furanylmethyl, Tetrahydrofuranylmethyl, Tetrahydropyranylmethyl, Dioxolanylmethyl, Dioxolanylethyl, Dioxanylmethyl oder Dioxanylethyl steht und

X für Sauerstoff, Schwefel oder eine $CH_2$-Gruppe steht.

Bevorzugte erfindungsgemäße Verbindungen sind auch pflanzenverträgliche Additionsprodukte aus Säuren und denjenigen substituierten Aminomethylheterocyclen der Formel (Ia), in denen die Substituenten X, $R^{1-1}$ $R^2$ und $R^3$ die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure und 1,5-Naphthalindisulfonsäure sowie Saccharin.

Verwendet man beispielsweise 4-Chlormethyl-2-(4-chlorphenyl)-2-methyl-1,3-dioxolan und Methylamin als Ausgangsstoffe, so läßt sich der Reaktionsablauf des Herstellungsverfahrens (a) durch das folgende Formelschema darstellen:

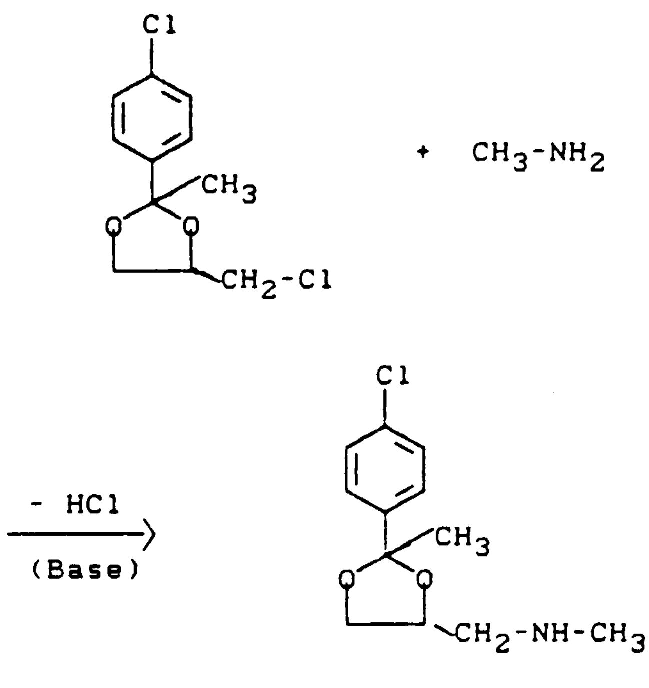


Verwendet man beispielsweise 2-(4-t-Butylphenyl)-2-methyl-5-(N-ethylaminomethyl)-tetrahydrofuran als Ausgangsverbindung, so läßt sich der Reaktionsablauf des Herstellungsverfahrens (b) durch das folgende Formelschema darstellen:

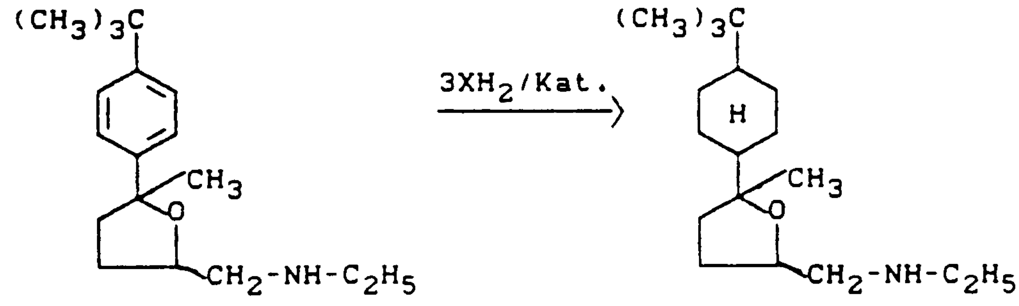


Die zur Durchführung des Herstellungsverfahrens (a) als Ausgangsstoffe benötigten heterocyclischen Verbindungen sind durch die Formel (II) allgemein definiert. In dieser Formel (II) stehen $R^{1-1}$ und $R^2$ vorzugsweise für diejenigen Reste, die bereits im Zusammenhang mit der Beschreibung der erfindungsgemäßen Stoffe der Formel (Ia) als bevorzugt für diese Substituenten genannt wurden.

E steht vorzugsweise für Halogen, insbesondere für Chlor, Brom oder Iod, oder für jeweils gegebenenfalls substituiertes Alkylsulfonyloxy oder Arylsulfonyloxy wie beispielsweise Methansulfonyloxy oder p-Toluolsulfonyloxy.

Die heterocyclischen Ausgangsverbindungen der Formel (II) sind bekannt oder erhältlich in Analogie zu bekannten Verfahren (vgl. z.B. EP 97 822, DE-OS 3 413 996; DE-OS 3 324 769 oder DE-OS 3 328 151).

Die zur Durchführung des Herstellungsverfahren (a) weiterhin als Ausgangsstoffe benötigten Amine sind durch die Formel (III) allgemein definiert. In dieser Formel (III) steht $R^3$ vorzugsweise für diejenigen Reste, die bereits im Zusammenhang mit der Beschreibung der erfindungsgemäßen Stoffe der Formel (Ia) als bevorzugt für diesen Substituenten genannt wurden.

Die Amine der Formel (III) sind allgemein bekannte Verbindungen der organischen Chemie.

Die zur Durchführung des Herstellungsverfahrens (b) als Ausgangsstoffe benötigten substituierten Aminomethylheterocyclen sind durch die Formel (Ia-1) allgemein definiert. In dieser Formel (Ia-1) stehen $R^2$ und $R^3$ vorzugsweise für diejenigen Reste, die bereits im Zusammenhang mit der Beschreibung der erfindungsgemäßen Stoffe der Formel (Ia) als bevorzugt für diese Substituenten genannt wurden.

$R^{1-2}$ steht vorzugsweise für einfach bis mehrfach, gleich oder verschieden substituiertes Phenyl oder für gegebenenfalls einfach bis mehrfach, gleich oder verschieden substituiertes Naphthyl, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy, Halogenalkylthio mit jeweils 1 bis 6 Kohlenstoffatomen und gegebenenfalls 1 bis 9 gleichen oder verschiedenen Halogenatomen, Cyclohexyl oder Phenyl;
außerdem für einen Rest

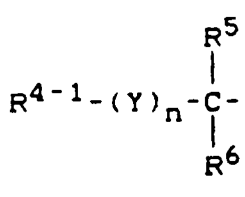

oder für einen Rest

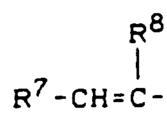

wobei

$R^{4-1}$ für gegebenenfalls ein- bis dreifach, gleich oder verschieden substituiertes Phenyl steht, wobei

als Substituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, Cyclohexyl oder Phenyl,

Y für Sauerstoff, Schwefel, eine $-CH_2$-Gruppe, eine $-O-CN_2$-Gruppe oder eine $-S-CH_2$-Gruppe eine Sulfinylgruppe, eine Sulfonylgruppe, eine

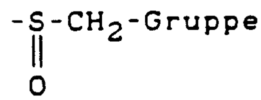

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff oder Methyl steht,

$R^7$ für gegebenenfalls ein- bis dreifach, gleich oder verschieden substituiertes Phenyl steht, wobei als Phenylsubstituenten die bei $R^{4-1}$ genannten infrage kommen und

$R^8$ für Methyl oder Ethyl steht.

$R^{1-2}$ steht besonders bevorzugt für jeweils ein- bis dreifach, gleich oder verschieden substituiertes Phenyl oder Naphthyl, wobei als Substituenten infrage kommen: Fluor, Chlor, Brom, Methyl, i-Propyl, t-Butyl, Trifluormethyl, Trifluormethoxy, Trifluormethylthio, Methoxy, Phenyl oder Cyclohexyl;

außerdem für einen Rest

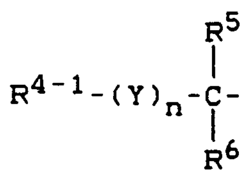

oder für einen Rest

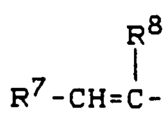

wobei

$R^{4-1}$ für gegebenenfalls ein- bis dreifach, gleich oder verschieden durch Fluor, Chlor, Brom, Methyl, i-Propyl, t-Butyl, Trifluormethyl, Trifluormethoxy, Trifluormethylthio, Methoxy, Propoxy, Butoxy, Phenyl oder Cyclohexyl substituiertes Phenyl steht,

Y für Sauerstoff, Schwefel, eine $-CH_2$-Gruppe, eine $-O-CH_2$-Gruppe oder eine $-S-CH_2$-Gruppe steht,

n für eine Zahl 0 oder 1 steht,

$R^5$ für Methyl oder Ethyl steht,

$R^6$ für Wasserstoff oder Methyl steht,

$R^7$ für gegebenenfalls ein- bis dreifach, gleich oder verschieden durch Fluor, Chlor, Methyl, Isopropyl, t-Butyl, Trifluormethyl, Trifluormethoxy, Trifluormethylthio, Methoxy, Phenyl oder Cyclohexyl substituiertes Phenyl steht und

$R^8$ für Methyl oder Ethyl steht.

Die substituierten Aminomethylheterocyclen der Formel (Ia-1) sind erfindungsgemäße Verbindungen und erhältlich mit Hilfe des Herstellungsverfahrens (a).

Als Verdünnungsmittel zur Durchführung des Herstellungsverfahrens (a) kommen inerte organische

Lösungsmittel infrage.
Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder - diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Propanol oder Butanol. Das Herstellungsverfahren (a) kann jedoch auch ohne Zusatz eines Verdünnungmittels durchgeführt werden.

Das Herstellungsverfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (a) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist jedoch auch möglich, das als Reaktionspartner verwendete Amin der Formel (III) in entsprechendem Überschuß gleichzeitig als Reaktionshilfsmittel und/oder als Verdünnungsmittel zu verwenden.

Die Reaktionstemperaturen können bei der Durchführung des Herstellungsverfahrens (a) in einem größeren Bereich variiert werden. Im allgemeinen arbeitet man bei Temperaturen zwischen 50 °C und 250 °C, vorzugsweise bei Temperaturen zwischen 80 °C und 200 °C.

Das Herstellungsverfahren (a) wird üblicherweise bei Normaldruck durchgeführt. Es ist jedoch auch möglich bei erhöhtem Druck zwischen 1,5 und 5,0 atü zu arbeiten.

Zur Durchführung des Herstellungsverfahrens (a) setzt man pro Mol an Heterocyclus der Formel (II) im allgemeinen 1,0 bis 5,0 Mol; vorzugsweise 1,0 bis 3,0 Mol an Amin der Formel (III) und gegebenenfalls 1,0 bis 5,0 Mol, vorzugsweise 1,0 bis 3,0 Mol an Säurebindemittel ein. Die Reaktionsdurchführung, Aufarbeitung und Isolierung der Reaktionsprodukte der Formel (Ia) erfolgt nach üblichen Methoden.

Als Verdünnungmsittel zur Durchführung des Herstellungsverfahrens (b) kommen ebenfalls inerte organische Lösungsmittel infrage.
Hierzu gehören insbesondere aliphatische oder alicyclische, gegebenenfalls halogenierte Kohlenwasserstoffe, wie beispielsweise Benzin, Petrolether, Hexan, Cyclohexan; Ether, wie Diethylether, Dioxan, Tetrahydrofuran oder Ethylenglylkoldimethyl- oder -diethylether; Ester, wie Essigsäureethylester oder Alkohole, wie Methanol, Ethanol oder Isopropanol.

Als Katalysator zur Durchführung des Herstellungsverfahrens (b) können alle übliche Hydrierkatalysatoren eingesetzt werden, Vorzugsweise verwendet man Edelmetall-, Edelmetalloxid- oder Edelmetallhydroxid-Katalysatoren bzw. sogenannte Raney-Katalysatoren, wie insbesondere Platin, Platinoxid, Palladium, Nickel und Ruthenium, gegebenenfalls auf einen geeigneten Träger wie Kohlenstoff, Aluminiumoxid oder Siliciumdioxid.

Die Reaktionstemperaturen können bei der Durchführung des Herstellungsverfahrens in einem größeren Bereich variiert werden. Im allgemeinen arbeitet man bei Temperaturen zwischen 20 °C und 250 °C, vorzugsweise bei Temperaturen zwischen 20 °C und 200 °C.

Das Herstellungsverfahren (b) wird gegebenenfalls unter erhöhtem Druck durchgeführt. Im allgemeinen arbeitet man in Druckbereichen zwischen 1 und 250 atm, vorzugsweise zwischen 1 und 150 atm.

Zur Durchführung des Herstellungsverfahrens (b) setzt man pro Mol an substituierten Aminomethylheterocyclus der Formel (Ia-1) im allgemeinen 0,001 bis 0,1 Mol, vorzugsweise 0,01 bis 0,05 Mol an Hydrierkatalysator zu und gibt in einem Autoklaven Wasserstoff zu, bis sich der erforderliche Druck eingestellt hat. Die Reaktionsdurchführung, Aufarbeitung und Isolierung der Reaktionsprodukte erfolgt nach üblichen Methoden.

Zur Herstellung von pflanzenverträglichen Säureadditionssalzen der Verbindungen der Formel (Ia) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure,

Maleinsäure, Bernsteinsäure, Fumarsäure, Weinsäure, Zitronensäure, Salicylsäure, Sorbinsäure, Milchsäure, Sulfonsäuren, wie z.B. p-Toluolsulfonsäure und 1,5-Naphthalindisulfonsäure sowie Saccharin.

Die Säureadditionssalze der Verbindungen der Formel (Ia) können in einfacher Weise nach üblichen Salzbildungsmethoden, wie z.B. durch Lösen einer Verbindung der Formel (Ia) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäß verwendbaren Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel geeignet insbesondere als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäß verwendbaren Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Braunfleckenkrankheit des Weizens (Cochliobolus sativus), gegen den Erreger der Netzfleckenkrankheit der Gerste (Pyrenophora teres) oder gegen den Erreger der Braunspelzigkeit des Weizens (Septoria nodorum), zur Bekämpfung von Reiskrankheiten, wie beispielsweise gegen den Erreger der Reisfleckenkrankheit (Pyricularia oryzae) oder zur Bekämpfung von Krankheiten im Obstbau, wie beispielsweise gegen den Erreger des Apfelschorfes (Venturia inaequalis) einsetzen. Dabei zeigen die erfindungsgemäß verwendbaren Wirkstoffe neben guten protektiven Eigenschaften auch systemische Wirksamkeit.

Darüberhinaus besitzen die erfindungsgemäß verwendbaren Wirkstoffe eine gute bakterizide Wirkung und eine hervorragende in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage:
z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

<u>Herstellungsbeispiele</u>

Beispiel 1

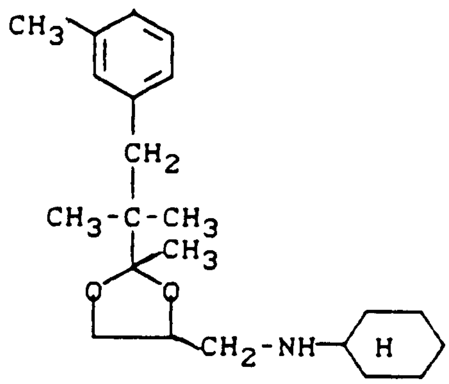

(Verfahren a)

28,3 g (0,1 Mol) 4-Chlormethyl-2-methyl-2-[1-(3-methylphenyl)-2-methyl-prop-2-yl]-1,3-dioxolan und 56,4 g (0,5 Mol) Cyclohexylamin werden 16 Stunden auf Rückflußtemperatur (134 °C) erhitzt, abgekühlt, in 250 ml Essigester aufgenommen, viermal mit jeweils 150 ml Wasser gewaschen, über Natriumsulfat getrocknet, im Vakuum eingeengt und säulenchromatographisch gereinigt (Kieselgel 60; Essigester/Essigester-Ethanol 1:1)
Man erhält 27,5 g (80 % der Theorie) an 4-Cyclohexylaminomethyl-2-methyl-2-[1-(3-methylphenyl)-2-methylprop-2-yl]-1,3-dioxolan vom Brechungsindex $n_D^{20}$ 1.5133.

Herstellung der Ausgangsverbindung

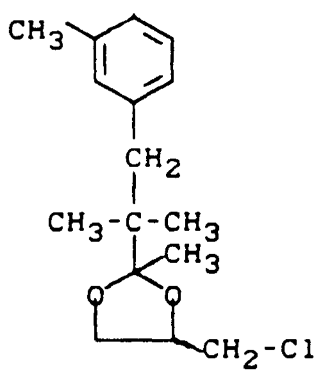

120 g (0,63 Mol) 3,3-Dimethyl-4-(3-methyl-phenyl)-butan-2-on und 139,2 g (1,26 Mol) 3-Chlor-propan-1,2-diol werden zusammen mit 12 g (0,063 Mol) p-Toluolsulfonsäure in 1,2 l Toluol 30 Stunden über einem Wasserabscheider unter Rückfluß erhitzt. Die erkaltete Reaktionsmischung wird 2 mal mit jeweils 500 ml gesättigter wässriger Natriumhydrogencarbonatlösung gewaschen, über Natriumsulfat getrocknet, im Vakuum eingeengt und im Hochvakuum destilliert.
Man erhält 123,3 g (70 % der Theorie) an 4-Chlormethyl-2-methyl-2-[1-(3-methylphenyl)-2-methyl-prop-2-yl]-1,3-dioxolan vom Siedepunkt 100 °C bei 0,13 mbar.

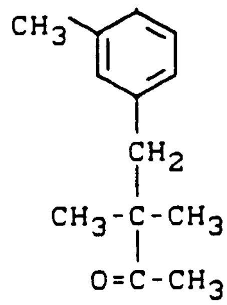

Zu einer Mischung aus 336 g (6 Mol) gepulvertem Kaliumhydroxid und 48,2 g (0,015 Mol) Tetrabutylammoniumbromid in 2 l Cyclohexan gibt man bei Rückflußtemperatur tropfenweise unter Rühren eine Lösung von 421,8 g (3 Mol) 3-Methylbenzylchlorid und 515 g (6 Mol) Methylisopropylketon. Nach beendeter Zugabe erhitzt man 44 Stunden über einem Wasserabscheider auf Rückflußtemperatur, trennt aus der abgekühlten Reaktionsmischung das feste Kaliumhydroxid durch Absaugen über Celite ab, engt das Filtrat im Vakuum ein und destilliert den Rückstand im Hochvakuum.
Man erhält 420 g (37 % der Theorie) an 3,3-Dimethyl-4-(3-methylphenyl)-butan-2-on vom Siedepunkt 74 °c bei 0,35 mbar.

Beispiel 2

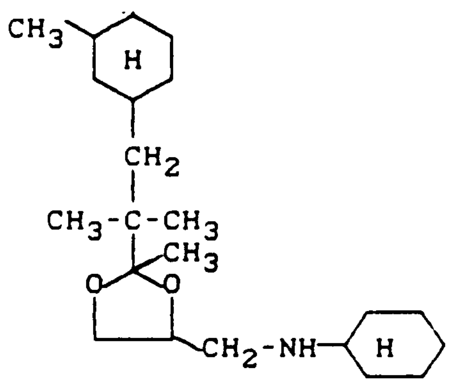

(Verfahren b)

40 g (0,116 Mol) 4-Cyclohexylaminomethyl-2-methyl-2-[1-(3-methylphenyl)-2-methyl-prop-2-yl]-1,3-dioxolan werden zusammen mit 8 g Ruthenium Kohlenstoff-Katalysator (5 %) in 300 ml Isopropanol 2,5 Stunden bei 190 bis 200 bar Wasserstoffdruck und 130 °C hydriert. Zur Aufarbeitung wird der Katalysator abfiltriert und das Lösungsmittel im Vakuum abdestilliert.
Man erhält 36,8 g (91 % der Theorie) an 4-Cyclohexylaminomethyl-2-methyl-[2-(1-(3-methylcylohexyl )-2-methylprop-2-yl]-1,3-dioxolan als Öl vom Brechungsindex $n_D^{20}$ 1,4839.

In entsprechender Weise und gemäß den allgemeinen Angaben zur Herstellung erhält man die folgenden substituierten Aminomethylheterocyclen der allgemeinen Formel (I):

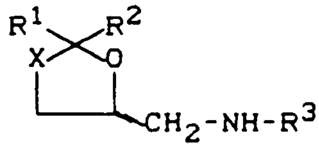

(I)

| Bsp. Nr. | $R^1$ | $R^2$ | $R^3$ | X | physikalische Eigenschaften |
|---|---|---|---|---|---|
| 3 | 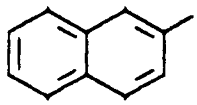 | $CH_3$ | $CH_3$ | O | $^1$H-NMR*): 2,4-2,9; 7,4-8,05 |
| 4 | 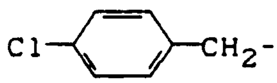 | $CH_3$ | $CH_3$ | O | $^1$H-NMR*): 2,2-3,0; 7,15-7,3 |
| 5 | 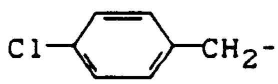 | $CH_3$ | $C_2H_5$ | O | $^1$H-NMR*): 2,3-2,7; 7,15-7,3 |
| 6 | 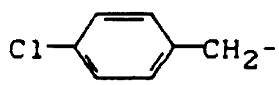 | $CH_3$ | $CH_2=CH-CH_2-$ | O | $^1$H-NMR*): 7,15-7,3 |
| 7 | 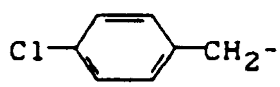 | $CH_3$ | $CH_3-(CH_2)_2-$ | O | $^1$H-NMR*): 7,15-7,3 |

| Bsp. Nr. | $R^1$ | $R^2$ | $R^3$ | X | physikalische Eigenschaften |
|---|---|---|---|---|---|
| 8 | 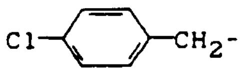 | $CH_3$ | $CH_3-(CH_2)_3-$ | O | $^1H$-NMR*): 7,17-7,3 |
| 9 | 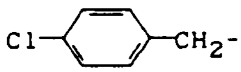 | $CH_3$ | $CH_3-(CH_2)_4-$ | O | $^1H$-NMR*): 7,15-7,3 |
| 10 | 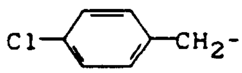 | $CH_3$ | $CH_3-(CH_2)_5-$ | O | $^1H$-NMR*): 7,15-7,3 |
| 11 | 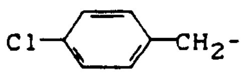 | $CH_3$ | $(CH_3)_2CH-$ | O | $^1H$-NMR*): 7,15-7,3 |
| 12 | 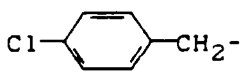 | $CH_3$ | 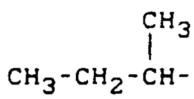 | O | $^1H$-NMR*): 7,15-7,3 |
| 13 | 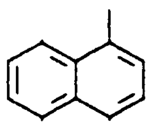 | $CH_3$ | $CH_3$ | O | $^1H$-NMR*): 7,3-7,85; 8,5-8,6 |
| 14 | 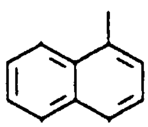 | $CH_3$ | $C_2H_5$ | O | $^1H$-NMR*): 7,35-7,9; 8,55-8,65 |
| 15 | 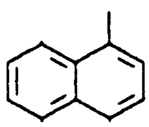 | $CH_3$ | $CH_2=CH-CH_2-$ | O | $^1H$-NMR*): 7,35-7,9; 8,55-8,65 |

| Bsp. Nr. | $R^1$ | $R^2$ | $R^3$ | X | physikalische Eigenschaften |
|---|---|---|---|---|---|
| 16 | 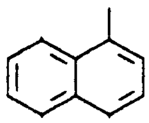 | $CH_3$ | $CH_3-(CH_2)_2-$ | O | $^1H$-NMR*): 7,35-7,85; 8,55-8,65 |
| 17 | 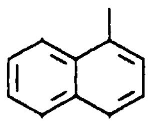 | $CH_3$ | $CH_3-(CH_2)_3-$ | O | $^1H$-NMR*): 7,35-7,9; 8,55-8,65 |
| 18 | 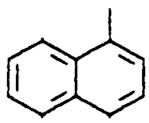 | $CH_3$ | $CH_3-(CH_2)_4-$ | O | $^1H$-NMR*): 7,3-7,9; 8,55-8,65 |
| 19 | 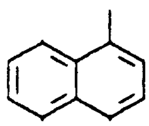 | $CH_3$ | $CH_3-(CH_2)_5-$ | O | $^1H$-NMR*): 7,35-7,85; 8,55-8,65 |
| 20 | 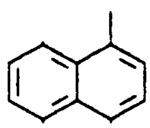 | $CH_3$ | $(CH_3)_2CH-$ | O | $^1H$-NMR*): 7,35-7,85; 8,55-8,65 |
| 21 | 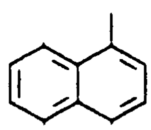 | $CH_3$ | $C_2H_5-CH(CH_3)-$ | O | $^1H$-NMR*): 7,35-7,85; 8,55-8,65 |
| 22 | 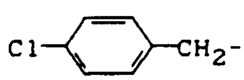 | $CH_3$ | $(C_2H_5)_2CH-$ | O | $^1H$-NMR*): 7,15-7,3 |

| Bsp. Nr. | $R^1$ | $R^2$ | $R^3$ | X | physikalische Eigenschaften |
|---|---|---|---|---|---|
| 23 | 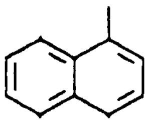 | $CH_3$ | $(C_2H_5)_2CH-$ | O | $^1H$-NMR*): 7,35-7,85; 8,55-8,65 |
| 24 | 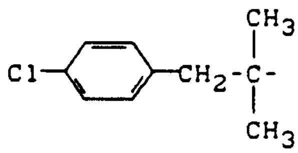 | H | $CH_3$ | O | $^1H$-NMR*): 7,05,7,3 |
| 25 | 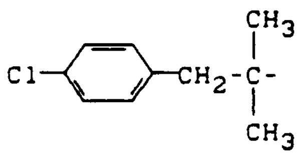 | H | $C_2H_5$ | O | $^1H$-NMR*): 7,05-7,3 |
| 26 | 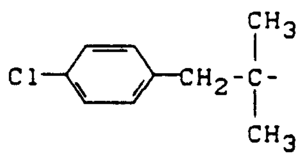 | H | $CH_3-(CH_2)_3-$ | O | $^1H$-NMR*): 7,05-7,3 |
| 27 | 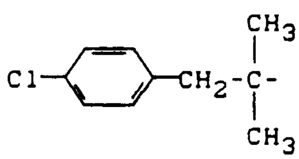 | H | $(CH_3)_2CH-$ | O | $^1H$-NMR*): 7,05-7,35 |
| 28 | 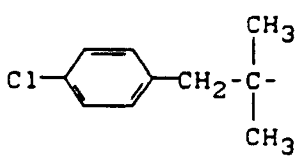 | H | $CH_3-(CH_2)_3-$ | O | $^1H$-NMR*): 7,05-7,3 |

| Bsp. Nr. | $R^1$ | $R^2$ | $R^3$ | X | physikalische Eigenschaften |
|---|---|---|---|---|---|
| 29 | 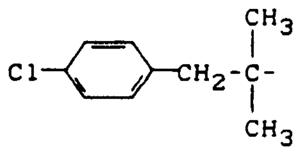 | H | $CH_3-(CH_2)_5-$ | O | $^1H$-NMR*): 7,05-7,3 |
| 30 | 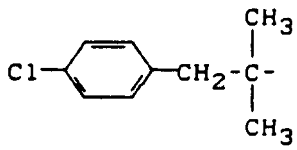 | H | 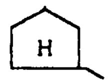 | O | $^1H$-NMR*): 7,02-7,28 |
| 31 | 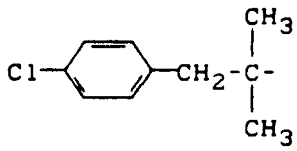 | H | 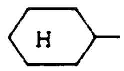 | O | $^1H$-NMR*): 7,05-7,32 |
| 32 | 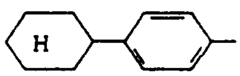 | $CH_3$ | $CH_3$ | O | $^1H$-NMR*): 7,1-7,4 |
| 33 | 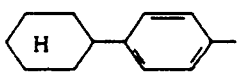 | $CH_3$ | $C_2H_5$ | O | $^1H$-NMR*): 7,1-7,45 |
| 34 | 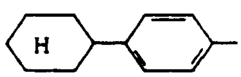 | $CH_3$ | 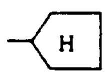 | O | $^1H$-NMR*): 7,1-7,45 |
| 35 | 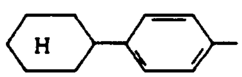 | $CH_3$ | 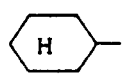 | O | $^1H$-NMR*): 7,1-7,4 |

| Bsp. Nr. | $R^1$ | $R^2$ | $R^3$ | X | physikalische Eigenschaften |
|---|---|---|---|---|---|
| 36 | 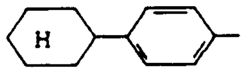 | $CH_3$ | $CH_3-(CH_2)_3-$ | O | $^1H$-NMR*): 7,1-7,4 |
| 37 | 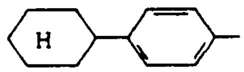 | $CH_3$ | $(CH_3)_2CH-$ | O | $^1H$-NMR*): 7,1-7,45 |
| 38 | 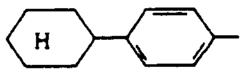 | $CH_3$ | $(CH_3)_2CH-CH_2-$ | O | $^1H$-NMR*): 7,08-7,4 |
| 39 | 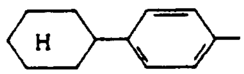 | $CH_3$ | $CH_3-(CH_2)_5-$ | O | $^1H$-NMR*): 7,1-7,4 |
| 40 | 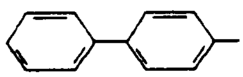 | $CH_3$ | $CH_3$ | O | $^1H$-NMR*): 7,28-7,65 |
| 41 | 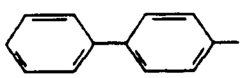 | $CH_3$ | $C_2H_5$ | O | $^1H$-NMR*): 7,28-7,6 |
| 42 | 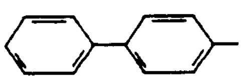 | $CH_3$ | 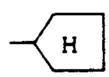 | O | $^1H$-NMR*): 7,3-7,6 |
| 43 | 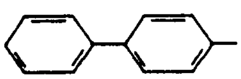 | $CH_3$ | 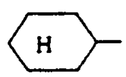 | O | $^1H$-NMR*): 7,28,7,6 |

| Bsp. Nr. | $R^1$ | $R^2$ | $R^3$ | X | physikalische Eigenschaften |
|---|---|---|---|---|---|
| 44 | 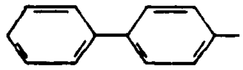 | $CH_3$ | $CH_3-(CH_2)_3-$ | O | $^1$H-NMR*): 7,28-7,6 |
| 45 | 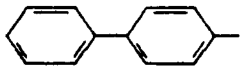 | $CH_3$ | $(CH_3)_2CH-$ | O | $^1$H-NMR*): 7,25-7,6 |
| 46 | 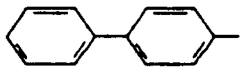 | $CH_3$ | $(CH_3)_2CH-CH_2-$ | O | $^1$H-NMR*): 7,28-7,62 |
| 47 | 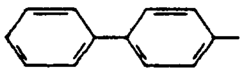 | $CH_3$ | $CH_3-(CH_2)_5-$ | O | $^1$H-NMR*): 7,28-7,6 |
| 48 | 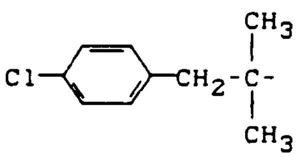 | $CH_3$ | $CH_3$ | O | $n_D^{20}$ 1,5190 |
| 49 | 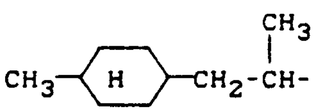 | $CH_3$ | $CH_3-(CH_2)_2-$ | O | $n_D^{20}$ 1,4659 |
| 50 | 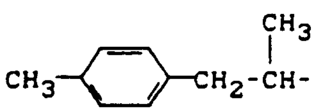 | $CH_3$ | $CH_3$ | O | $n_D^{20}$ 1,5064 |
| 51 | 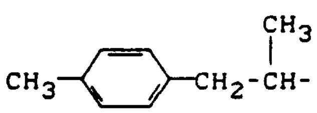 | $CH_3$ | $C_2H_5$ | O | $n_D^{20}$ 1,5015 |

| Bsp. Nr. | $R^1$ | $R^2$ | $R^3$ | X | physikalische Eigenschaften |
|---|---|---|---|---|---|
| 52 | 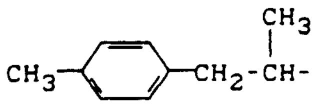 | $CH_3$ | $CH_3-(CH_2)_3-$ | O | $^1H$-NMR*): 2,7; 3,1 |
| 53 | 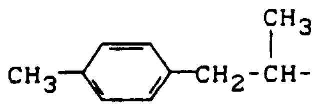 | $CH_3$ | $(CH_3)_2CH-CH_2-$ | O | $n_D^{20}$ 1,4914 |
| 54 | 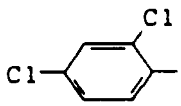 | H | $CH_3-(CH_2)_2-$ | O | $^1H$-NMR*): 2,6; 2,85 |
| 55 | 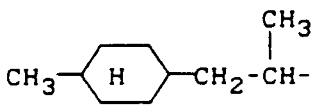 | $CH_3$ | $CH_3$ | O | $n_D^{20}$ 1,4663 |
| 56 | 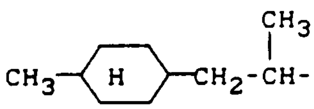 | $CH_3$ | $CH_3-(CH_2)_3-$ | O | $n_D^{20}$ 1,4664 |
| 57 | 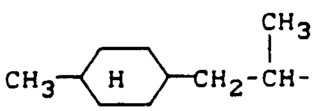 | $CH_3$ | $(CH_3)_2CH-CH_2-$ | O | $^1H$-NMR*): 2,43; 2,78 |
| 58 | 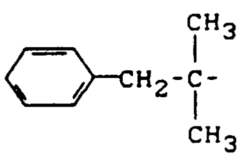 | $CH_3$ | $CH_3$ | O | $^1H$-NMR*): 2,5; 2,7 |

| Bsp. Nr. | $R^1$ | $R^2$ | $R^3$ | X | physikalische Eigenschaften |
|---|---|---|---|---|---|
| 59 | 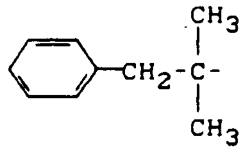 | $CH_3$ | $C_2H_5$ | O | $n_D^{20}$ 1,5097 |
| 60 | 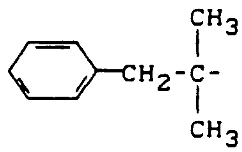 | $CH_3$ | $CH_3-(CH_2)_2-$ | O | $n_D^{20}$ 1,5010 |
| 61 | 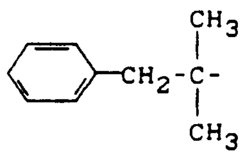 | $CH_3$ | $(CH_3)_2CH-CH_2-$ | O | $n_D^{20}$ 1,4944 |
| 62 | 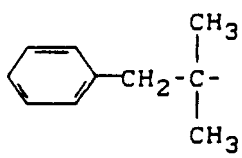 | $CH_3$ | $CH_3-(CH_2)_3-$ | O | $^1H$-NMR*): 2,5; 2,7 |
| 63 | 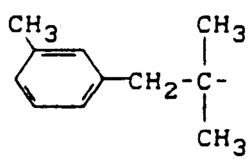 | $CH_3$ | $(CH_3)_2CH-CH_2-$ | O | $^1H$-NMR*): 2,45; 2,65 |
| 64 | 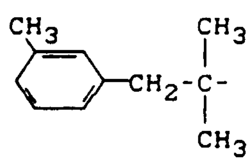 | $CH_3$ | $C_2H_5$ | O | $^1H$-NMR*): 2,7; 2,75 |

| Bsp. Nr. | $R^1$ | $R^2$ | $R^3$ | X | physikalische Eigenschaften |
|---|---|---|---|---|---|
| 65 | 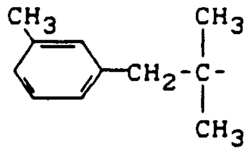 | $CH_3$ | $CH_2$-$(CH_2)_2$- | O | $^1H$-NMR*): 2,65; 2,8 |
| 66 | 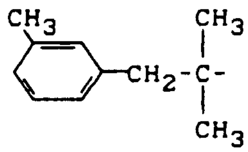 | $CH_3$ | $CH_3$ | O | $n_D^{20}$ 1,5098 |
| 67 | 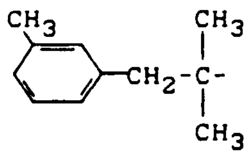 | $CH_3$ | $CH_3$-$(CH_2)_3$- | O | $n_D^{20}$ 1,4978 |
| 68 | 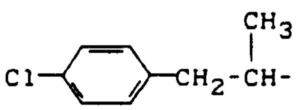 | $CH_3$ | $CH_3$-$(CH_2)_2$- | O | $n_D^{20}$ 1,5079 |
| 69 | 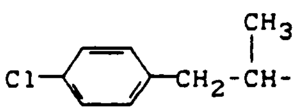 | $CH_3$ | $CH_3$-$(CH_2)_3$- | O | $^1H$-NMR*): 2,65; 2,8 |
| 70 | 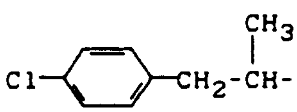 | $CH_3$ | $(CH_3)_2CH$-$CH_2$- | O | $n_D^{20}$ 1,5028 |

| Bsp. Nr. | $R^1$ | $R^2$ | $R^3$ | X | physikalische Eigenschaften |
|---|---|---|---|---|---|
| 71 | 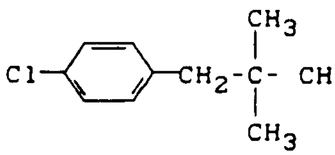 | $CH_3$ | $(CH_3)_2CH-CH_2-$ | O | $n_D^{20}$ 1,5063 |
| 72 | 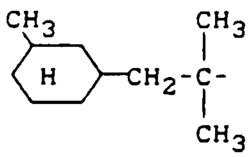 | $CH_3$ | $CH_3-(CH_2)_2-$ | O | $^1H$-NMR*): 2,6; 2,7 |
| 73 | 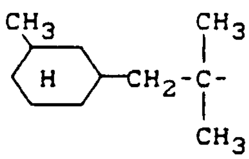 | $CH_3$ | $CH_3-(CH_2)_3-$ | O | $^1H$-NMR*): 2,6; 2,72 |
| 74 | 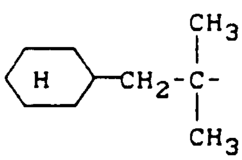 | $CH_3$ | $C_2H_5$ | O | $n_D^{20}$ 1,4717 |
| 75 | 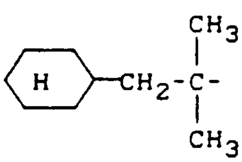 | $CH_3$ | $(CH_3)_2CH-CH_2-$ | O | $n_D^{20}$ 1,4709 |
| 76 | 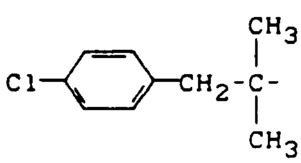 | $CH_3$ | $CH_3-(CH_2)_3-$ | O | $^1H$-NMR*): 2,62; 2,75 |

| Bsp. Nr. | $R^1$ | $R^2$ | $R^3$ | X | physikalische Eigenschaften |
|---|---|---|---|---|---|
| 77 | 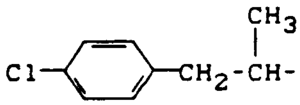 | $CH_3$ | $C_2H_5$ | O | $n_D^{20}$ 1,5120 |
| 78 | 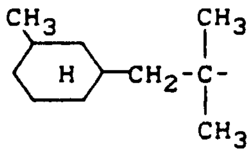 | $CH_3$ | $CH_3$ | O | $n_D^{20}$ 1,4724 |
| 79 | 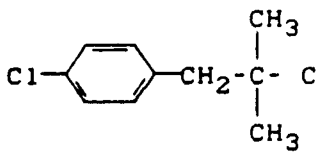 | $CH_3$ | $C_2H_5$ | O | $^1H$-NMR*): 2,65; 2,8 |
| 80 | 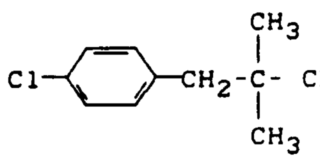 | $CH_3$ | $CH_3-(CH_2)_2-$ | O | $^1H$-NMR*): 2,65; 2,75 |
| 81 | 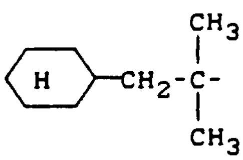 | $CH_3$ | $CH_3(CH_2)_2-$ | O | $^1H$-NMR*): 2,65; 2,75 |
| 82 | 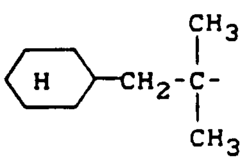 | $CH_3$ | $CH_3-(CH_2)_3-$ | O | $^1H$-NMR*): 2,70; 2,78 |

| Bsp. Nr. | $R^1$ | $R^2$ | $R^3$ | X | physikalische Eigenschaften |
|---|---|---|---|---|---|
| 83 | 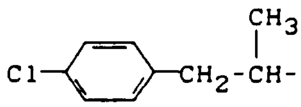 | $CH_3$ | $CH_3$ | O | $n_D^{20}$ 1,5171 |
| 84 | 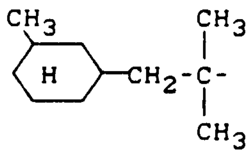 | $CH_3$ | $C_2H_5$ | O | $^1$H-NMR*): 2,7 |
| 85 | 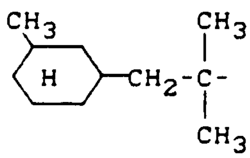 | $CH_3$ | $(CH_3)_2CH-CH_2-$ | O | $n_D^{20}$ 1,4682 |
| 86 | 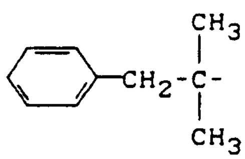 | $CH_3$ | 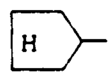 | O | $n_D^{20}$ 1,5143 |
| 87 | 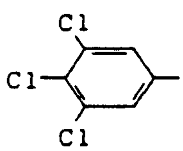 | H | $CH_3-(CH_2)_2-$ | O | $n_D^{20}$ 1,5424 |
| 88 | 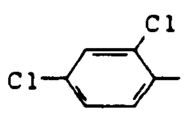 | H | $C_2H_5$ | O | $^1$H-NMR*): 2,6; 2,85 |

| Bsp. Nr. | $R^1$ | $R^2$ | $R^3$ | X | physikalische Eigenschaften |
|---|---|---|---|---|---|
| 89 | 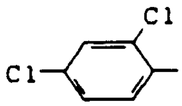 | H | $CH_3-(CH_2)_3-$ | O | $^1H$-NMR*): 2,65; 2,85 |
| 90 | 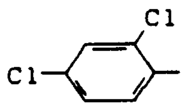 | H | $(CH_3)_2CH-CH_2-$ | O | $^1H$-NMR*): 2,45; 2,85 |
| 91 | 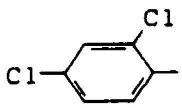 | H | $(CH_3)_3C-$ | O | $n_D^{20}$ 1,5236 |
| 92 | 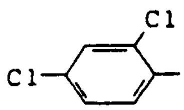 | H | $CH_3-(CH_2)_4-$ | O | $n_D^{20}$ 1,5223 |
| 93 | 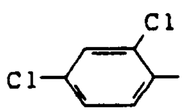 | H | $CH_3-(CH_2)_5-$ | O | $^1H$-NMR*): 2,65; 2,85 |
| 94 | 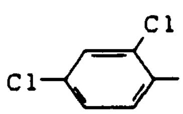 | H | 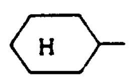 | O | $^1H$-NMR*): 2,45; 2,9 |
| 95 | 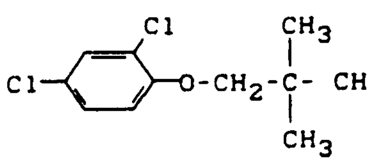 | $CH_3$ | $CH_3-(CH_2)_2-$ | O | $^1H$-NMR*): 2,6; 2,75 |

| Bsp. Nr. | $R^1$ | $R^2$ | $R^3$ | X | physikalische Eigenschaften |
|---|---|---|---|---|---|
| 96 | 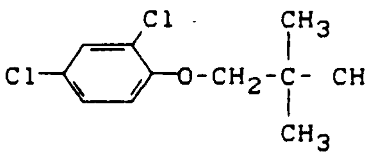 | $CH_3$ | $CH_3$-$(CH_2)_3$- | O | $n_D^{20}$ 1,5146 |
| 97 | 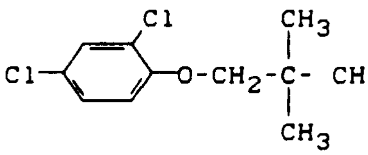 | $CH_3$ | $(CH_3)_2$CH-$CH_2$- | O | $n_D^{20}$ 1,5116 |
| 98 | 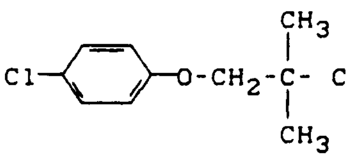 | $CH_3$ | $CH_3$-$(CH_2)_2$- | O | $^1$H-NMR*): 2,6; 2,75 |
| 99 | 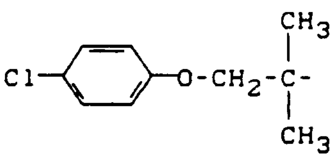 | $CH_3$ | $CH_3$-$(CH_2)_3$- | O | $n_D^{20}$ 1,5057 |
| 100 | 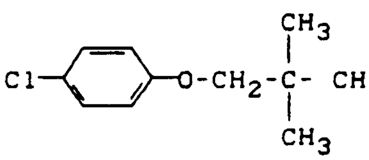 | $CH_3$ | $(CH_3)_2$CH-$CH_2$- | O | $n_D^{20}$ 1,5019 |
| 101 | 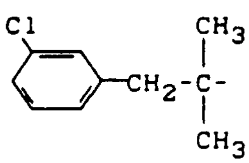 | $CH_3$ | $CH_3$-$(CH_2)_2$- | O | $^1$H-NMR*): 2,6; 2,75 |

| Bsp. Nr. | $R^1$ | $R^2$ | $R^3$ | X | physikalische Eigenschaften |
|---|---|---|---|---|---|
| 102 | 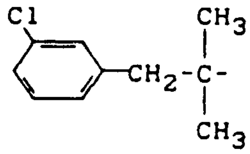 | $CH_3$ | $CH_3-(CH_2)_3-$ | O | $n_D^{20}$ 1,5093 |
| 103 | 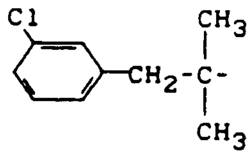 | $CH_3$ | $(CH_3)_2CH-CH_2-$ | O | $n_D^{20}$ 1,5074 |
| 104 | 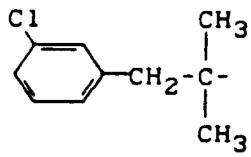 | $CH_3$ | $CH_3$ | O | $n_D^{20}$ 1,5199 |
| 105 | 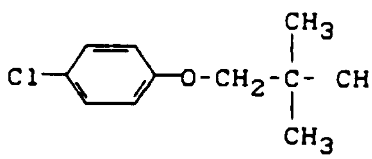 | $CH_3$ | $CH_3$ | O | $n_D^{20}$ 1,5151 |
| 106 | 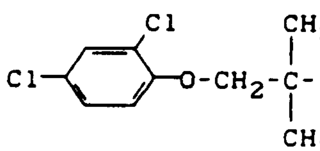 | $CH_3$ | $CH_3$ | O | $^1$H-NMR*): 2,48; 2,7 |
| 107 | 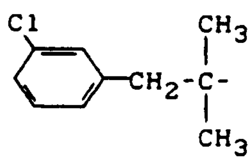 | $CH_3$ | $C_2H_5$ | O | $n_D^{20}$ 1,5156 |

| Bsp. Nr. | $R^1$ | $R^2$ | $R^3$ | X | physikalische Eigenschaften |
|---|---|---|---|---|---|
| 108 | 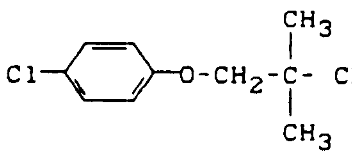 | $CH_3$ | $C_2H_5$ | O | $^1$H-NMR*): 2,7; 2,78 |
| 109 | 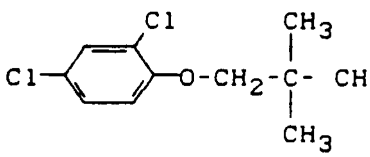 | $CH_3$ | $C_2H_5$ | O | $^1$H-NMR*): 2,6; 2,7 |
| 110 | 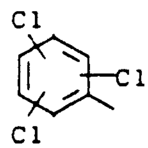 | H | $CH_3-(CH_2)_3-$ | O | $^1$H-NMR*): 2,65; 2,85 |
| 111 | 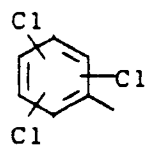 | H | $(CH_3)_2CH-CH_2-$ | O | $n_D^{20}$ 1,5320 |
| 112 | 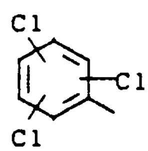 | H | $CH_3$ | O | $^1$H-NMR*): 2,5; 2,85 |
| 113 | 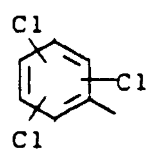 | H | $C_2H_5$ | O | $n_D^{20}$ 1,5502 |

| Bsp. Nr. | $R^1$ | $R^2$ | $R^3$ | X | physikalische Eigenschaften |
|---|---|---|---|---|---|
| 114 | 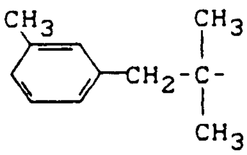 | $CH_3$ | 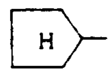 | O | $n_D^{20}$ 1,5143 |
| 115 | 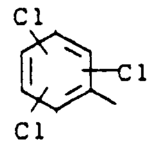 | H | $CH_3-(CH_2)_5-$ | O | $n_D^{20}$ 1,5230 |
| 116 | 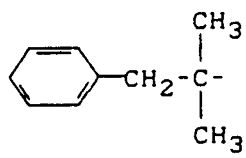 | $CH_3$ | 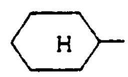 | O | $^1H$-NMR*): 2,45; 2,75 |
| 117 | 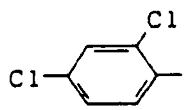 | H | $CH_3-(CH_2)_5-$ | O | $n_D^{20}$ 1,5142 |
| 118 | 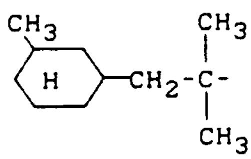 | $CH_3$ | 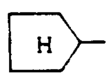 | O | $n_D^{20}$ 1,4830 |
| 119 | 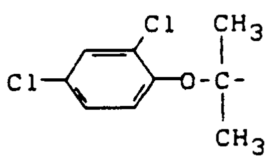 | $CH_3$ | 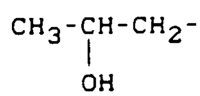 | O | $n_D^{20}$ 1,5297 |

| Bsp. Nr. | $R^1$ | $R^2$ | $R^3$ | X | physikalische Eigenschaften |
|---|---|---|---|---|---|
| 120 | 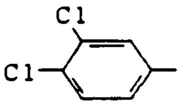 | H | $(CH_3)_2CH-CH_2-$ | O | $n_D^{20}$ 1,5237 |
| 121 | 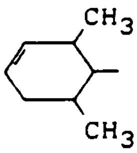 | H | $CH_3-(CH_2)_3-$ | O | $n_D^{20}$ 1,4781 |
| 122 | 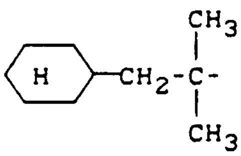 | $CH_3$ | 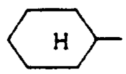 | O | $n_D^{20}$ 1,4854 |
| 123 | 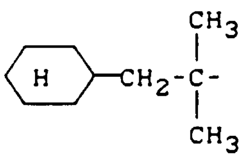 | $CH_3$ | 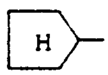 | O | $^1H$-NMR*): 2,7; 3,1 |
| 124 | 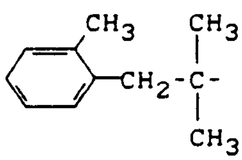 | $CH_3$ | $(CH_3)_2CH-CH_2-$ | O | $n_D^{20}$ 1,4978 |
| 125 | 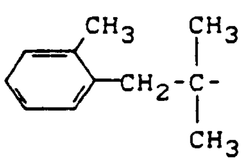 | $CH_3$ | $CH_3-(CH_2)_3-$ | O | $n_D^{20}$ 1,5008 |

| Bsp. Nr. | $R^1$ | $R^2$ | $R^3$ | X | physikalische Eigenschaften |
|---|---|---|---|---|---|
| 126 | 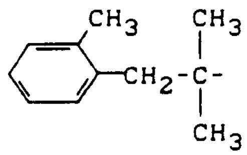 | $CH_3$ | $(CH_3)_2CH-$ | O | $n_D^{20}$ 1,5029 |
| 127 | 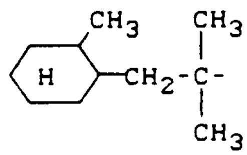 | $CH_3$ | $(CH_3)_2CH-CH_2-$ | O | $n_D^{20}$ 1,4700 |
| 128 | 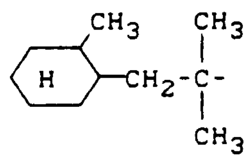 | $CH_3$ | $CH_3-(CH_2)_3-$ | O | $^1H$-NMR*): 2,65; 2,75 |
| 129 | 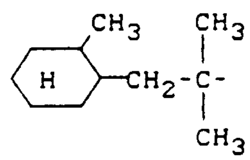 | $CH_3$ | $(CH_3)_2CH-$ | O | $n_D^{20}$ 1,4709 |
| 130 | 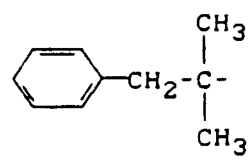 | $CH_3$ | $(CH_3)_3C-$ | O | $n_D^{20}$ 1,4958 |
| 131 | 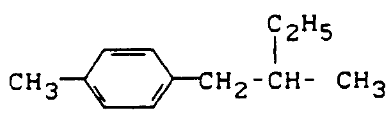 | $CH_3$ | $(CH_3)_3C-$ | O | $n_D^{20}$ 1,4941 |

| Bsp. Nr. | $R^1$ | $R^2$ | $R^3$ | X | physikalische Eigenschaften |
|---|---|---|---|---|---|
| 132 | 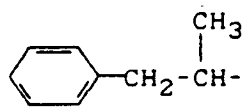 | $CH_3$ | $(CH_3)_3C-$ | O | $n_D^{20}$ 1,4925 |
| 133 | 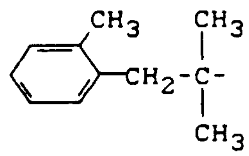 | $CH_3$ | $(CH_3)_3C-$ | O | $n_D^{20}$ 1,4977 |
| 134 | 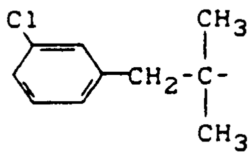 | $CH_3$ | $(CH_3)_3C-$ | O | $n_D^{20}$ 1,5035 |
| 135 | 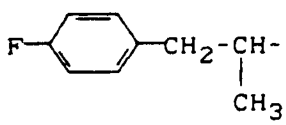 | $CH_3$ | $(CH_3)_3C-$ | O | $n_D^{20}$ 1,4840 |
| 136 | 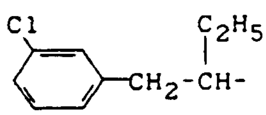 | $CH_3$ | $(CH_3)_3C-$ | O | $n_D^{20}$ 1,5025 |
| 137 | 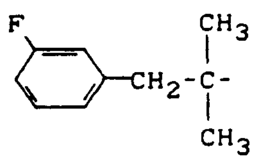 | $CH_3$ | $(CH_3)_3C-$ | O | $n_D^{20}$ 1,4888 |

| Bsp. Nr. | $R^1$ | $R^2$ | $R^3$ | X | physikalische Eigenschaften |
|---|---|---|---|---|---|
| 138 | 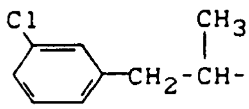 | $CH_3$ | $(CH_3)_3C-$ | O | $n_D^{20}$ 1,5036 |
| 139 | 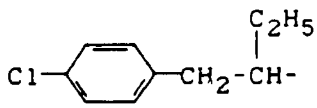 | $CH_3$ | $(CH_3)_3C-$ | O | $n_D^{20}$ 1,5009 |
| 140 | 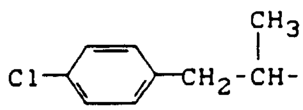 | $CH_3$ | $(CH_3)_3C-$ | O | $n_D^{20}$ 1,5026 |
| 141 | 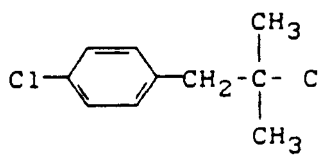 | $CH_3$ | $(CH_3)_3C-$ | O | $n_D^{20}$ 1,5054 |
| 142 | 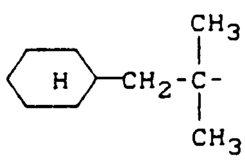 | $CH_3$ | $(CH_3)_3C-$ | O | $^1H$-NMR*): 2,6 |
| 143 | 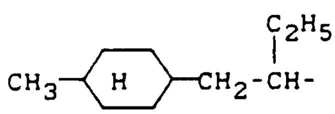 | $CH_3$ | $(CH_3)_3C-$ | O | $n_D^{20}$ 1,4645 |
| 144 | 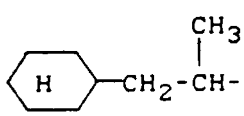 | $CH_3$ | $(CH_3)_3C-$ | O | $n_D^{20}$ 1,4638 |

| Bsp. Nr. | $R^1$ | $R^2$ | $R^3$ | X | physikalische Eigenschaften |
|---|---|---|---|---|---|
| 145 | 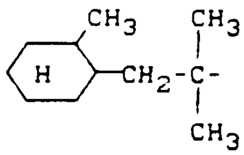 | $CH_3$ | $(CH_3)_3C-$ | O | $n_D^{20}$ 1,4698 |
| 146 | 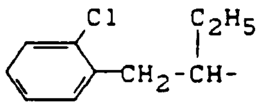 | $CH_3$ | $(CH_3)_3C-$ | O | $n_D^{20}$ 1,5015 |
| 147 | 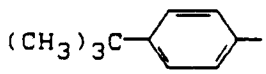 | H | $CH_3-(CH_2)_3-$ | O | $n_D^{20}$ 1,4996 |
| 148 | 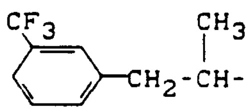 | $CH_3$ | $CH_3-(CH_2)_3-$ | O | $n_D^{20}$ 1,4667 |
| 149 | 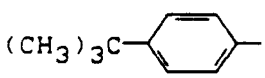 | H | $CH_3-(CH_2)_2-$ | O | $n_D^{20}$ 1,5035 |
| 150 | 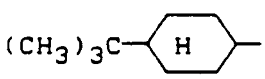 | H | $CH_3-(CH_2)_3-$ | O | $n_D^{20}$ 1,4701 |
| 151 | 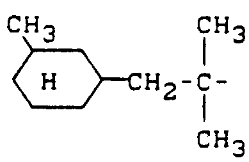 | $CH_3$ | $(CH_3)_3C-$ | O | $n_D^{20}$ 1,4663 |

| Bsp. Nr. | $R^1$ | $R^2$ | $R^3$ | X | physikalische Eigenschaften |
|---|---|---|---|---|---|
| 152 | 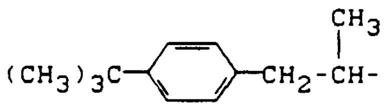 | H | 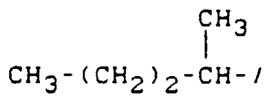 / $CH_3-(CH_2)_4-$ (60:40) | | $n_D^{20}$ 1,4926 |
| 153 | 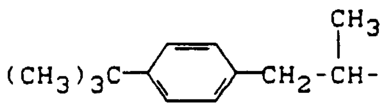 | H | $CH_3-(CH_2)_5-$ | O | $n_D^{20}$ 1,4918 |
| 154 | 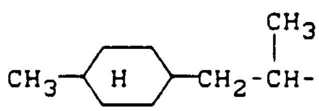 | $CH_3$ | $(CH_3)_3C-$ | O | $^1$H-NMR*): 2,6 |
| 155 | 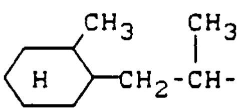 | $CH_3$ | $(CH_3)_3C-$ | O | $n_D^{20}$ 1,4658 |
| 156 | 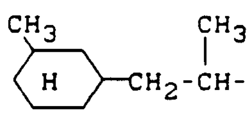 | $CH_3$ | $(CH_3)_3C-$ | O | $^1$H-NMR*): 2,78 |
| 157 | 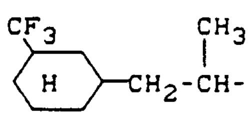 | $CH_3$ | $CH_3-(CH_2)_3-$ | O | $n_D^{20}$ 1,4449 |

| Bsp. Nr. | $R^1$ | $R^2$ | $R^3$ | X | physikalische Eigenschaften |
|---|---|---|---|---|---|
| 158 | 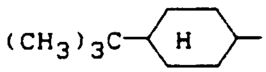 | H | $CH_3-(CH_2)_2-$ | O | $n_D^{20}$ 1,4702 |
| 159 | 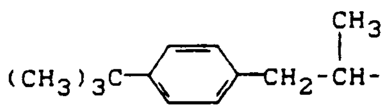 | H | $CH_3-(CH_2)_2-$ | O | $n_D^{20}$ 1,4971 |
| 160 | 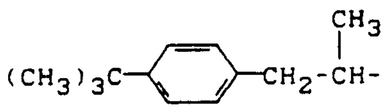 | H | $(CH_3)_2CH-CH_2-$ | O | $n_D^{20}$ 1,4924 |
| 161 | 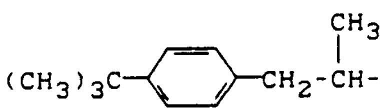 | H | $CH_3$ | O | $n_D^{20}$ 1,5036 |
| 162 | 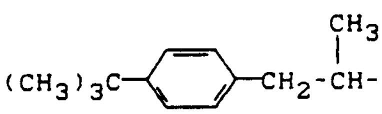 | H | $CH_3-(CH_2)_3-$ | O | $n_D^{20}$ 1,4954 |
| 163 | 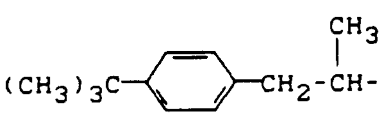 | H | $(CH_3)_3C-$ | O | $n_D^{20}$ 1,4917 |

| Bsp. Nr. | $R^1$ | $R^2$ | $R^3$ | X | physikalische Eigenschaften |
|---|---|---|---|---|---|
| 164 | 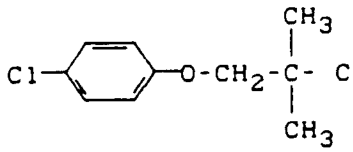 | $CH_3$ | $(CH_3)_3C-$ | O | $^1H$-NMR*): 2,7 |
| 165 | 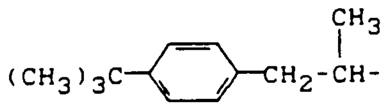 | H | $C_2H_5$ | O | $n_D^{20}$ 1,5002 |
| 166 | 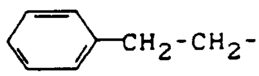 | $CH_3$ | $CH_3-(CH_2)_2-$ | O | $n_D^{20}$ 1,4962 |
| 167 | 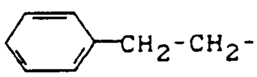 | $CH_3$ | $CH_3-(CH_2)_3-$ | O | $n_D^{20}$ 1,4934 |
| 168 | 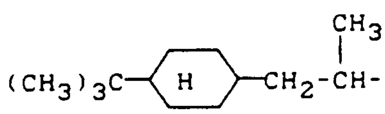 | H | $CH_3$ | O | $n_D^{20}$ 1,4741 |
| 169 | 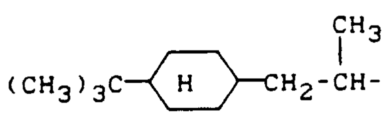 | H | $C_2H_5$ | O | $n_D^{20}$ 1,4711 |

| Bsp. Nr. | $R^1$ | $R^2$ | $R^3$ | X | physikalische Eigenschaften |
|---|---|---|---|---|---|
| 170 | 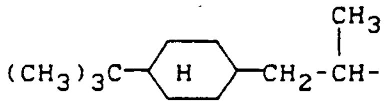 | H | $CH_3-(CH_2)_2-$ | O | $n_D^{20}$ 1,4707 |
| 171 | 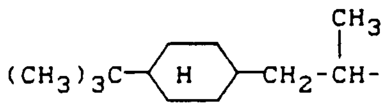 | H | $CH_3-(CH_2)_3-$ | O | $n_D^{20}$ 1,4694 |
| 172 | 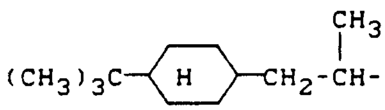 | H | $(CH_3)_2CH-CH_2-$ | O | $n_D^{20}$ 1,4679 |
| 173 | 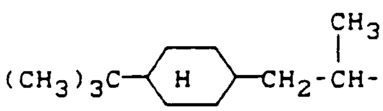 | H | $(CH_3)_3C-$ | O | $n_D^{20}$ 1,4704 |
| 174 | 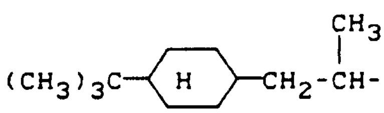 | H | 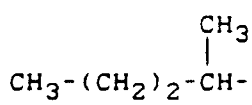 / $CH_3(CH_2)_4-$ (60:40) | O | $n_D^{20}$ 1,4687 |

| Bsp. Nr. | $R^1$ | $R^2$ | $R^3$ | X | physikalische Eigenschaften |
|---|---|---|---|---|---|
| 175 | $(CH_3)_3C$– 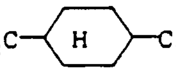 –$CH_2$–$CH(CH_3)$– | H | $CH_3-(CH_2)_5-$ | O | $n_D^{20}$ 1,4685 |
| 176 | 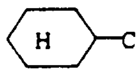 –$CH_2-CH_2-$ | $CH_3$ | $CH_3-(CH_2)_2-$ | O | $n_D^{20}$ 1,4645 |
| 177 | 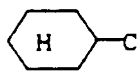 –$CH_2-CH_2-$ | $CH_3$ | $CH_3-(CH_2)_3-$ | O | $n_D^{20}$ 1,4634 |
| 178 | 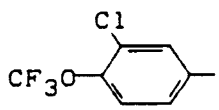 | H | $CH_3-(CH_2)_2-CH(CH_3)-$ / $CH_3-(CH_2)_4-$ (60:40) | O | $n_D^{20}$ 1,4715 |
| 179 | 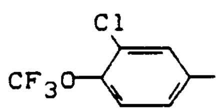 | H | $CH_3-(CH_2)_5-$ | O | $n_D^{20}$ 1,4720 |
| 180 | 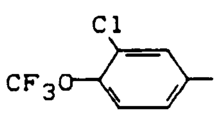 | H | 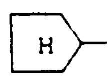 | O | $n_D^{20}$ 1,4886 |
| 181 | 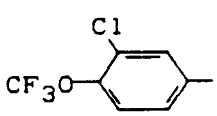 | H | 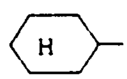 | O | $n_D^{20}$ 1,4905 |

| Bsp. Nr. | $R^1$ | $R^2$ | $R^3$ | X | physikalische Eigenschaften |
|---|---|---|---|---|---|
| 182 | 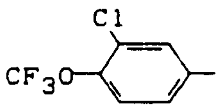 | H | $CH_3-(CH_2)_3-$ | O | $n_D^{20}$ 1,4788 |
| 183 | 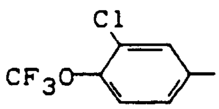 | H | $(CH_3)_2CH-CH_2-$ | O | $n_D^{20}$ 1,4730 |
| 184 | 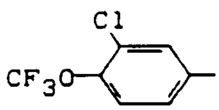 | H | $(CH_3)_3C-$ | O | $^1H$-NMR*): 2,83 |
| 185 | 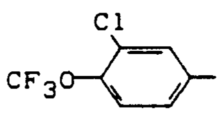 | H | $CH_3-(CH_2)_2-$ | O | $n_D^{20}$ 1,4750 |
| 186 | 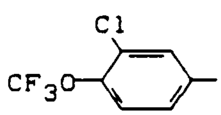 | H | $C_2H_5$ | O | $^1H$-NMR*): 2,75; 2,88 |
| 187 | 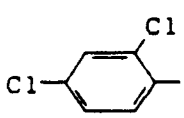 | H | $CH_2=CH-CH_2-$ | O | $n_D^{20}$ 1,5411 |
| 188 | 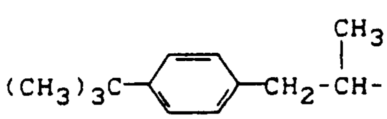 | H | 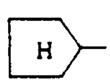 | O | $n_D^{20}$ 1,5096 |

| Bsp. Nr. | $R^1$ | $R^2$ | $R^3$ | X | physikalische Eigenschaften |
|---|---|---|---|---|---|
| 189 | 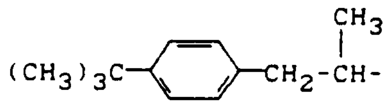 | H | 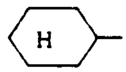 | O | $n_D^{20}$ 1,5096 |
| 190 | 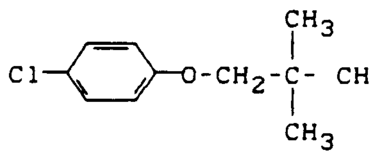 | $CH_3$ | 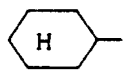 | O | $n_D^{20}$ 1,5184 |
| 191 | 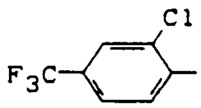 | H | $CH_3$-$(CH_2)_2$- | O | $^1$H-NMR*): 2,6; 2,9 |
| 192 | 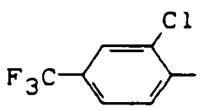 | H | 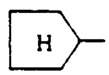 | O | $n_D^{20}$ 1,4950 |
| 193 | 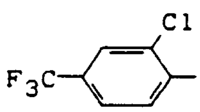 | H | 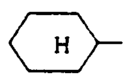 | O | $n_D^{20}$ 1,5942 |
| 194 | 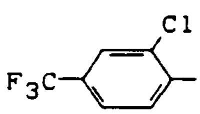 | H | $C_2H_5$ | O | $n_D^{20}$ 1,4842 |

| Bsp. Nr. | $R^1$ | $R^2$ | $R^3$ | X | physikalische Eigenschaften |
|---|---|---|---|---|---|
| 195 | 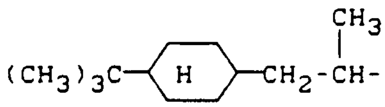 | H | 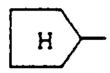 | O | $n_D^{20}$ 1,4823 |
| 196 | 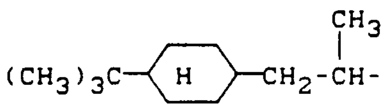 | H | 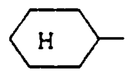 | O | $^1H$-NMR*): 2,45; 2,7 |
| 197 | 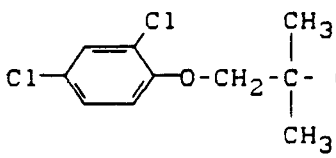 | $CH_3$ | 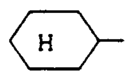 | O | $n_D^{20}$ 1,5260 |
| 198 | 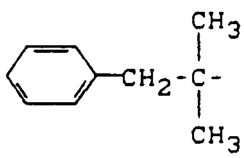 | $CH_3$ | 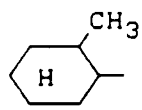 | O | $n_D^{20}$ 1,5142 |
| 199 | 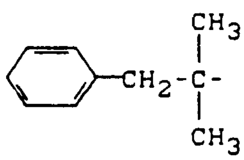 | $CH_3$ | 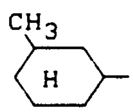 | O | $n_D^{20}$ 1,5069 |
| 200 | 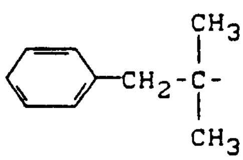 | $CH_3$ | 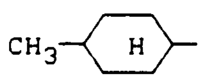 | O | $n_D^{20}$ 1,5062 |

| Bsp. Nr. | $R^1$ | $R^2$ | $R^3$ | X | physikalische Eigenschaften |
|---|---|---|---|---|---|
| 201 | 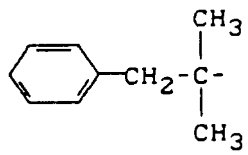 | $CH_3$ | 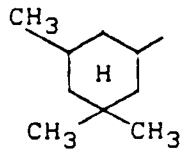 | O | $n_D^{20}$ 1,5041 |
| 202 | 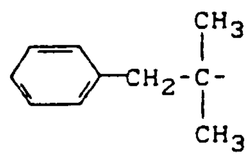 | $CH_3$ | $CH_3O-(CH_2)_2-$ | O | $n_D^{20}$ 1,5028 |
| 203 | 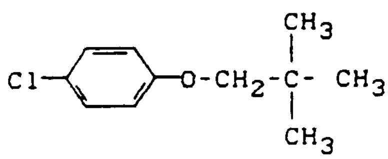 | $CH_3$ | $CH_3$ | O | Kp 146°C/ 0,01 mbar |
| 204 | 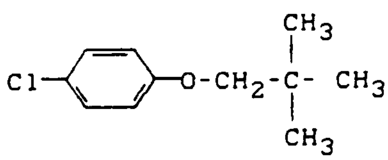 | $CH_3$ | $CH_3$ | S | $n_D^{20}$ 1,5361 |
| 205 | 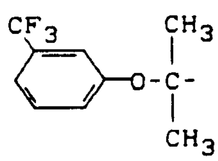 | $CH_3$ | $CH_3$ | O | Kp 125°C/ 0,1 mbar |
| 206 | 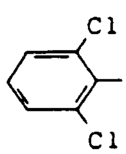 | H | $CH_3-(CH_2)_2-$ | $CH_2$ | Fp 43 °C |

| Bsp. Nr. | $R^1$ | $R^2$ | $R^3$ | X | physikalische Eigenschaften |
|---|---|---|---|---|---|
| 207 | 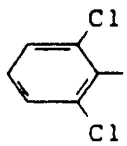 | H | $CH_3-(CH_2)_3-$ | $CH_2$ | Fp 46 °C |
| 208 | 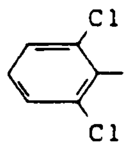 | H | $CH_3-(CH_2)_5-$ | $CH_2$ | $n_D^{20}$ 1,5254 |
| 209 | 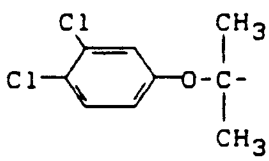 | $CH_3$ | $(CH_3)_3C-$ | O | $n_D^{20}$ 1,5090 |
| 210 | 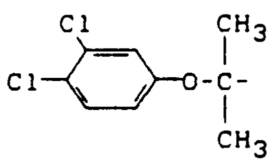 | $CH_3$ | $(C_2H_5)_2CH-CH_2-$ | O | $n_D^{20}$ 1,5100 |
| 211 | 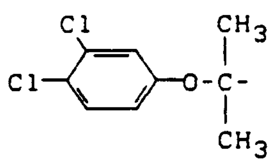 | $CH_3$ | $CH_3O-CH_2-CH_2-$ | O | $n_D^{20}$ 1,5198 |
| 212 | 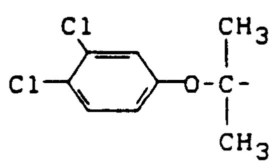 | $CH_3$ | $C_2H_5-O-(CH_2)_3-$ | O | $n_D^{20}$ 1,5131 |

| Bsp. Nr. | $R^1$ | $R^2$ | $R^3$ | X | physikalische Eigenschaften |
|---|---|---|---|---|---|
| 213 | 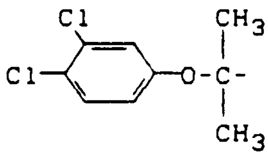 | $CH_3$ | $CH_3O-CH_2-CH_2-$ | O | $n_D^{20}$ 1,5420 |
| | | | | | x 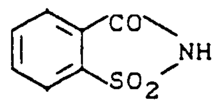 |
| 214 | 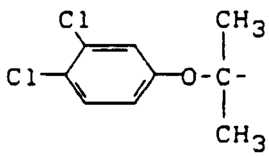 | $CH_3$ | $C_2H_5O-(CH_2)_3-$ | O | $n_D^{20}$ 1,5424 |
| | | | | | x 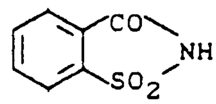 |
| 215 | 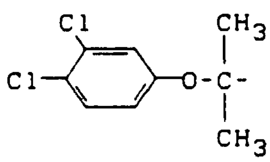 | $CH_3$ | $(C_2H_5)_2CH-CH_2-$ | O | $n_D^{20}$ 1,5448 |
| | | | | | x 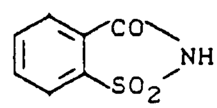 |
| 216 | 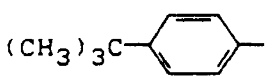 | H | $(CH_3)_2CH-$ | $CH_2$ | $n_D^{20}$ 1,5151 |
| 217 | 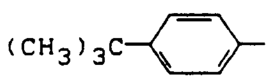 | H | 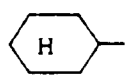 | $CH_2$ | $n_D^{20}$ 1,5243 |

| Bsp. Nr. | $R^1$ | $R^2$ | $R^3$ | X | physikalische Eigenschaften |
|---|---|---|---|---|---|
| 218 | $(CH_3)_3C$ 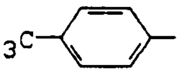 | H | $CH_3-(CH_2)_5-$ | $CH_2$ | $n_D^{20}$ 1,5038 |
| 219 | 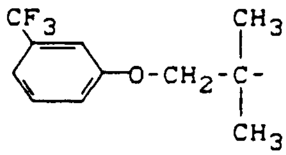 | $CH_3$ | 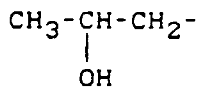 | O | $n_D^{20}$ 1,4648 |
| 220 | 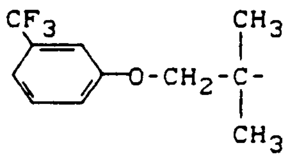 | $CH_3$ | $C_2H_5O-(CH_2)_3-$ | O | $n_D^{20}$ 1,4695 |
| 221 | 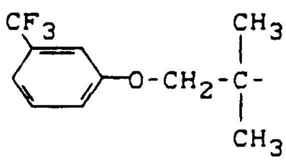 | $CH_3$ | $(CH_3)_2CH-CH_2-$ | O | $n_D^{20}$ 1,4590 |
| 222 | 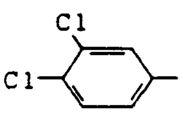 | H | 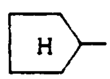 | O | $n_D^{20}$ 1,5456 |
| 223 | 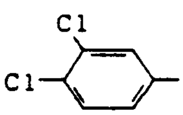 | H | 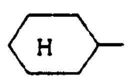 | O | $^1H$-NMR*): |
| 224 | 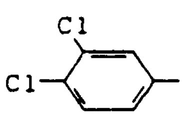 | H | 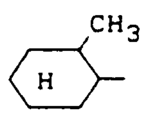 | O | $n_D^{20}$ 1,5374 |

| Bsp. Nr. | $R^1$ | $R^2$ | $R^3$ | X | physikalische Eigenschaften |
|---|---|---|---|---|---|
| 225 | 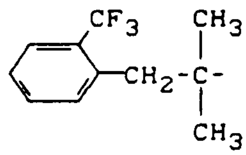 | $CH_3$ | 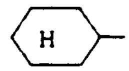 | O | $n_D^{20}$ 1,4909 |
| 226 | 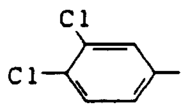 | H | 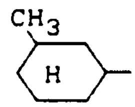 | O | $n_D^{20}$ 1,5345 |
| 227 | 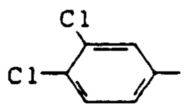 | H | 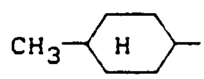 | O | $n_D^{20}$ 1,5323 |
| 228 | 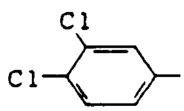 | H | 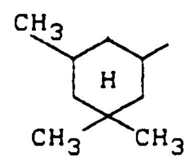 | O | $n_D^{20}$ 1,5267 |
| 229 | 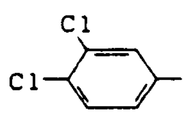 | H | $CH_2-CH=CH_2-$ | O | $n_D^{20}$ 1,5440 |
| 230 | 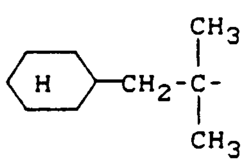 | $CH_3$ | 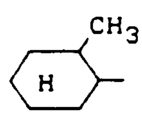 | O | $n_D^{20}$ 1,4846 |

| Bsp. Nr. | $R^1$ | $R^2$ | $R^3$ | X | physikalische Eigenschaften |
|---|---|---|---|---|---|
| 231 | 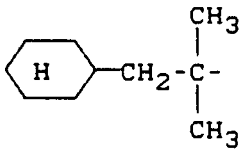 | $CH_3$ | 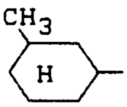 | O | $n_D^{20}$ 1,4836 |
| 232 | 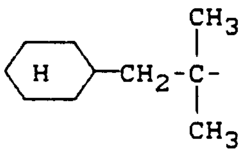 | $CH_3$ | 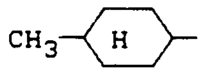 | O | $n_D^{20}$ 1,4836 |
| 233 | 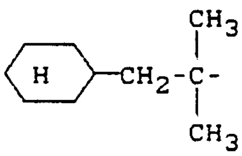 | $CH_3$ | $CH_3O-CH_2-CH_2-$ | O | $n_D^{20}$ 1,4714 |
| 234 | 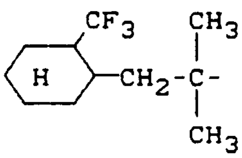 | $CH_3$ | 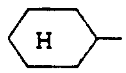 | O | $^1H$-NMR*): |
| 235 | 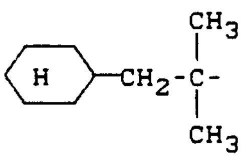 | $CH_3$ | 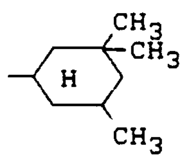 | O | $n_D^{20}$ 1.4824 |
| 236 | 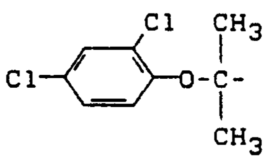 | $CH_3$ | $-CH_2-CH_2-OCH_3$ | O | $n_D^{20}$ 1.5178 |
| 237 | 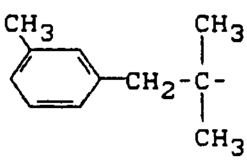 | $CH_3$ | 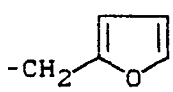 | O | $n_D^{20}$ 1.5266 |

| Bsp. Nr. | $R^1$ | $R^2$ | $R^3$ | X | physikalische Eigenschaften |
|---|---|---|---|---|---|
| 238 | 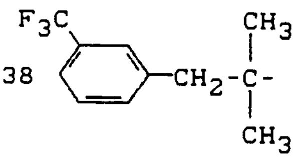 | $CH_3$ | 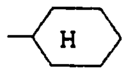 | O | $n_D^{20}$ 1.4849 |
| 239 | 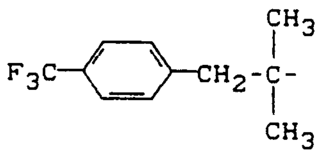 | $CH_3$ | 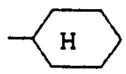 | O | $n_D^{20}$ 1.4860 |
| 240 | 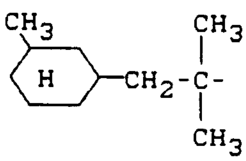 | $CH_3$ | 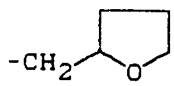 | O | $n_D^{20}$ 1.4803 |
| 241 | 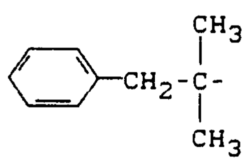 | $CH_3$ | 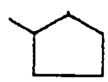 | O | |
| | | | | x | 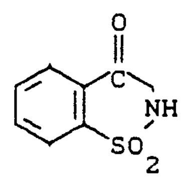 |
| 242 | 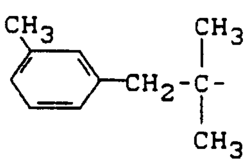 | $CH_3$ | 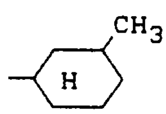 | O | $n_D^{20}$ 1.5090 |
| 243 | 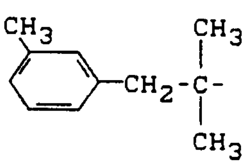 | $CH_3$ | 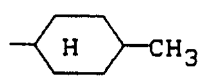 | O | $n_D^{20}$ 1.5080 |

| Bsp. Nr. | $R^1$ | $R^2$ | $R^3$ | X | physikalische Eigenschaften |
|---|---|---|---|---|---|
| 244 | 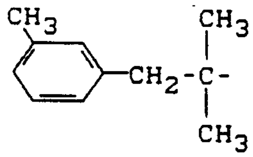 | $CH_3$ | 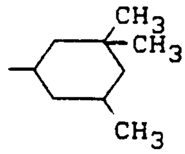 | O | $n_D^{20}$ 1.5056 |
| 245 | 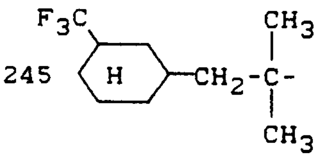 | $CH_3$ | 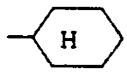 | O | $n_D^{20}$ 1.4631 |
| 246 | 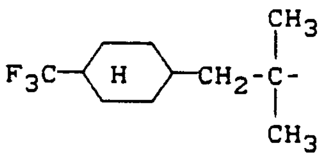 | $CH_3$ | 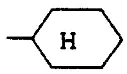 | O | $n_D^{20}$ 1.4644 |
| 247 | 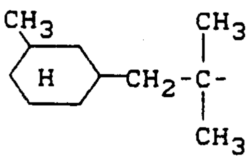 | $CH_3$ | 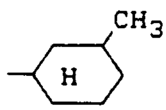 | O | $n_D^{20}$ 1.4814 |
| 248 | 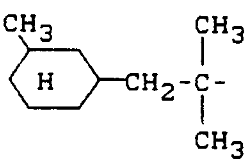 | $CH_3$ | 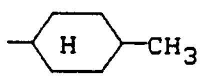 | O | $n_D^{20}$ 1.4807 |
| 249 | 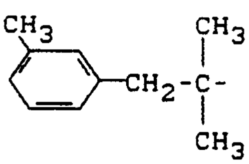 | $CH_3$ | 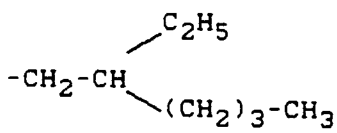 | O | $n_D^{20}$ 1.4916 |
| 250 | 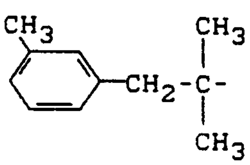 | $CH_3$ | 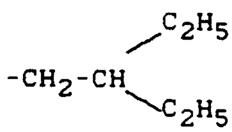 | O | $n_D^{20}$ 1.4960 |

| Bsp. Nr. | $R^1$ | $R^2$ | $R^3$ | X | physikalische Eigenschaften |
|---|---|---|---|---|---|
| 251 | 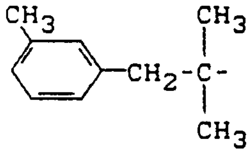 | $CH_3$ | $-(CH_2)_3-OC_2H_5$ | O | $n_D^{20}$ 1.4980 |
| 252 | 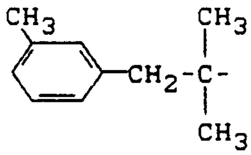 | $CH_3$ | $-(CH_2)_3-O-(CH_2)_3-CH_3$ | O | $n_D^{20}$ 1.4904 |
| 253 | 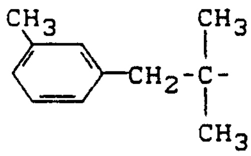 | $CH_3$ | $-(CH_2)_2-OCH_3$ | O | $n_D^{20}$ 1.5026 |
| 254 | 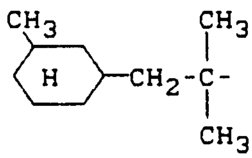 | $CH_3$ | 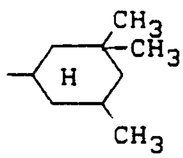 | O | $n_D^{20}$ 1.4809 |
| 255 | 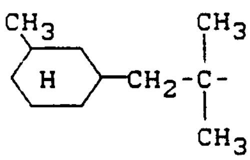 | $CH_3$ | 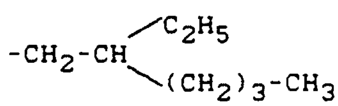 | O | $n_D^{20}$ 1.4683 |
| 256 | 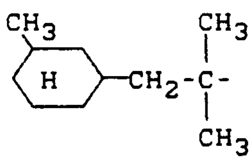 | $CH_3$ | 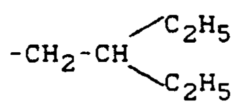 | O | |
| 257 | 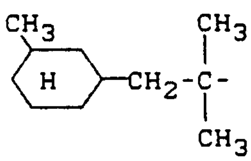 | $CH_3$ | $-(CH_2)_3-OC_2H_5$ | O | $n_D^{20}$ 1.4693 |

| Bsp. Nr. | $R^1$ | $R^2$ | $R^3$ | X | physikalische Eigenschaften |
|---|---|---|---|---|---|
| 258 | 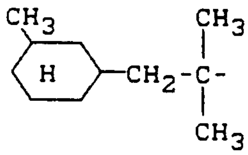 | $CH_3$ | 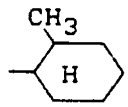 | O | $n_D^{20}$ 1.4826 |
| 259 | 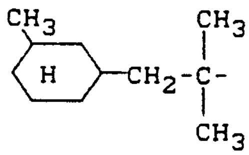 | $CH_3$ | $-(CH_2)_3-O-(CH_2)_3-CH_3$ | O | $n_D^{20}$ 1.4667 |
| 260 | 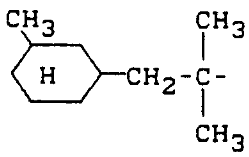 | $CH_3$ | $-(CH_2)_2-OCH_3$ | O | $n_D^{20}$ 1.4690 |
| 261 | 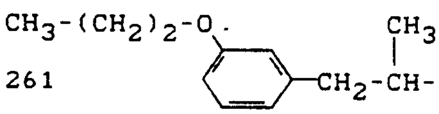 | $CH_3$ | 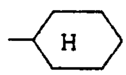 | O | $n_D^{20}$ 1.5094 |
| 262 | 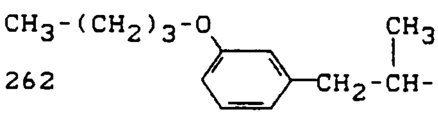 | $CH_3$ | 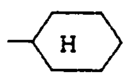 | O | $n_D^{20}$ 1.5072 |
| 263 | 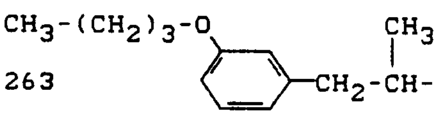 | $CH_3$ | $-C(CH_3)_3$ | O | $n_D^{20}$ 1.4944 |
| 264 | 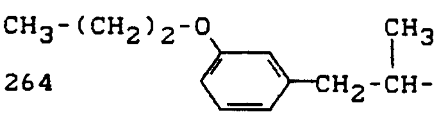 | $CH_3$ | $-C(CH_3)_3$ | O | $n_D^{20}$ 1.4944 |

| Bsp. Nr. | $R^1$ | $R^2$ | $R^3$ | X | physikalische Eigenschaften |
|---|---|---|---|---|---|
| 265 | 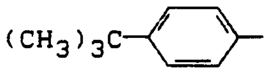 | $CH_3$ | 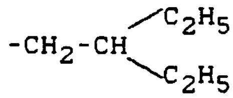 | $-CH_2-$ | $n_D^{20}$ 1.5031 |
| 266 | 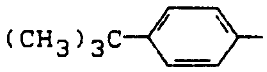 | H | 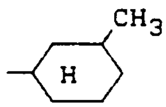 | $-CH_2-$ | $n_D^{20}$ 1.5208 |
| 267 | 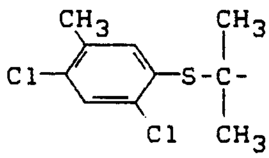 | $CH_3$ | 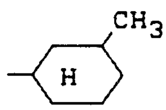 | O | $n_D^{20}$ 1.5310 |
| 268 | 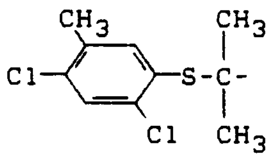 | $CH_3$ | $-(CH_2)_3-OC_2H_5$ | O | $n_D^{20}$ 1.5375 |
| 269 | 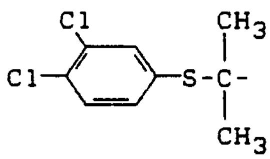 | $CH_3$ | 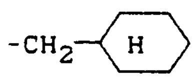 | O | $n_D^{20}$ 1.5369 |
| 270 | 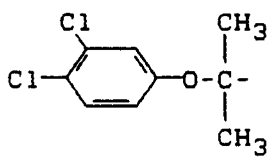 | $CH_3$ | 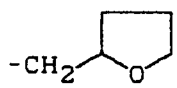 | O | $n_D^{20}$ 1.5228 |
| 271 | 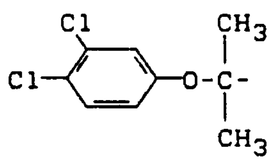 | $CH_3$ | 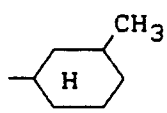 | O | $n_D^{20}$ 1.5211 |

| Bsp. Nr. | $R^1$ | $R^2$ | $R^3$ | X | physikalische Eigenschaften |
|---|---|---|---|---|---|
| 272 | 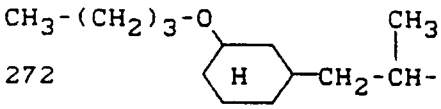 | $CH_3$ | 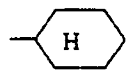 | O | $n_D^{20}$ 1.4777 |
| 273 | 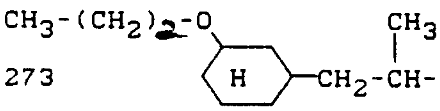 | $CH_3$ | 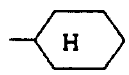 | O | $n_D^{20}$ 1.4773 |
| 274 | 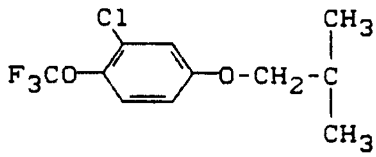 | $CH_3$ | 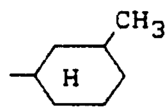 | S | $n_D^{20}$ 1.5039 |
| 275 | 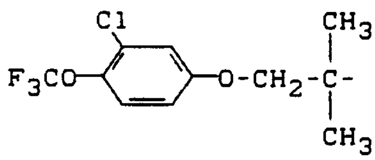 | $CH_3$ | $-CH_2-CH(CH_3)_2$ | S | $n_D^{20}$ 1.4942 |
| 276 | 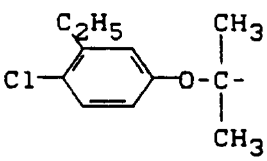 | $CH_3$ | 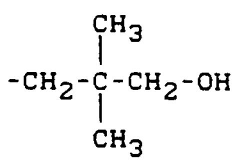 | O | $n_D^{20}$ 1.4979 |
| 277 | 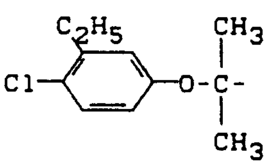 | $CH_3$ | 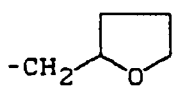 | O | $n_D^{20}$ 1.5087 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurden die nachstehend aufgeführten Verbindungen als Vergleichsubstanzen eingesetzt:

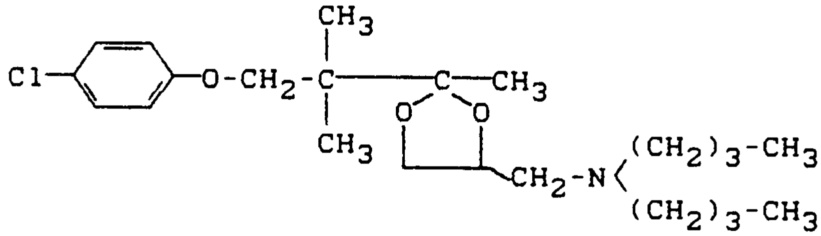

(A)

2-[3-(4-Chlorphenoxy)-2-methyl-prop-2-yl]-2-methyl-4-[N,N-di-(n-butyl)-aminomethyl]-1,3-dioxolan

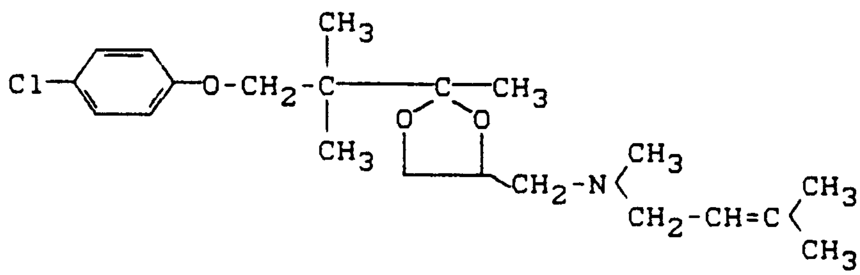

(B)

2-[3-(4-Chlorphenoxy)-2-methyl-prop-2-yl]-2-methyl-4-[N-methyl-N-(3-methylbut-2-en-1-yl)-aminomethyl]-1,3-dioxolan

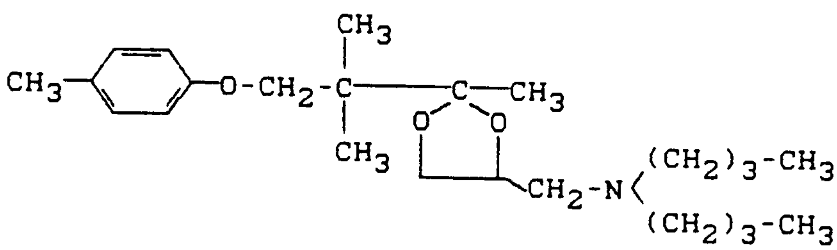

(C)

2-[3-(4-Methylphenoxy)-2-methyl-propy-2-yl]-2-methyl-4-[N,N-di-(n-butyl)-aminomethyl]-1,3-dioxolan (bekannt aus EP 97 822)

Beispiel A

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur

Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 55, 56, 57 und 86.

## Tabelle A

Pyricularia-Test (Reis)/systemisch

| Wirkstoff | Aufwandmenge in mg Wirkstoff pro 100 $cm^2$ | Krankheitsbefall in % der unbehandelten Kontrolle |
|---|---|---|
| 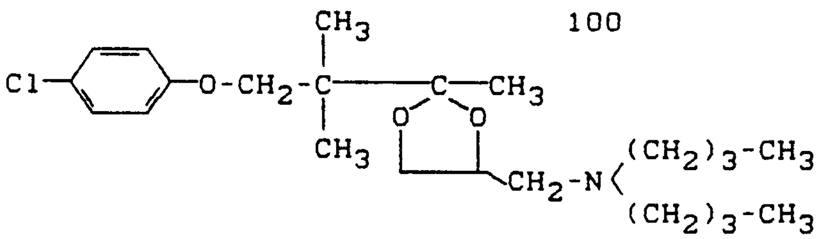 (A) (bekannt) | 100 | 100 |
| 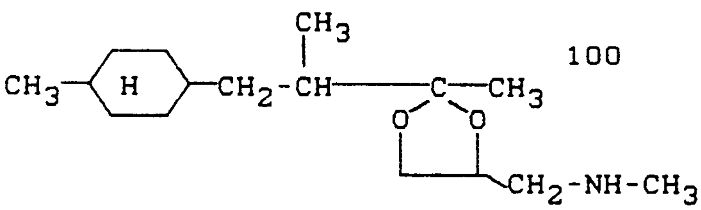 (55) | 100 | 20 |
| 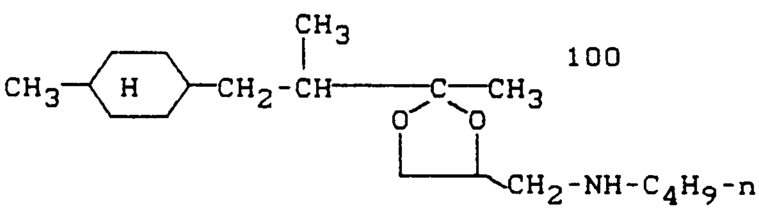 (56) | 100 | 20 |
| 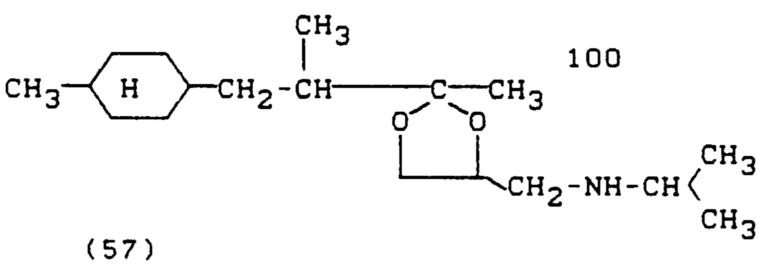 (57) | 100 | 20 |

Tabelle A - Fortsetzung

Pyricularia-Test (Reis)/systemisch

| Wirkstoff | Aufwandmenge in mg Wirkstoff pro 100 $cm^2$ | Krankheitsbefall in % der unbehandelten Kontrolle |
|---|---|---|
| 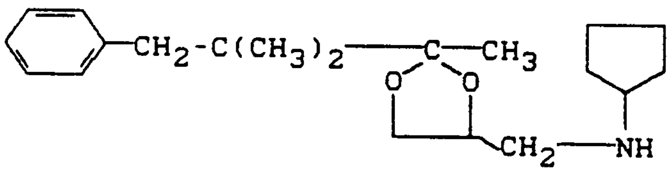 (86) | 100 | 20 |

Beispiel B

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeipsielen: 2, 58, 62, 63, 64, 65, 67, 72, 73, 76, 112, 118, 122, 123, 127, 128.

Tabelle B

Venturia-Test (Apfel)/ protektiv/

| Wirkstoff | Befall in % bei einer Wirkstoffkonzentration von 10 ppm. |
|---|---|
| 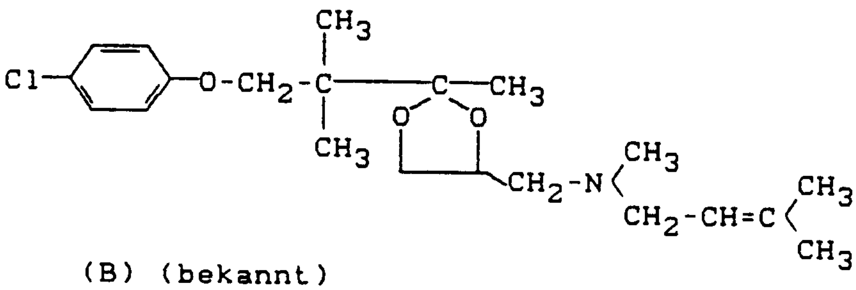 (B) (bekannt) | 63 |
| 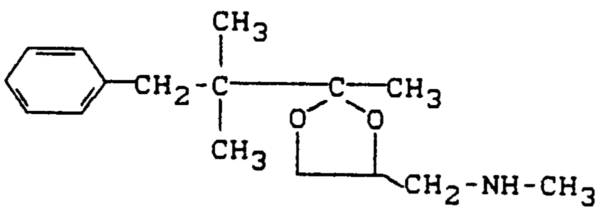 (58) | 16 |
| 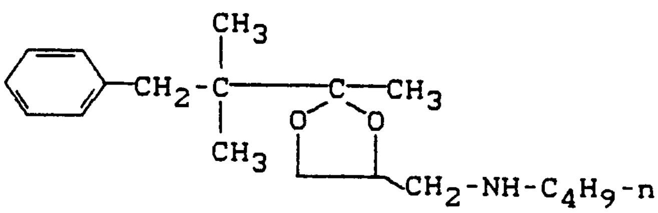 (62) | 9 |
| 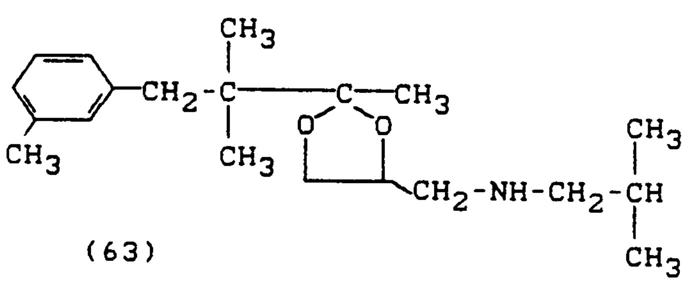 (63) | 11 |

Tabelle B - Fortsetzung

Venturia-Test (Apfel)/ protektiv

| Wirkstoff | Befall in % bei einer Wirkstoffkonzentration von 10 ppm. |
|---|---|
| 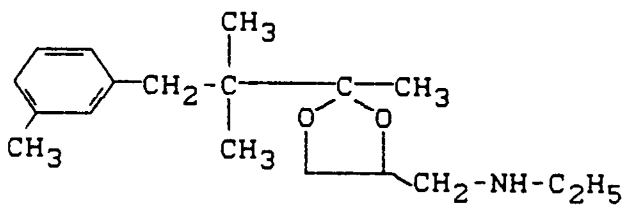 (64) | 6 |
| 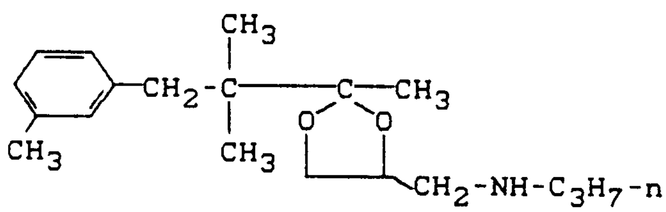 (65) | 25 |
| 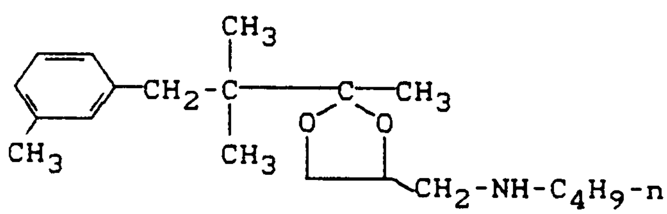 (67) | 25 |
| 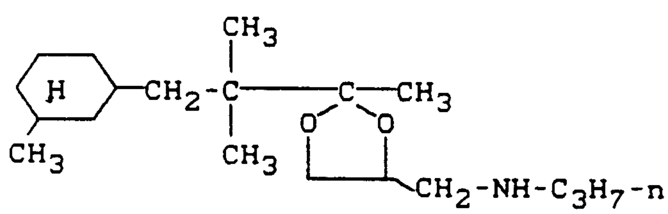 (72) | 28 |

Tabelle B - Fortsetzung

Venturia-Test (Apfel)/ protektiv

| Wirkstoff | Befall in % bei einer Wirkstoffkonzentration von 10 ppm. |
|---|---|
| 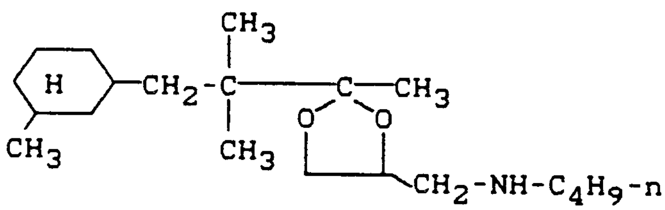 (73) | 23 |
| 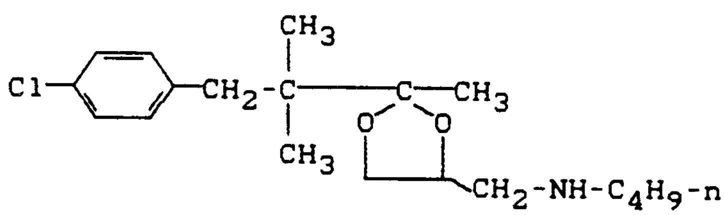 (76) | 25 |
| 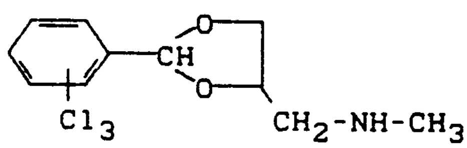 (112) | 20 |
| 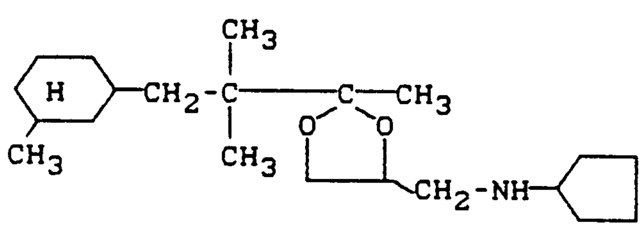 (118) | 16 |

Tabelle B - Fortsetzung

Venturia-Test (Apfel)/ protektiv/

| Wirkstoff | Befall in % bei einer Wirkstoffkonzentration von 10 ppm. |
|---|---|
| 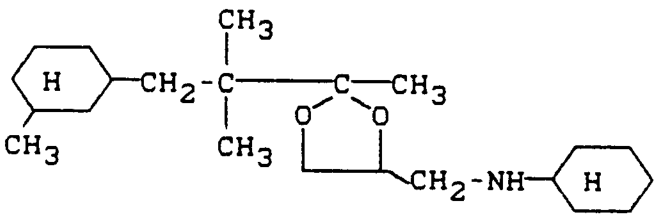 (2) | 9 |
| 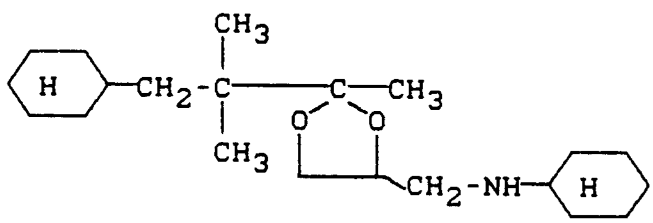 (122) | 14 |
| 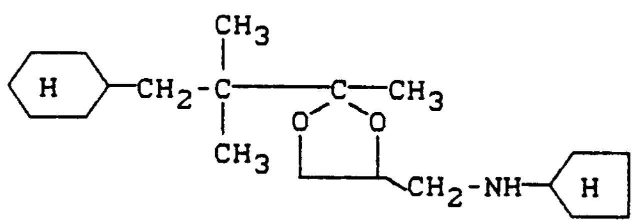 (123) | 40 |
| 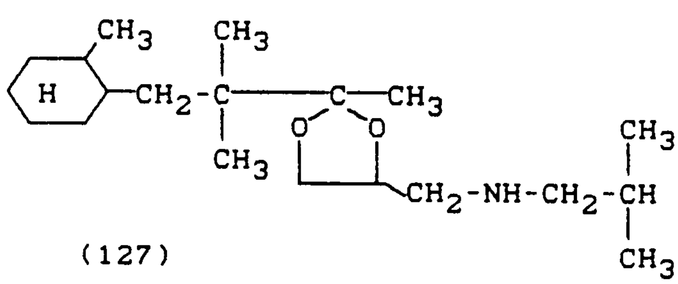 (127) | 23 |

Tabelle B - Fortsetzung

Venturia-Test (Apfel)/ protektiv/

| Wirkstoff | Befall in % bei einer Wirkstoffkonzentration von 10 ppm. |
|---|---|
| 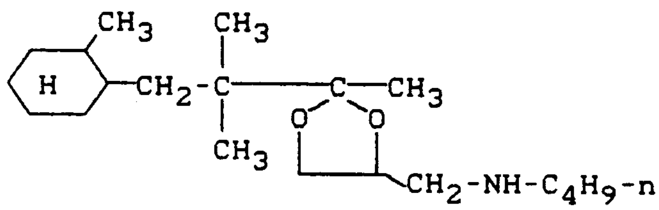 (128) | 9 |

Beispiel C

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 2, 78, 122, 123, 125, 127, 128, 129, 134, 136, 138, 141, 142, 144, 145, 151, 154, 155, 156, 173, 179.

<u>Tabelle C</u>

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 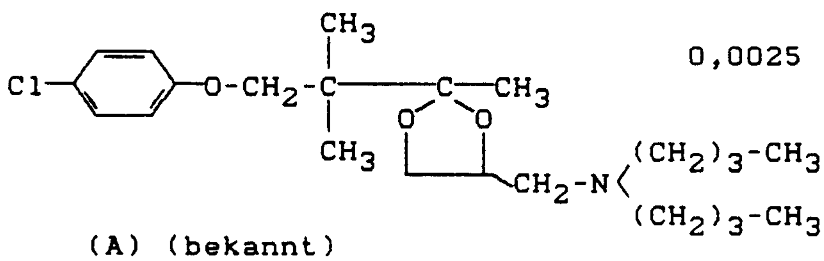 (A) (bekannt) | 0,0025 | 83,8 |
| 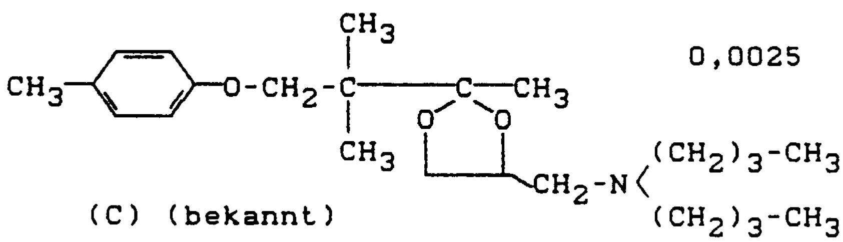 (C) (bekannt) | 0,0025 | 100 |
| 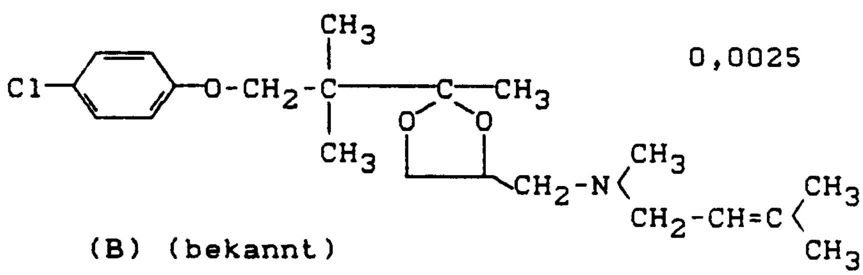 (B) (bekannt) | 0,0025 | 100 |
| 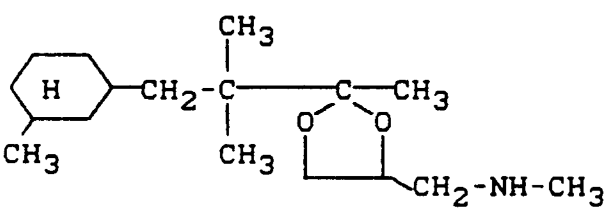 (78) | 0,0025 | 12,5 |

Tabelle C - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 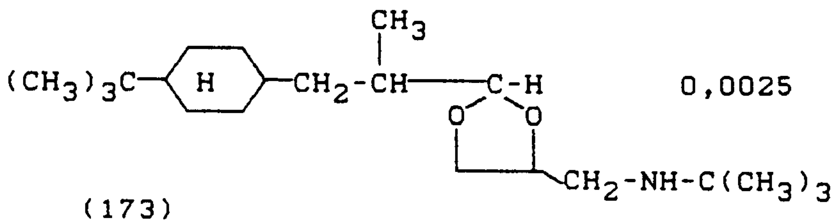 (173) | 0,0025 | 27,4 |
| 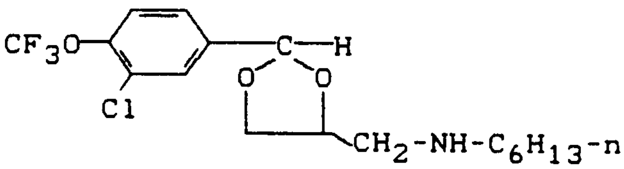 (179) | 0,0025 | 25,0 |
| 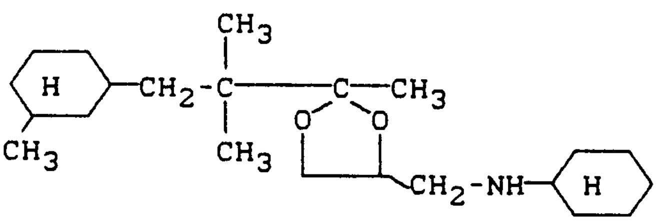 (2) | 0,0025 | 21,2 |
| 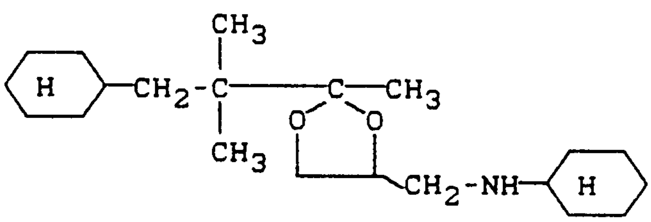 (122) | 0,0025 | 12,5 |

Tabelle C - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 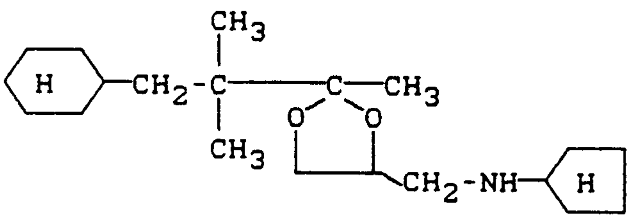 (123) | 0,0025 | 12,5 |
| 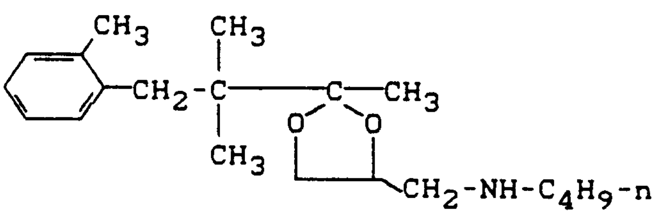 (125) | 0,0025 | 21,2 |
| 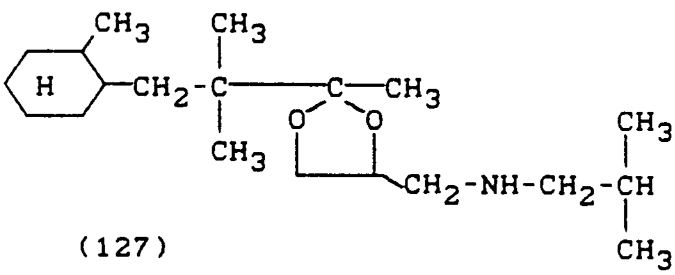 (127) | 0,0025 | 0,0 |
| 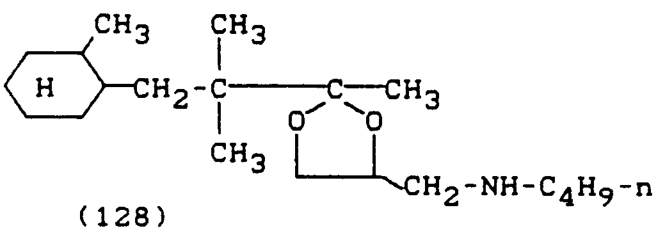 (128) | 0,0025 | 0,0 |

## Tabelle C - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 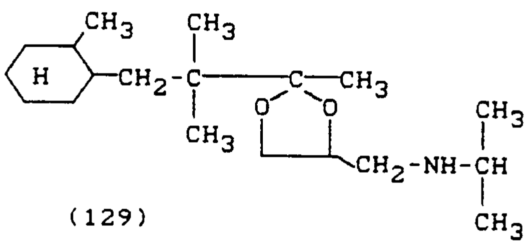 (129) | 0,0025 | 0,0 |
| 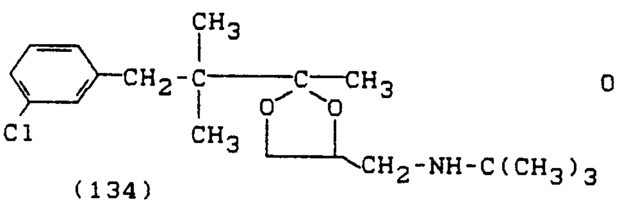 (134) | 0,0025 | 12,5 |
| 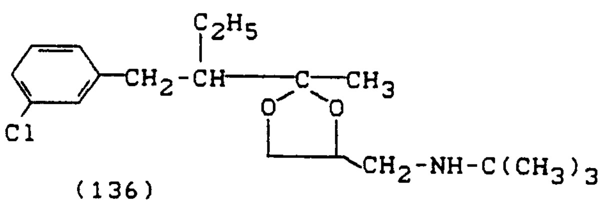 (136) | 0,0025 | 21,2 |
| 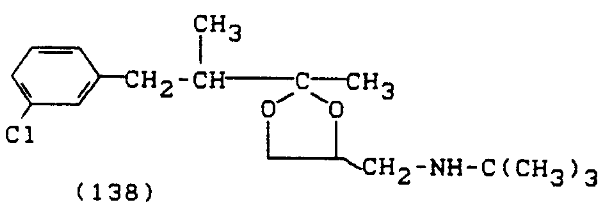 (138) | 0,025 | 25,0 |

Tabelle C - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 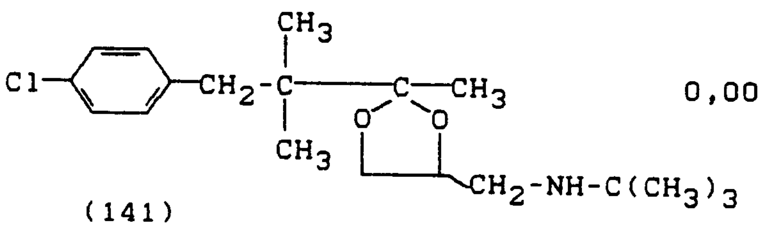 (141) | 0,0025 | 16,2 |
| 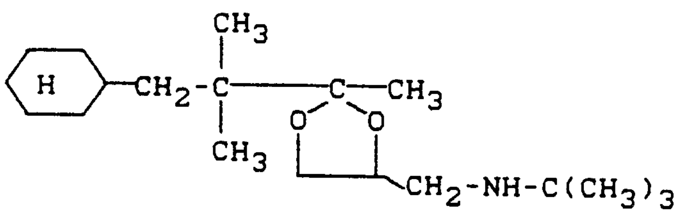 (142) | 0,0025 | 0,0 |
| 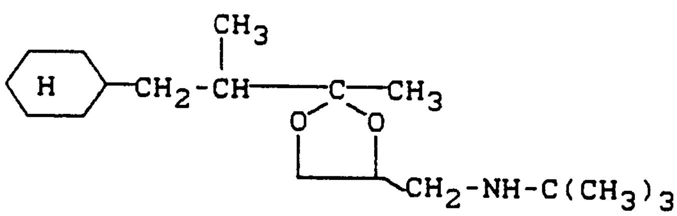 (144) | 0,0025 | 21,2 |
| 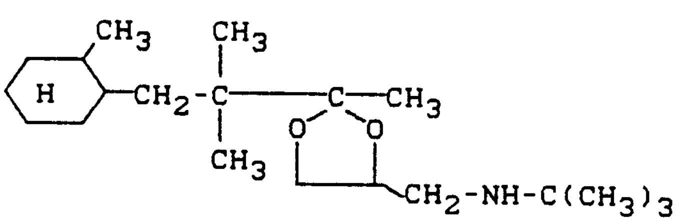 (145) | 0,0025 | 12,5 |

Tabelle C - Fortsetzung

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 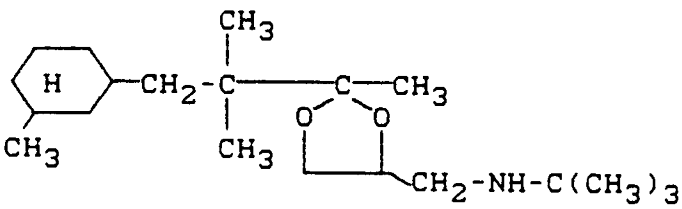 (151) | 0,0025 | 3,7 |
| 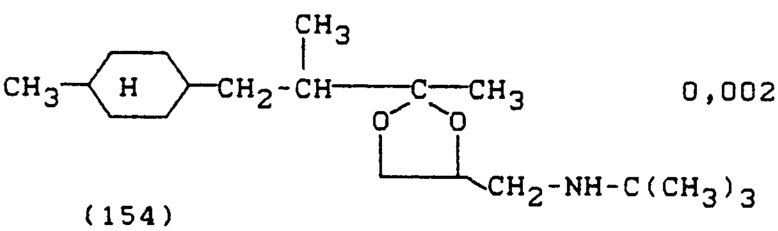 (154) | 0,0025 | 12,5 |
| 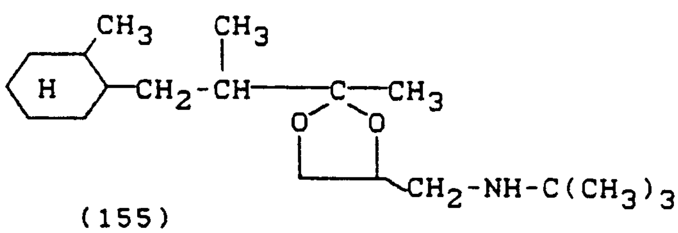 (155) | 0,0025 | 0,0 |
| 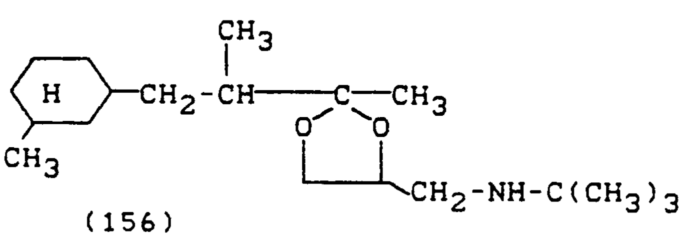 (156) | 0,0025 | 12,5 |

## Patentansprüche

1. Schädlingsbekämpfungsmittel, gekennzeichnet durch einen Gehalt an mindestens einem Aminomethylheterocyclus der allgemeinen Formel (I),

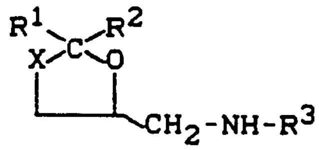

(I)

in welcher

R[1] für Wasserstoff, für Alkyl, für Alkenyl, für jeweils gegebenenfalls substituiertes Tetrahydronaphthyl, Decahydronaphthyl, Cycloalkyl oder Cycloalkenyl steht, ferner für durch Cycloalkyl, Cycloalkenyl, Cycloalyloxy oder Cycloalkylthio substituiertes Alkyl steht, wobei die cyclischen Reste gegenenfalls substituiert sein können, ferner für gegebenenfalls substituiertes Aryl steht, weiterhin für durch Aryl, Aryloxy, Arylthio, Arylsulfinyl oder Arylsulfonyl substituiertes Alkyl oder für durch Aryl substituiertes Alkenyl steht, wobei jeweils die Arylreste gegebenenfalls substituiert sein können;

R[2] für Wasserstoff oder Methyl steht,

R[3] für Alkyl, Alkenyl, Alkinyl, Alkoxyalkyl, Hydroxyalkyl, Hydroxyalkoxyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und

X für Sauerstoff, Schwefel oder eine $CH_2$-Gruppe steht,

sowie deren Säureadditionssalze, Isomere und Isomerengemische.

**2.** Schädingsbekämpfungsmittel gemäß Anspruch 1, wobei in der Formel (I)

R[1] für Wasserstoff; für jeweils geradkettiges oder verzweigtes Alkyl mit 1 bis 18 Kohlenstoffatomen oder Alkenyl mit 3 bis 12 Kohlenstoffatomen steht;
außerdem für jeweils gegebenenfalls einfach bis mehrfach, gleich oder verschieden substituiertes Tetrahydronaphthyl, Decahydronaphthyl, Cycloalkyl, Cycloalkenyl, Cycloalkylalkyl, Cycloalkenylalkyl, Cycloalkyloxyalkyl oder Cycloalkylthioalkyl mit jeweils gegebenenfalls 5 bis 7 Kohlenstoffatomen in den Cycloalkyl- bzw. Cycloalkenylteilen und gegebenenfalls 1 bis 6 Kohlenstoffatomen in den geradkettigen oder verzweigten Alkylteilen steht, wobei als Substituenten jeweils infrage kommen: jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 9 gleichen oder verschiedenen Halogenatomen; und schließlich für jeweils gegebenenfalls einfach bis mehrfach, gleich oder verschieden substituiertes Aryl, Aralkyl, Aryloxyalkyl, Arylthioalkyl, Arylsulfinylalkyl, Arylsulfonylalkyl oder Arylalkenyl mit jeweils 6 bis 10 Kohlenstoffatomen im Arylteil und gegebenenfalls bis zu 6 Kohlenstoffatomen in den geradkettigen oder verzweigten Alkyl- bzw. Alkenylteilen steht, wobei als Arylsubstituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Cyclohexyl oder Phenyl;

R[2] für Wasserstoff oder Methyl steht,

R[3] für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen; für jeweils gegebenenfalls im Cycloalkylteil einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen; und außerdem für jeweils gegebenenfalls einfach bis mehrfach gleich oder verschieden im Arylteil substituiertes Aryl, Arylalkyl oder Arylalkenyl mit jeweils 6 bis 10 Kohlenstoffatomen im Arylteil und gegebenenfalls bis zu 6 Kohlenstoffatomen im jeweils geradkettigen oder verzweigten Alkyl-bzw. Alkenylteil steht, wobei als Arylsubstituenten jeweils infrage kommen:
Halogen, Cyano, Nitro sowie jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen; oder für jeweils geradkettiges oder verzweigtes Furanylalkyl, Tetrahydrofuranylalkyl, Tetrahydropyranylalkyl, Dioxolanylalkyl oder Dioxanylalkyl mit jeweils 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gebenenfalls 1 bis 9 gleichen oder verschiedenen Halogenatomen und

X für Sauerstoff, Schwefel oder eine $CH_2$-Gruppe steht.

**3.** Schädlingsbekämpfungsmittel gemäß Anspruch 1, wobei in der Formel (I)

$R^1$ für Wasserstoff; für jeweils geradkettiges oder verzweigtes Alkyl mit 1 bis 12 Kohlenstoffatomen oder Alkenyl mit 3 bis 8 Kohlenstoffatomen oder für jeweils gegebenenfalls ein- bis dreifach, gleich oder verschieden durch geradkettiges oder verzweigtes Alkyl mit 1 bis 4 Kohlenstoffatomen substituiertes Tetrahydronaphthyl, Decahydronaphthyl, Cyclohexyl oder Cyclohexenyl steht; außerdem für einen

Rest

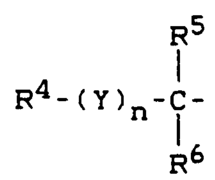

oder für einen Rest

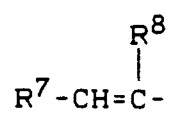

steht,
wobei

$R^4$ für jeweils gegebenenfalls ein- bis dreifach, gleich oder verschieden substituiertes Cyclohexyl, Cyclohexenyl oder Phenyl steht, wobei als Cyclohexyl- bzw. Cyclohexenylsubstituenten infrage kommen: jeweils geradkettiges oder verzweigtes Alkyl, Alkoxy oder Halogenalkyl mit jeweils 1 bis 4 Kohlenstoffatomen und gegebenenfalls 1 bis 9 gleichen oder verschiedenen Halogenatomen und wobei als Phenylsubstituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, Cyclohexyl oder Phenyl,

Y für Sauerstoff, Schwefel, eine $-CH_2$-Gruppe, eine $-O-CH_2$-Gruppe, eine $-S-CH_2$-Gruppe, eine Sulfinylgruppe, eine Sulfonylgruppe, eine

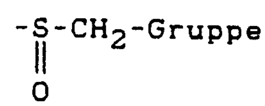

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff oder Methyl steht,

$R^7$ für gegebenenfalls ein- bis dreifach, gleich oder verschieden substituiertes Phenyl steht, wobei als Phenylsubstituenten die bei $R^4$ genannten infrage kommen und

$R^8$ für Methyl oder Ethyl steht,

$R^1$ außerdem für jeweils gegebenenfalls ein- bis dreifach, gleich oder verschieden substituiertes Phenyl oder Naphthyl steht, wobei als Substituenten jeweils infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen, Cyclohexyl oder Phenyl;

$R^2$ für Wasserstoff oder Methyl steht,

$R^3$ für Methyl, Ethyl, n- oder i-Propyl, n-, i-, s-oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für jeweils gegebenenfalls ein-bis fünffach, gleich oder verschieden durch Fluor, Chlor, Brom, Methyl, Ethyl, n- oder i-Propyl, n-, i-, s- oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl steht oder für jeweils gegebenenfalls ein- bis dreifach, gleich oder verschieden substituiertes Phenyl, Benzyl oder Phenylethyl steht, wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethoxy, Trifluormethylthio, Trifluormethyl, Methoxycarbonyl, Ethoxycarbonyl oder Methoximinomethyl;
oder für Furanylmethyl, Tetrahydrofuranylmethyl, Tetrahydropyranylmethyl, Dioxolanylmethyl, Dioxolanylethyl, Dioxanylmethyl oder Dioxanylethyl steht und

X für Sauerstoff, Schwefel oder eine $CH_2$-Gruppe steht.

**4.** Schädlingsbekämpfungsmittel gemäß Anspruch 1, wobei in der Formel (I)

$R^1$ für jeweils gegebenenfalls ein- bis dreifach, gleich oder verschieden durch Methyl oder t-Butyl substituiertes Tetrahydronaphthyl, Decahydronaphthyl, Cyclohexyl oder Cyclohexenyl steht, oder für einen Rest

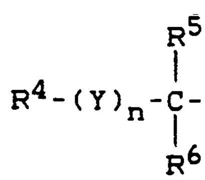

oder einen Rest

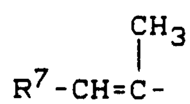

steht,

wobei

$R^4$ für jeweils gegebenenfalls ein- bis dreifach, gleich oder verschieden durch Methyl, i-Propyl, t-Butyl oder Trifluormethyl substituiertes Cyclohexyl oder Cyclohexenyl steht oder für gegebenenfalls ein- bis dreifach, gleich oder verschieden durch Fluor, Chlor, Brom, Methyl, i-Propyl, t-Butyl, Methoxy, Propoxy, Butoxy, Trifluormethyl, Trifluormethoxy, Trifluormethylthio, Methoxy, Phenyl oder Cyclohexyl substituiertes Phenyl steht,

Y für Sauerstoff, Schwefel, eine $-CH_2$-Gruppe, eine $-O-CH_2$-Gruppe oder eine $-S-CH_2$-Gruppe steht,

n für eine Zahl 0 oder 1 steht,

$R^5$ für Methyl oder Ethyl steht,

$R^6$ für Wasserstoff oder Methyl steht und

$R^7$ für gegebenenfalls ein- bis dreifach, gleich oder verschieden durch Fluor, Chlor, Methyl, Isopropyl, t-Butyl, Trifluormethyl, Trifluormethoxy, Trifluormethylthio, Methoxy, Phenyl oder Cyclohexyl substituiertes Phenyl steht,

$R^1$ außerdem für jeweils gegebenenfalls ein- bis dreifach, gleich oder verschieden durch Fluor, Chlor, Brom, Methyl, i-Propyl, t-Butyl Trifluormethyl, Trifluormethoxy, Trifluormethylthio, Methoxy, Phenyl oder Cyclohexyl substituiertes Phenyl oder Naphthyl steht,

$R^2$ für Wasserstoff oder Methyl steht,

$R^3$ für Methyl, Ethyl, n- oder i-Propyl, n-, i-, s- oder t-Butyl, n- oder i-Pentyl, n- oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Dichlorcyclopropylmethyl, Dimethylcyclopropylmethyl, Dichlordimethylcyclopropylmethyl, Cyclopentyl oder für jeweils gegebenenfalls ein- bis dreifach durch Methyl substituiertes Cyclohexyl oder Cyclohexylmethyl oder für Furanylmethyl, Tetrahydrofuranylmethyl, Tetrahydropyranylmethyl, Dioxolanylmethyl, Dioxolanylethyl oder Dioxanylmethyl steht und

X für Sauerstoff, Schwefel oder eine $CH_2$-Gruppe steht.

**5.** Verwendung von substituierten Aminomethylheterocyclen der Formel (I) gemäß Anspruch 1 zur Bekämpfung von Schädlingen.

**6.** Verfahren zur Herstellung von Schädlingsbekämpfungsmitteln zur Bekämpfung von Schädlingen, dadurch gekennzeichnet, daß man substituierte Aminomethylheterocyclen der Formel (I) gemäß Anspruch 1 mit Streckmitteln und/oder oberflächenaktiven Stoffen vermischt.

**7.** Substituierte Aminomethylheterocyclen der Formel (Ia),

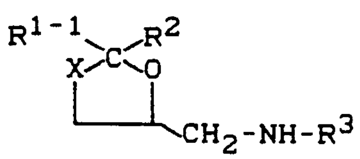

(Ia)

in welcher

$R^{1-1}$ für substituiertes Phenyl, für jeweils gegebenenfalls substituiertes Cycloalkyl, Cycloalkenyl, Tetrahydronaphthyl, Decahydronaphthyl oder Naphthyl, für einen Rest

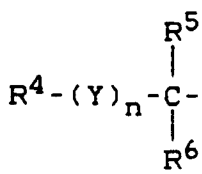

oder für einen Rest

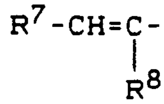

steht,

wobei

$R^4$ für jeweils gegebenenfalls substituiertes Cyclohexyl, Cyclohexenyl oder Phenyl steht,

Y für Sauerstoff, Schwefel, eine $-CH_2$-Gruppe eine $-O-CH_2$-Gruppe, eine $-S-CH_2$-Gruppe, eine

Sulfinylgruppe, eine Sufonylgruppe, eine

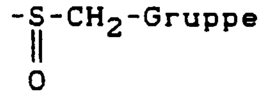

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff, Methyl oder Ethyl steht

$R^7$ für gegebenenfalls ein- bis dreifach, gleich oder verschieden substituiertes Phenyl steht,

$R^8$ für Methyl oder Ethyl steht;

$R^2$ für Wasserstoff oder Methyl steht,

$R^3$ für Alkyl, Alkenyl, Alkinyl, Alkoxyalkyl, Hydroxyalkyl, Hydroxyalkyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und

X für Sauerstoff, Schwefel oder eine $CH_2$-Gruppe steht,

deren Säureadditonssalze, deren Isomere oder Isomerengemische.

**8.** Substituierte Aminomethylheterocyclen gemäß Anspruch 7, wobei in der Formel (Ia)

$R^{1-1}$ für einfach bis mehrfach, gleich oder verschieden substituiertes Phenyl oder für gegebenenfalls einfach bis mehrfach, gleich oder verschieden substituiertes Naphthyl steht, wobei als Substituenten jeweils infrage kommen; Halogen, Cyano, Nitro, jeweils geradkettiges oder verzweigtes Alkyl, Alkoxy, Alkylthio, Halogenalkyl, Halogenalkoxy, Halogenalkylthio mit jeweils 1 bis 6 Kohlenstoffatomen und gegebenenfalls 1 bis 9 gleichen oder verschiedenen Halogenatomen, Cyclohexyl oder Phenyl;

außerdem für jeweils gegebenenfalls einfach bis mehrfach, gleich oder verschieden substituiertes Cycloalkyl oder Cycloalkenyl mit jeweils 5 bis 7 Kohlenstoffatomen, Tetrahydronaphthyl oder Decahydronaphthyl steht, wobei als Substituenten jeweils infrage kommen: jeweils geradkettiges oder verzweigtes Alkyl, Halogenalkyl oder Halogenalkoxy mit jeweils 1 bis 6 Kohlenstoffatomen und gegebenenfalls 1 bis 9 gleichen oder verschiedenen Halogenatomen; außerdem für einen Rest

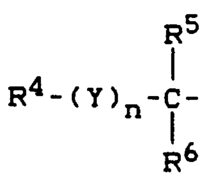

oder für einen Rest

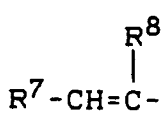

steht,

wobei

$R^4$ für jeweils gegebenenfalls ein- bis dreifach, gleich oder verschieden substituiertes Cyclohexyl, Cyclohexenyl oder Phenyl steht, wobei als Cyclohexyl- bzw. Cyclohexenylsubstituenten infrage kommen: jeweils geradkettiges oder verzweigtes Alkyl, Alkoxy oder Halogenalkyl mit jeweils 1 bis 4 Kohlenstoffatomen und gegenenfalls 1 bis 9 Halogenatomen und wobei als Phenylsubstituenten infrage kommen: Halogen, Cyano, Nitro, jeweils geradkettiges oder

verzweigtes Alkyl, Alkoxy, Alkylthio, Halogenalkyl, Halogenalkoxy, oder Halogenalkylthio mit jeweils 1 bis 4 Kohlenstoffatomen und gegebenenfalls 1 bis 9 gleichen oder verschiedenen Halogenatomen, Cyclohexyl oder Phenyl,

Y für Sauerstoff, Schwefel, eine $-CH_2$-Gruppe, eine $-O-CH_2$-Gruppe, eine $-S-CH_2$-Gruppe, eine Sulfinylgruppe, eine Sulfonylgruppe, eine

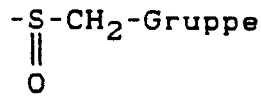

oder eine $-SO_2-CH_2$-Gruppe steht,

n für eine Zahl 0 oder 1 steht,

$R^5$ für Wasserstoff, Methyl oder Ethyl steht

$R^6$ für Wasserstoff oder Methyl steht,

$R^7$ für gegebenenfalls ein- bis dreifach, gleich oder verschieden substituiertes Phenyl steht, wobei als Phenylsubstituenten die bei $R^4$ genannten infrage kommen und

$R^8$ für Methyl oder Ethyl steht,

$R^2$ für Wasserstoff oder Methyl steht,

$R^3$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, oder für jeweils gegebenenfalls im Cycloalkylteil einfach oder mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten jeweils infrage kommen:
Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen;
außerdem für jeweils gegebenenfalls einfach bis mehrfach, gleich oder verschieden im Arylteil substituiertes Aryl, Arylalkyl oder Arylalkenyl mit jeweils 6 bis 10 Kohlenstoffatomen im Arylteil und gegebenenfalls bis zu 6 Kohlenstoffatomen im jeweils geradkettigen oder verzweigten Alkyl- bzw. Alkenylteil steht, wobei als Arylsubstituenten jeweils infrage kommen:
Halogen, Cyano, Nitro sowie jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder für jeweils geradkettiges oder verzweigtes Furanylalkyl, Tetrahydrofuranylalkyl, Tetrahydropyranylalkyl, Dioxolanylalkyl oder Dioxanylalkyl mit jeweils 1 bis 4 Kohlenstoffatomen im Alkylteil steht und

X für Sauerstoff, Schwefel oder eine $CH_2$-Gruppe steht,

**9.** Substituierte Aminomethylheterocylen gemäß Anspruch 7, wobei in der Formel (I)

$R^{1-1}$ für ein- bis dreifach, gleich oder verschieden substituiertes Phenyl oder für gegebenenfalls ein- bis dreifach, gleich oder verschieden substituiertes Naphthyl steht, wobei als Substituenten jeweils infrage kommen: Fluor, Chlor, Brom, Methyl, i-Propyl, t-Butyl, Trifluormethyl, Trifluormethoxy, Trifluormethylthio, Methoxy, Phenyl oder Cyclohexyl; außerdem für jeweils gegebenenfalls ein- bis dreifach, gleich oder verschieden substituiertes Cyclohexyl, Cyclohexenyl, Tetrahydronaphthyl oder Decahydronaphthyl steht, wobei als Substituenten jeweils infrage kommen: geradkettiges oder verzweigtes Alkyl mit 1 bis 4 Kohlenstoffatomen;
außerdem für einen Rest

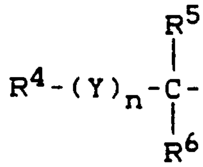

oder für einen Rest

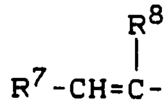

steht,

wobei

$R^4$ für jeweils gegebenenfalls ein- bis dreifach, gleich oder verschieden durch Methyl, i-Propyl, t-Butyl, Methoxy, Propoxy, Butoxy oder Trifluormethyl substituiertes Cyclohexyl oder Cyclohexenyl steht oder für gegebenenfalls ein- bis dreifach, gleich oder verschieden durch Fluor, Chlor, Brom, Methyl, i-Propyl, t-Butyl, Trifluormethyl, Trifluormethoxy, Trifluormethylthio, Methoxy, Phenyl oder Cyclohexyl substituiertes Phenyl steht,

Y für Sauerstoff, Schwefel, eine $-CH_2$-Gruppe, eine $-O-CH_2$-Gruppe oder eine $-S-CH_2$-Gruppe steht,

n für eine Zahl 0 oder 1 steht,

$R^6$ für Wasserstoff oder Methyl steht und

$R^7$ für gegebenenfalls ein- bis dreifach, gleich oder verschieden durch Fluor, Chlor, Methyl, Isopropyl, t-Butyl, Trifluormethyl, Trifluormethoxy, Trifluormethylthio, Methoxy, Phenyl oder Cyclohexyl substituiertes Phenyl steht,

$R^8$ für Methyl oder Ethyl steht,

$R^2$ für Wasserstoff oder Methyl steht,

$R^3$ für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, für jeweils gegebenenfalls ein- bis fünffach, gleich oder verschieden durch Fluor, Chlor, Brom, Methyl, Ethyl, n- oder i-Propyl, n-, i-, s- oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl steht, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl; oder für Furanylmethyl, Tetrahydrofuranylmethyl, Tetrahydropyranylmethyl, Dioxolanylmethyl, Dioxolanylethyl, Dioxanylmethyl oder Dioxanylethyl steht und

X für Sauerstoff, Schwefel oder eine $CH_2$-Gruppe steht.

**10.** Verfahren zur Herstellung von substituierten Aminomethylheterocyclen der Formel (Ia),

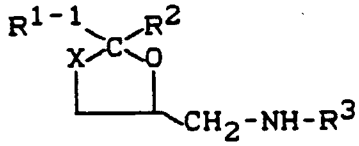

(Ia)

in welcher

$R^{1-1}$ für substituiertes Phenyl, für jeweils gegebenenfalls substituiertes Cycloalkyl, Cycloalkenyl, Tetrahydronaphthyl, Decahydronaphthyl oder Naphthyl, für einen Rest

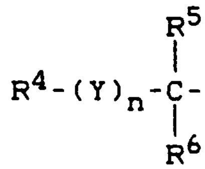

oder für einen Rest

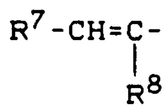

steht,

wobei

$R^4$ für jeweils gegebenenfalls substituiertes Cyclohexyl, Cyclohexenyl oder Phenyl steht,

Y für Sauerstoff, Schwefel, eine $-CH_2$-Gruppe eine $-O-CH_2$-Gruppe, eine $-S-CH_2$-Gruppe, eine Sulfinylgruppe, eine Sufonylgruppe, eine

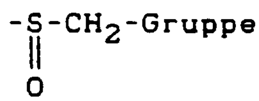

oder eine $-SO_2-CH_2$-Gruppe steht,

n für eine Zahl 0 oder 1 steht,

$R^5$ für Wasserstoff, Methyl oder Ethyl steht,

$R^6$ für Wasserstoff, Methyl oder Ethyl steht,

$R^7$ für gegebenenfalls ein- bis dreifach, gleich oder verschieden substituiertes Phenyl steht und

$R^8$ für Methyl oder Ethyl steht,

$R^2$ für Wasserstoff oder Methyl steht,

$R^3$ für Alkyl, Alkenyl, Alkinyl, Alkoxyalkyl, Hydroxyalkyl, Hydroxyalkoxyalkyl, Dialkoxyalkyl, für jeweils gegebenenfalls substituiertes Cycloalkyl, Cycloalkylalkyl, Aryl, Aralkyl, Aralkenyl, Furanylalkyl, Tetrahydrofuranylalkyl, Tetrahydropyranylalkyl, Dioxolanylalkyl oder Dioxanylalkyl steht und

X für Sauerstoff, Schwefel oder eine $CH_2$ Gruppe steht,

deren Säureadditionssalze, deren Isomere oder Isomerengemische, dadurch gekennzeichnet, daß man

(a) heterocyclische Verbindungen der Formel (II),

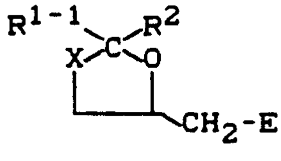

(II)

in welcher

$R^{1-1}$ und $R^2$ die oben angegebene Bedeutung haben und

E für eine elektronenanziehende Abgangsgruppe steht,

mit Aminen der Formel (III),

$H_2N\text{-}R^3$ (III)

in welcher
$R^3$ die oben angegebene Bedeutung hat
gegebenenfalls in Gegenwart eines Verdünnungsmittels und gegebenenfalls in Gegenwart eines Säurebindemittels umsetzt oder wenn man
(b) die mit Hilfe des Verfahrens (a) erhältichen substituierten Aminomethylheterocyclen der Formel (Ia-1),

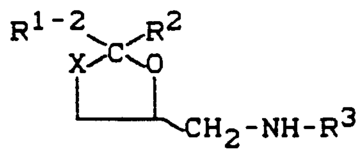

(Ia-1)

in welcher
$R^{1-2}$ für substituiertes Phenyl, für gegebenenfalls substituiertes Naphthyl, für einen Rest

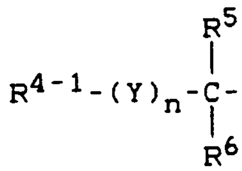

oder für einen Rest

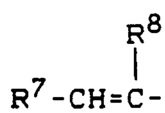

steht,
X, $R^2$, $R^3$, $R^5$, $R^6$, $R^7$ und $R^8$ die oben angegebene Bedeutung haben und
$R^{4-1}$ für gegebenenfalls substituiertes Phenyl steht,
mit Wasserstoff in Gegenwart eines Katalysators und gegebenenfalls in Gegenwart eines Verdünnungsmittels hydriert.

## Claims

1. Pesticides, characterized in that they contain at least one aminomethylheterocyclic compound of the general formula (I)

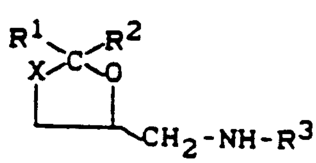

(I)

in which
$R^1$ represents hydrogen, alkyl, alkenyl, in each case optionally substituted tetrahydronaphthyl, decahydronaphthyl, cycloalkyl or cycloalkenyl, furthermore alkyl which is substituted by cycloalkyl,

cycloalkenyl, cycloalkyloxy or cycloalkylthio, where the cyclic radicals may optionally be substituted, furthermore optionally substituted aryl, in addition alkyl which is substituted by aryl, aryloxy, arylthio, arylsulphinyl or arylsulphonyl, or alkenyl which is substituted by aryl, where the aryl radicals may in each case optionally be substituted;

$R^2$ represents hydrogen or methyl,

$R^3$ represents alkyl, alkenyl, alkinyl, alkoxyalkyl, hydroxyalkyl, hydroxyalkoxyalkyl, dialkoxyalkyl, or in each case optionally substituted cycloalkyl, cycloalkylalkyl, aryl, aralkyl, aralkenyl, furanylalkyl, tetrahydrofuranylalkyl, tetrahydropyranylalkyl, dioxolanylalkyl or dioxanylalkyl, and

X represents oxygen, sulphur or a $CH_2$ group, and the acid addition salts, isomers and isomer mixtures thereof.

**2.** Pesticides according to Claim 1, where, in the formula (I),

$R^1$ represents hydrogen; in each case straight-chain or branched alkyl having 1 to 18 carbon atoms or alkenyl having 3 to 12 carbon atoms; in addition in each case represents optionally monosubstituted to polysubstituted tetrahydronaphthyl, decahydronaphthyl, cycloalkyl, cycloalkenyl, cycloalkylalkyl, cycloalkenylalkyl, cycloalkyloxyalkyl or cycloalkylthioalkyl in each case having, if appropriate, 5 to 7 carbon atoms in the cycloalkyl or cycloalkenyl parts and, if appropriate, 1 to 6 carbon atoms in the straight-chain or branched alkyl parts, the substituents being identical or different and suitable substituents being in each case: in each case straight-chain or branched alkyl, alkoxy, halogenoalkyl or halogenoalkoxy in each case having 1 to 6 carbon atoms and, if appropriate, 1 to 9 identical or different halogen atoms; and finally represents optionally monosubstituted to polysubstituted aryl, aralkyl, aryloxyalkyl, arylthioalkyl, arylsulphinylalkyl, arylsulphonylalkyl or arylalkenyl in each case having 6 to 10 carbon atoms in the aryl part and, if appropriate up to 6 carbon atoms in the straight-chain or branched alkyl or alkenyl parts, the substituents being identical or different and suitable aryl substituents being in each case: halogen, cyano, nitro, in each case straight-chain or branched alkyl, alkoxy, alkylthio, halogenoalkyl, halogenoalkoxy or halogenoalkylthio in each case having 1 to 6 carbon atoms and, if appropriate 1 to 9 identical or different halogen atoms, cyclohexyl or phenyl;

$R^2$ represents hydrogen or methyl,

$R^3$ represents in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl in each case having 1 to 6 carbon atoms, or hydroxyalkoxyalkyl having 2 to 6 carbon atoms in the individual alkyl parts; or cycloalkyl or cycloalkylalkyl each of which has 3 to 7 carbon atoms in the cycloalkyl part and, if appropriate, 1 to 4 carbon atoms in the alkyl part and each of which is optionally monosubstituted to polysubstituted in the cycloalkyl part by identical or different substituents, suitable substituents being in each case: halogen and in each case straight-chain or branched alkyl, alkoxy, halogenoalkyl or halogenoalkoxy in each case having 1 to 4 carbon atoms and, if appropriate, 1 to 9 identical or different halogen atoms; and in addition represents aryl, arylalkyl or arylalkenyl each of which has 6 to 10 carbon atoms in the aryl part and, if appropriate, up to 6 carbon atoms in the in each case straight-chain or branched alkyl or alkenyl part and each of which is optionally monosubstituted to polysubstituted in the aryl part by identical or different substituents, suitable aryl substituents being in each case: halogen, cyano, nitro and in each case straight-chain or branched alkyl, alkoxy, alkylthio, halogenoalkyl, halogenoalkoxy, halogenoalkylthio, alkoxycarbonyl or alkoximinoalkyl in each case having 1 to 4 carbon atoms in the individual alkyl parts and, if appropriate 1 to 9 identical or different halogen atoms, or represents in each case straight-chain or branched furanylalkyl, tetrahydrofuranylalkyl, tetrahydropyranylalkyl, dioxolanylalkyl or dioxanylalkyl in each case having 1 to 4 carbon atoms in the alkyl part, suitable substituents being in each case: hydrogen and in each case straight-chain or branched alkyl, alkoxy, halogenoalkyl or halogenoalkoxy in each case having 1 to 4 carbon atoms and, if appropriate, 1 to 9 identical or different halogen atoms, and X represents oxygen, sulphur or a $CH_2$ group.

**3.** Pesticides according to Claim 1, where, in the formula (I),

$R^1$ represents hydrogen; in each case straight-chain or branched alkyl having 1 to 12 carbon atoms or alkenyl having 3 to 8 carbon atoms, or tetrahydronaphthyl, decahydronaphthyl, cyclohexyl or cyclohexenyl each of which is optionally monosubstituted, disubstituted or trisubstituted by identical or different, straight-chain or branched alkyl having 1 to 4 carbon atoms; in addition represents an

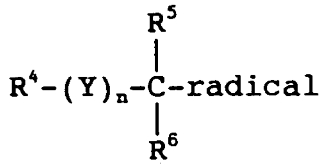

or an

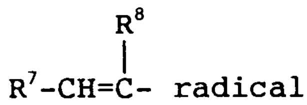

where

$R^4$ represents optionally monosubstituted, disubstituted or trisubstituted cyclohexyl, cyclohexenyl or phenyl, the substituents being identical or different and suitable cyclohexyl or cyclohexenyl substituents being: in each case straight-chain or branched alkyl, alkoxy or halogenoalkyl in each case having 1 to 4 carbon atoms and, if appropriate, 1 to 9 identical or different halogen atoms, and where suitable phenyl substituents are: halogen, cyano, nitro, in each case straight-chain or branched alkyl, alkoxy, alkylthio, halogenoalkyl, halogenoalkoxy or halogenoalkylthio in each case having 1 to 4 carbon atoms and, if appropriate, 1 to 9 identical or different halogen atoms, cyclohexyl or phenyl,

Y represents oxygen, sulphur, a $-CH_2-$ group, an $-C-CH_2-$ group, an $-S-CH_2-$ group, a sulphinyl group, a sulphonyl group, an

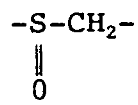

group or an $-SO_2-CH_2-$ group,

n represents a number 0 or 1,

$R^5$ represents hydrogen, methyl or ethyl,

$R^6$ represents hydrogen or methyl,

$R^7$ represents optionally monosubstituted, disubstituted or trisubstituted phenyl, the substituents being identical or different and suitable phenyl substituents being those mentioned in the case of $R^4$, and

$R^8$ represents methyl or ethyl,

$R^1$ in addition, represents in each case optionally monosubstituted, disubstituted or trisubstituted phenyl or naphthyl, the substituents being identical or different and suitable substituents being in each case: halogen, cyano, nitro, in each case straight-chain or branched alkyl, alkoxy, alkylthio, halogenoalkyl, halogenoalkoxy or halogenoalkylthio in each case having 1 to 4 carbon atoms and, if appropriate, 1 to 9 identical or different halogen atoms, cyclohexyl or phenyl;

$R^2$ represents hydrogen or methyl

$R^3$ represents methyl, ethyl, n- or i-propyl, n-, i, s-, or t-butyl, n- or i-pentyl, n- or i-hexyl, n- or i-heptyl, n- or i-octyl, allyl, n- or i-butenyl, n- or i-pentenyl, propargyl, n- or i-butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, represents cyclopropyl, cyclopropylmethyl, cyclopropylethyl, cyclopropylpropyl, cyclopentyl, cyclopentylmethyl, cyclohexyl or cyclohexylmethyl, each of which is optionally monosubstituted to pentasubstituted by fluorine, chlorine, bromine, methyl, ethyl, n- or i-propyl, n-, i-, s- or t-butyl, these substituents being identical or different, or represents in each case optionally monosubstituted, disubstituted or trisubstituted phenyl, benzyl or phenylethyl, the substituents being identical or different and suitable substituents being in each case: fluorine, chlorine, bromine, cyano, nitro, methyl, ethyl, n- or i-propyl, n-, i-, s- or t-butyl, methoxy, ethoxy, methylthio, trifluoromethoxy, trifluoromethylthio, trifluoromethyl, methoxycarbonyl, ethoxycarbonyl or methoximinomethyl; or represents furanylmethyl, tetrahydrofuranylmethyl, tetrahydropyranylmethyl, dioxolanylmethyl, dioxolanylethyl, dioxanylmethyl or

dioxanylethyl,
and
X represents oxygen, sulphur or a $CH_2$ group.

**4.** Pesticides according to Claim 1, where, in the formula (I),
$R^1$ represents tetrahydronaphthyl, decahydronapthyl, cyclohexyl or cyclohexenyl, each of which is optionally monosubstituted, disubstituted or trisubstituted by methyl or t-butyl, the substituents being identical or different, or represents an

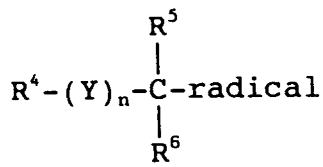

or an

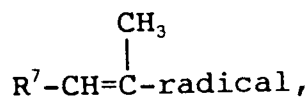

wherein
$R^4$ represents cyclohexyl or cyclohexenyl, each of which is optionally monosubstituted, disubstituted or trisubstituted by methyl, i-propyl, t-butyl or trifluoromethyl, the substituents being identical or different, or represents phenyl which is optionally monosubstituted, disubstituted or trisubstituted by fluorine, chlorine, bromine, methyl, i-propyl, t-butyl, methoxy, propoxy, butoxy, trifluoromethyl, trifluoromethoxy, trifluoromethylthio, methoxy, phenyl or cyclohexyl, the substituents being identical or different,
Y represents oxygen, sulphur, a $-CH_2-$ group, an $-O-CH_2-$ group or an $-S-CH_2-$ group,
n represents a number 0 or 1,
$R^5$ represents methyl or ethyl,
$R^6$ represents hydrogen or methyl, and
$R^7$ represents phenyl which is optionally monosubstituted, disubstituted or trisubstituted by fluorine, chlorine, methyl, isopropyl, t-butyl, trifluoromethyl, trifluoromethoxy, trifluoromethylthio, methoxy, phenyl or cyclohexyl, the substituents being identical or different, $R^1$, in addition, represents phenyl or naphthyl, each of which is optionally monosubstituted, disubstituted or trisubstituted by fluorine, chlorine, bromine, methyl, i-propyl, t-butyl, trifluoromethyl, trifluoromethoxy, trifluoromethylthio, methoxy, phenyl or cyclohexyl, the substituents being identical or different,
$R^2$ represents hydrogen or methyl,
$R^3$ represents methyl, ethyl, n- or i-propyl, n-, i- s- or t-butyl, n- or i-pentyl, n- or i-hexyl, allyl, n- or i-butenyl, n- or i-pentenyl, propargyl, n- or i-butinyl, hydroxyethyl, hydroxypropyl, methoxyethyl, methoxypropyl, ethoxyethyl, ethoxypropyl, hydroxyethoxyethyl, dimethoxyethyl, diethoxyethyl, dichlorocyclopropylmethyl, dimethylcyclopropylmethyl, dichlorodimethylcyclopropylmethyl, cyclopentyl, or cyclohexyl or cyclohexylmethyl, each of which is optionally monosubstituted, disubstituted or trisubstituted by methyl, or furanylmethyl, tetrahydrofuranylmethyl, tetrahydropyranylmethyl, dioxolanylmethyl, dioxolanylethyl or dioxanylmethyl, and
X represents oxygen, sulphur or a $CH_2$ group.

**5.** Use of substituted aminomethylheterocyclic compounds of the formula (I) according to Claim 1 for combating pests.

**6.** Process for the preparation of pesticides for combating pests, characterized in that substituted aminomethylheterocyclic compounds of the formula (I) according to Claim 1 are mixed with extenders and/or surface-active substances.

**7.** Substituted aminomethylheterocyclic compounds of the formula (Ia)

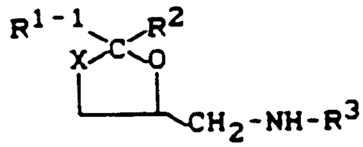

(Ia)

in which

$R^{1-1}$ represents substituted phenyl, in each case optionally substituted cycloalkyl, cycloalkenyl, tetrahydronaphthyl, decahydronaphthyl or naphthyl,

an

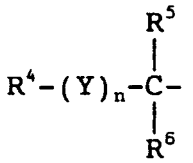 radical

or an

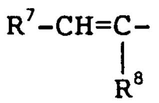 radical,

where

$R^4$ represents in each case optionally substituted cyclohexyl, cyclohexenyl or phenyl,

Y represents oxygen, sulphur, a $-CH_2-$ group an $-O-CH_2-$ group, an $-S-CH_2-$ group, a sulphinyl group, a sulphonyl group, an

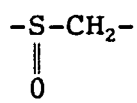

group or an $-SO_2-CH_2-$ group,

n represents a number 0 or 1,

$R^5$ represents hydrogen, methyl or ethyl,

$R^6$ represents hydrogen, methyl or ethyl,

$R^7$ represents optionally monosubstituted, disubstituted or trisubstituted phenyl, the substituents being identical or different, and $R^8$ represents methyl or ethyl,

$R^2$ represents hydrogen or methyl,

$R^3$ represents alkyl, alkenyl, alkinyl, alkoxyalkyl, hydroxyalkyl, hydroxyalkoxyalkyl, dialkoxyalkyl, in each case optionally substituted cycloalkyl, cycloalkylalkyl, aryl, aralkyl, aralkenyl, furanylalkyl, tetrahydrofuranylalkyl, tetrahydropyranylalkyl, dioxolanylalkyl or dioxanylalkyl,

and

X represents oxygen, sulphur or a $-CH_2-$ group, and the acid addition salts, isomers or isomer mixtures thereof.

**8.** Substituted aminomethylheterocyclic compounds according to Claim 7, where, in the formula (Ia),

$R^{1-1}$ represents monosubstituted to polysubstituted phenyl, the substituents being identical or different, or optionally monosubstituted to polysubstituted naphthyl, the substituents being identical or different and suitable substituents being in each case: halogen, cyano, nitro, in each case straight-chain or branched alkyl, alkoxy, alkylthio, halogenoalkyl, halogenoalkoxy, halogenoalkylthio in each case

having 1 to 6 carbon atoms and, if appropriate, 1 to 9 identical or different halogen atoms, cyclohexyl or phenyl; in addition represents in each case optionally monosubstituted to polysubstituted cycloalkyl or cycloalkenyl in each case having 5 to 7 carbon atoms, tetrahydronaphthyl or decahydronaphthyl, the substituents being identical or different and suitable substituents being in each case: in each case straight-chain or branched alkyl, halogenoalkyl or halogenoalkoxy in each case having 1 to 6 carbon atoms and, if appropriate, 1 to 9 identical or different halogen atoms; in addition represents an

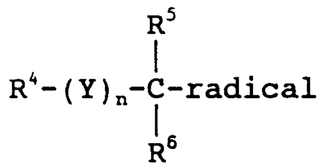

or an

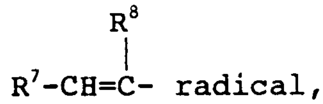

where

$R^4$ represents in each case optionally monosubstituted, disubstituted or trisubstituted cyclohexyl, cyclohexenyl or phenyl, the substituents being identical or different and suitable cyclohexyl or cyclohexenyl substituents being: in each case straight-chain or branched alkyl, alkoxy or halogenoalkyl in each case having 1 to 4 carbon atoms and, if appropriate, 1 to 9 halogen atoms, and suitable phenyl substituents being: halogen, cyano, nitro, in each case straight-chain or branched alkyl, alkoxy, alkylthio, halogenoalkyl, halogenoalkoxy or halogenoalkylthio in each case having 1 to 4 carbon atoms and, if appropriate, 1 to 9 identical or different halogen atoms, cyclohexyl or phenyl,

Y represents oxygen, sulphur, a $-CH_2-$ group, an $-O-CH_2-$ group, an $-S-CH_2-$ group, a sulphinyl group, a sulphonyl group, an

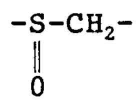

group or an $-SO_2-CH_2-$ group,

n represents a number 0 or 1,

$R^5$ represents hydrogen, methyl or ethyl,

$R^6$ represents hydrogen or methyl,

$R^7$ represents optionally monosubstituted, disubstituted or trisubstituted phenyl, the substituents being identical or different and suitable phenyl substituents being those mentioned in the case of $R^4$, and

$R^8$ represents methyl or ethyl,

$R^2$ represents hydrogen or methyl,

$R^3$ represents in each case straight-chain or branched alkyl having 1 to 12 carbon atoms, alkenyl having 3 to 8 carbon atoms, alkinyl having 3 to 8 carbon atoms, hydroxyalkyl having 2 to 6 carbon atoms, alkoxyalkyl or dialkoxyalkyl in each case having 1 to 6 carbon atoms or hydroxyalkoxyalkyl having 2 to 6 carbon atoms in the individual alkyl parts, or cycloalkyl or cycloalkylalkyl in each case having 3 to 7 carbon atoms in the cycloalkyl part and, if appropriate, 1 to 4 carbon atoms in the alkyl part and each of which is optionally monosubstituted to polysubstituted in the cycloalkyl part, the substituents being identical or different and suitable substituents being in each case: halogen and in each halogenoalkyl or halogenoalkoxy in each case having 1 to 4 carbon atoms and, if appropriate, 1 to 9 identical or different halogen atoms; in addition represents aryl, arylalkyl or arylalkenyl in each case having 6 to 10 carbon atoms in the aryl part and, if appropriate, up to 6 carbon atoms in each straight-chain or branched alkyl or alkenyl part and each of which is optionally monosubstituted or polysubstituted in the aryl part by identical or different substituents, suitable aryl susbtituents being in each case: halogen, cyano, nitro and in each case straight-chain or branched alkyl, alkoxy, alkylthio, halogenoalkyl, halogenoalkoxy, halogenoalkylthio, alkoxycarbonyl or alkoximinoalkyl in each case having 1 to 4 carbon atoms in the individual alkyl parts and, if appropriate, 1 to 9 identical or different halogen atoms, or represents in each case straight-chain or branched furanylalkyl, tetrahydrofuranylalkyl, tetrahydropyranylalkyl, dioxolanylalkyl or dioxanylalkyl in each case having 1 to 4 carbon atoms in the alkyl part, and X represents oxygen, sulphur or a $CH_2$ group.

**9.** Substituted aminomethylheterocyclic compounds according to Claim 7, where, in the formula (Ia),

$R^{1-1}$ represents monosubstituted, disubstituted or trisubstituted phenyl, the substituents being identical or different, or optionally monosubstituted, disubstituted or trisubstituted naphthyl, the substituents being identical or different and suitable substituents being in each case: fluorine, chlorine, bromine, methyl, i-propyl, t-butyl, trifluoromethyl, trifluoromethoxy, trifluoromethylthio, methoxy, phenyl or cyclohexyl; in addition represents in each case optionally monosubstituted, disubstituted or trisubstituted cyclohexyl, cyclohexenyl, tetrahydronaphthyl or decahydronaphthyl, the substituents being identical or different and suitable substituents being in each case: straight-chain or branched alkyl having 1 to 4 carbon atoms; in addition represents

an 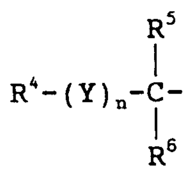 radical

or an

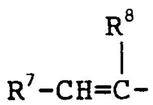 radical,

where

$R^4$ represents cyclohexyl or cyclohexenyl, each of which is optionally monosubstituted, disubstituted or trisubstituted by methyl, i-propyl, t-butyl, methoxy, propoxy, butoxy, or trifluoromethyl, the substituents being identical or different, or represents phenyl which is optionally monosubstituted, disubstituted or trisubstituted by fluorine, chlorine, bromine, methyl, i-propyl, t-butyl trifluoromethyl, trifluoromethoxy, trifluoromethylthio, methoxy, phenyl or cyclohexyl, the substituents being identical or different,

Y represents oxygen, sulphur, a $-CH_2-$ group, an $-O-CH_2-$ group or an $-S-CH_2-$ group,

n represents a number 0 or 1,

$R^6$ represents hydrogen or methyl, and

$R^7$ represents phenyl which is optionally monosubstituted, disubstituted or trisubstituted by fluorine, chlorine, methyl, isopropyl, t-butyl, trifluoromethyl, trifluoromethoxy, trifluoromethylthio, methoxy, phenyl or cyclohexyl, the substituents being identical or different, and

$R^8$ represents methyl or ethyl,

$R^2$ represents hydrogen or methyl,

$R^3$ represents methyl, ethyl, n- or i-propyl, n-, i-, s- or t-butyl, n- or i-pentyl, n- or i-hexyl, n- or i-heptyl, n- or i-octyl, allyl, n- or i-butenyl, n- or i-pentenyl, propargyl, n- or i-butinyl, hydroxyethyl, hydroxypropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl, represents cyclopropyl, cyclopropylmethyl, cyclopropylethyl, cyclopropylpropyl, cyclopentyl, cyclopentylmethyl, cyclohexyl or cyclohexylmethyl, each of which is optionally monosubstituted to pentasubstituted by fluorine, chlorine, bromine, methyl, ethyl, n- or i-propyl, n-, i-, s- and/or t-butyl, the substituents being identical or different, or represents in each case optionally monosubstituted, disubstituted or trisubstituted phenyl, benzyl or phenylethyl, the substituents being identical or different

and suitable substituents being in each case: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy, trifluoromethylthio, methoxycarbonyl, ethoxycarbonyl or methoxyiminomethyl; or represents furanylmethyl, tetrahydrofuranylmethyl, tetrahydropyranylmethyl, dioxolanylmethyl, dioxolanylethyl, dioxanylmethyl or dioxanylethyl, and

X represents oxygen, sulphur or a $CH_2$ group.

**10.** Process for the preparation of substituted aminomethylheterocyclic compounds of the formula (Ia)

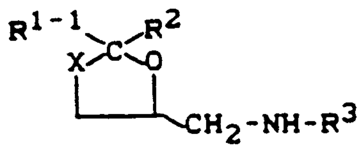

(Ia)

in which

$R^{1-1}$ represents substituted phenyl, in each case optionally substituted cycloalkyl, cycloalkenyl, tetrahydronaphthyl, decahydronaphthyl or naphthyl,

an

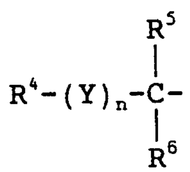
radical

or an

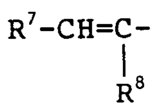
radical,

where

$R^4$ represents in each case optionally substituted cyclohexyl, cyclohexenyl or phenyl,

Y represents oxygen, sulphur, a $-CH_2-$ group an $-O-CH_2-$ group, an $-S-CH_2-$ group, a sulphinyl group, a sulphonyl group, an

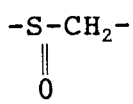

group or an $-SO_2-CH_2-$ group,

n represents a number 0 or 1,

$R^5$ represents hydrogen, methyl or ethyl,

$R^6$ represents hydrogen, methyl or ethyl,

$R^7$ represents optionally monosubstituted, disubstituted or trisubstituted phenyl, the substituents being identical or different, and

$R^8$ represents methyl or ethyl;

$R^2$ represents hydrogen or methyl,

$R^3$ represents alkyl, alkenyl, alkinyl, alkoxyalkyl, hydroxyalkyl, hydroxyalkoxyalkyl, dialkoxyalkyl, in

each case optionally substituted cycloalkyl, cycloalkylalkyl, aryl, aralkyl, aralkenyl, furanylalkyl, tetrahydrofuranylalkyl, tetrahydropyranylalkyl, dioxolanylalkyl or dioxanylalkyl,
and

X represents oxygen, sulphur or a $-CH_2-$ group, and the acid addition salts, isomers or isomer mixtures thereof, characterized in that

(a) heterocyclic compounds of the formula (II)

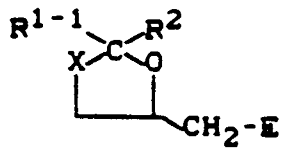

(II)

in which

$R^{1-1}$ and $R^2$ have the abovementioned meaning, and

E represents an electron-withdrawing leaving group,

are reacted with amines of the formula (III)

$H_2N-R^3$ (III)

in which

$R^3$ has the abovementioned meaning,

if appropriate in the presence of a diluent and if appropriate in the presence of an acid-binding agent, or

when

(b) the substituted aminomethylheterocyclic compounds of the formula (Ia-1)

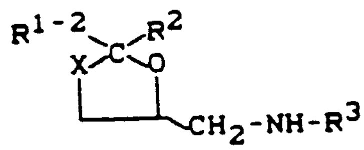

(Ia-1)

in which

$R^{1-2}$ represents substituted phenyl, optionally substituted naphthyl, an

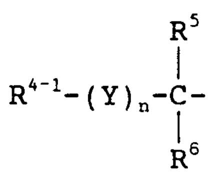 radical

or an

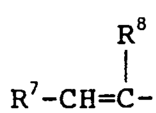 radical,

X, $R^2$, $R^3$, $R^5$, $R^6$, $R^7$ and $R^8$ have the abovementioned meaning, and

$R^{4-1}$ represents optionally substituted phenyl, obtainable with the aid of process (a) are hydrogenated using hydrogen in the presence of a catalyst and if appropriate in the presence of a

diluent.

**Revendications**

1. Produits parasiticides, caractérisés en ce qu'ils contiennent au moins un composé aminométhylhétérocyclique de formule générale I

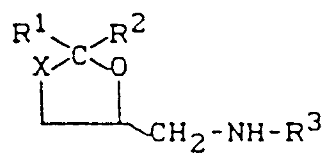

(I)

dans laquelle
$R^1$ représente l'hydrogène, un groupe alkyle, alcényle, un groupe tétrahydronaphtyle, déca-hydronaphtyle, cycloalkyle ou cycloalcényle, chacun de ceux-ci éventuellement substitué, ou encore un groupe alkyle substitué par un groupe cycloalkyle, cycloalcényle, cycloalcoxy ou cycloalkylthio, les radicaux cycliques pouvant éventuellement être substitués, ou encore un groupe aryle éventuellement substitué, ou encore un groupe alkyle substitué par un groupe aryle, aryloxy, arylthio, arylsulfinyle ou arylsulfonyle, ou un groupe alcényle substitué par un groupe aryle, tous les groupes aryle pouvant éventuellement être substitués;
$R^2$ représente l'hydrogène ou le groupe méthyle,
$R^3$ représente un groupe alkyle, alcényle, alcynyle, alcoxyalkyle, hydroxyalkyle, hydroxyalcoxyalkyle, dialcoxyalkyle, un groupe cycloalkyle, cycloalkylalkyle, aryle, aralkyle, aralcényle, furannylalkyle, tétrahydrofurannylalkyle, tétrahydropyrannylalkyle, dioxolannylalkyle ou dioxannylalkyle, chacun de ces groupes pouvant éventuellement être substitué et
X représente l'oxygène, le soufre ou un groupe $CH_2$,
ou leurs sels formés par addition avec des acides, leurs isomères et mélanges d'isomères.

2. Produits parasiticides selon revendication 1, pour lesquels, dans la formule I
$R^1$ représente l'hydrogène, un groupe alkyle en $C_1$-$C_{18}$ ou alcényle en $C_3$-$C_{12}$, chacun d'eux a chaîne droite ou ramifiée ; ou encore un groupe tétrahydronaphtyle, décahydronaphtyle, cycloalkyle, cycloalcényle, cycloalkylalkyle, cycloalcénylalkyle, cycloalkyloxyalkyle ou cycloalkylthioalkyle portant chacun le cas échéant un à plusieurs substituants identiques ou différents et contenant chacun le cas échéant 5 à 7 atomes de carbone dans les parties cycloalkyle et cycloalcényle et le cas échéant 1 à 6 atomes de carbone dans les parties alkyle à chaîne droite ou ramifiée, les substituants en question étant les suivants : des groupes alkyle, alcoxy, halogénoalkyle ou halogénoalcoxy à chaîne droite ou ramifiée, contenant chacun 1 à 6 atomes de carbone et le cas échéant 1 à 9 atomes d'halogènes identiques ou différents ; et finalement, un groupe aryle, aralkyle, aryloxyalkyle, arylthioalkyle, arylsulfinylalkyle, arylsulfonylalkyle ou arylalcényle portant chacun le cas échéant un ou plusieurs substituants identiques ou différents et contenant chacun 6 à 10 atomes de carbone dans la partie aryle et le cas échéant jusqu'à 6 atomes de carbone dans les parties alkyle et alcényle à chaîne droite ou ramifiée, les substituants du groupe aryle étant : des halogènes, des groupes cyano, nitro, des groupes alkyle, alcoxy, alkylthio, halogénoalkyle, halogénoalcoxy ou halogénoalkylthio, chacun d'eux à chaîne droite ou ramifiée et contenant chacun 1 à 6 atomes de carbone et le cas échéant 1 à 9 atomes d'halogènes identiques ou différents, des groupes cyclohexyle ou phényle;
$R^2$ représente l'hydrogène ou le groupe méthyle,
$R^3$ représente un groupe alkyle en $C_1$-$C_{12}$, alcényle en $C_3$-$C_8$, alcynyle en $C_3$-$C_8$, hydroxyalkyle en $C_2$-$C_6$, alcoxyalkyle ou dialcoxyalkyle contenant chacun 1 à 6 atomes de carbone ou hydroxyalcoxyalkyle contenant 2 à 6 atomes de carbone dans chacune des parties alkyle, tous ces groupes pouvant être à chaîne droite ou ramifiée ; un groupe cycloalkyle ou cycloalkylalkyle portant chacun le cas échéant un ou plusieurs substituants identiques ou différents dans la partie cycloalkyle et contenant chacun 3 à 7 atomes de carbone dans la partie cycloalkyle et le cas échéant 1 à 4 atomes de carbone dans la partie alkyle, les substituants en question étant les suivants : les halogènes et les groupes alkyle, alcoxy, halogénoalkyle ou halogénoalcoxy, chacun d'eux à chaîne droite ou ramifiée et contenant chacun 1 à 4 atomes de carbone et le cas échéant 1 à 9 atomes d'halogènes identiques ou différents ; et en outre

un groupe aryle, arylalkyle ou arylalcényle portant chacun le cas échéant un ou plusieurs substituants identiques ou différents dans la partie aryle et contenant chacun 6 à 10 atomes de carbone dans la partie aryle et le cas échéant jusqu'à 6 atomes de carbone dans les parties alkyle et alcényle à chaîne droite ou ramifiée, les substituants des parties aryle étant les suivants : les halogènes, les groupes cyano, nitro, les groupes alkyle, alcoxy, alkylthio, halogénoalkyle, halogénoalcoxy, halogènoalkylthio, alcoxycarbonyle ou alcoxyiminoalkyle, chacun d'eux à chaîne droite ou ramifiée et contenant chacun 1 à 4 atomes de carbone dans les diverses parties alkyle et le cas échéant 1 à 9 atomes d'halogènes identiques ou différents ; ou bien un groupe furannylalkyle, tétrahydrofurannylalkyle, tétrahydropyrannylalkylthio, dioxolannylalkyle ou dioxannylalkyle, chacun d'eux à chaîne droite ou ramifiée et contenant chacun 1 à 4 atomes de carbone dans la partie alkyle, les substituants en question étant les suivants : les halogènes et les groupes alkyle, alcoxy, halogénoalkyle ou halogénoalcoxy, chacun d'eux à chaîne droite ou ramifiée et contenant chacun 1 à 4 atomes de carbone et le cas échéant 1 à 9 atomes d'halogènes identiques ou différents, et
X représente l'oxygène, les soufre ou un groupe $CH_2$.

**3.** Produits parasiticides selon la revendication 1, pour lesquels, dans la formule I
$R^1$ représente l'hydrogène, un groupe alkyle en $C_1$-$C_{12}$ ou alcényle en $C_3$-$C_8$, chacun d'eux à chaîne droite ou ramifiée, ou un groupe tétrahydronaphtyle, décahydronaphtyle, cyclohexyle ou cyclohexényle, portant chacun le cas échéant 1 à 3 substituants alkyle en $C_1$-$C_4$ à chaîne droite ou ramifiée ; ou encore un groupe

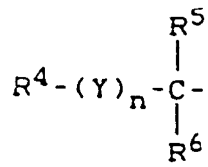

ou

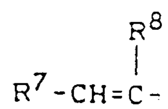

dans lesquels
$R^4$ représente un groupe cyclohexyle, cyclohexényle ou phényle, portant chacun le cas échéant 1 à 3 substituants identiques ou différents, les substituants des groupes cyclohexyle et cyclohexényle étant les suivants: des groupes alkyle, alcoxy ou halogénoalkyle, chacun d'eux à chaîne droite ou ramifiée et contenant chacun 1 à 4 atomes de carbone et le cas échéant 1 à 9 atomes d'halogènes identiques ou différents, et les substituants du groupe phényle étant les suivants : les halogènes, les groupes cyano, nitro, les groupes alkyle, alcoxy, alkylthio, halogénoalkyle, halogénoalcoxy ou halogénoalkylthio, chacun d'eux à chaîne droite ou ramifiée et contenant chacun 1 à 4 atomes de carbone et le cas échant 1 à 9 atomes d'halogènes identiques ou différents, les groupes cyclohexyle ou phényle,
Y représente l'oxygène, le soufre, un groupe $-CH_2-$, un groupe $-O-CH_2-$, un groupe $-S-CH_2-$, un groupe sulfinyle, un groupe sulfonyle, un groupe

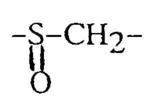

ou un groupe $-SO_2-CH_2-$,
n est égal à 0 ou 1,
$R^5$ représente l'hydrogène, un groupe méthyle ou éthyle,

$R^6$ représente l'hydrogène ou un groupe méthyle,
$R^7$ représente un groupe phényle portant le cas échéant 1 à 3 substituants identiques ou différents, ces substituants étant les mêmes que ceux qui ont été mentionnés en référence à $R^4$ et
$R^8$ représente un groupe méthyle ou éthyle,
$R^1$ peut en outre représenter un groupe phényle ou naphtyle portant le cas échéant chacun 1 à 3 substituants identiques ou différents, les substituants en question étant : des halogènes, des groupes cyano, nitro, des groupes alkyle, alcoxy, alkylthio, halogénoalkyle, halogénoalcoxy ou halogénoalkylthio, chacun d'eux à chaîne droite ou ramifiée, et contenant chacun 1 à 4 atomes de carbone et le cas échéant 1 à 9 atomes d'halogènes identiques ou différents ; des groupes cyclohexyle ou phényle ;
$R^2$ représente l'hydrogène ou un groupe méthyle,
$R^3$ représente un groupe méthyle, éthyle, n- ou iso-propyle, n-, iso-, sec- ou tert-butyle, n- ou iso-pentyle, n- ou iso-hexyle, n- ou iso-heptyle, n- ou iso-octyle, allyle, n- ou iso-butényle, n- ou iso-pentényle, propargyle n- ou iso-butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, un groupe cyclopropyle, cyclopropylméthyle, cyclopropyléthyle, cyclopropylpropyle, cyclopentyle, cyclopentylméthyle, cyclohexyle ou cyclohexylméthyle portant chacun, le cas échéant, 1 à 5 substituants identiques ou différents choisis parmi le fluor, le chlore, le brome, les groupes méthyle, éthyle n-ou iso-propyle, n-, iso-, sec- ou tert-butyle, ou un groupe phényle, benzyle ou phényléthyle portant chacun le cas échéant, 1 à 3 substituants identiques ou différents, les substituants en question étant : le fluor, le chlore, le brome, les groupes cyano, nitro, méthyle, éthyle, n- ou iso-propyle, n-, iso-, sec- ou tert-butyle, méthoxy, éthoxy, méthylthio, trifluorométhoxy, trifluorométhylthio, trifluorométhyle, méthoxycarbonyle, éthoxycarbonyle ou méthoxyiminométhyle ; ou un groupe furannylméthyle, tétrahydrofurannylméthyle, tétrahydropyrannylméthyle, dioxolannylméthyle, dioxolannyléthyle, dioxannylméthyle ou dioxannyléthyle et X représente l'oxygène, le soufre ou un groupe $CH_2$.

**4.** Produits parasiticides selon la revendication 1, pour lesquels, dans la formule I
$R^1$ représente un groupe tétrahydronaphtyle, décahydronaphtyle, cyclohexyle ou cyclohexényle portant chacun, le cas échéant, 1 à 3 substituants identiques ou différents choisis parmi les groupes méthyle et tert-butyle, ou un groupe

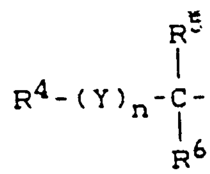

ou

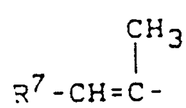

dans lesquels
$R^4$ représente un groupe cyclohexyle ou cyclohexényle portant chacun, le cas échéant, 1 à 3 substituants identiques ou différents choisis parmi les groupes méthyle, isopropyle, tert-butyle ou trifluorométhyle, ou un groupe phényle portant éventuellement 1 à 3 substituants identiques ou différents choisis parmi le fluor, le chlore, le brome, les groupes méthyle, isopropyle, tert-butyle, méthoxy, propoxy, butoxy, trifluorométhyle, trifluorométhoxy, trifluorométhylthio, méthoxy, phényle ou cyclohexyle,
Y représente l'oxygène, le soufre, un groupe $-CH_2-$, un groupe $-O-CH_2-$ ou un groupe $-S-CH_2-$,
n est égal à 0 ou 1,
$R^5$ représente un groupe méthyle ou éthyle,

$R^6$ représente l'hydrogène ou un groupe méthyle et
$R^7$ représente un groupe phényle portant le cas échéant 1 à 3 substituants identiques ou différents, choisis parmi le fluor, le chlore, les groupes méthyle, isopropyle, tert-butyle, trifluorométhyle, trifluorométhoxy, trifluorométhylthio, méthoxy, phényle ou cyclohexyle,
$R^1$ peut en outre représenter un groupe phényle ou naphtyle portant chacun le cas échéant 1 à 3 substituants identiques ou différents choisis parmi le fluor, le chlore, le brome, les groupes méthyle, isopropyle, tert-butyle, trifluorométhyle, trifluorométhoxy, trifluorométhylthio, méthoxy, phényle ou cyclohexyle,
$R^2$ représente l'hydrogène ou un groupe méthyle,
$R^3$ représente un groupe méthyle, éthyle, n- ou iso-propyle, n-, iso-, sec- ou tert-butyle, n- ou isopentyle, n- ou iso-hexyle, allyle, n- ou iso-butényle, n- ou iso-pentényle, propargyle, n- ou iso-butynyle, hydroxyéthyle, hydroxypropyle, méthoxyéthyle, méthoxypropyle, éthoxyéthyle, éthoxypropyle, hydroxyéthoxyéthyle, diméthoxyéthyle, diéthoxyéthyle, dichlorocyclopropylméthyle, diméthylcyclopropylméthyle, dichlorodiméthylcyclopropylméthyle, cyclopentyle, ou un groupe cyclohexyle ou cyclohexylméthyle portant chacun le cas échéant 1 à 3 substituants méthyle, ou un groupe furannylméthyle, tétrahydrofurannylméthyle, tétrahydropyrannylméthyle, dioxolannylméthyle, dioxolannyléthyle ou dioxannylméthyle et
X représente l'oxygène, le soufre ou un groupe $CH_2$.

5. Utilisation des composés aminométhylhétérocycliques substitués de formule I de la revendication 1 pour la lutte contre les parasites.

6. Procédé de préparation de produits parasiticides pour la lutte contre les parasites, caractérisé en ce que l'on mélange les composés aminométhylhétérocycliques substitués de formule I de la revendication 1 avec des diluants et/ou des agents tensioactifs.

7. Composés aminométhylhétérocycliques substitutés de formule Ia

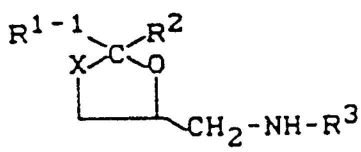

(Ia)

dans laquelle
$R^{1-1}$ représente un groupe phényle substitué, un groupe cycloalkyle, cycloalcényle, tétrahydronaphtyle, décahydronaphtyle ou naphtyle, chacun d'eux éventuellement substitué, un groupe

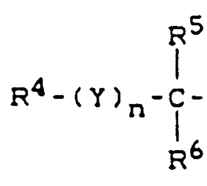

ou

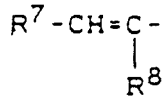

dans lesquels
$R^4$ représente un groupe cyclohexyle, cyclohexényle ou phényle, chacun d'eux éventuellement substitué,

Y représente l'oxygène, le soufre, un groupe $-CH_2-$ ou $-O-CH_2-$, un groupe $-S-CH_2-$, un groupe sulfinyle, un groupe sulfonyle, un groupe

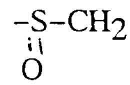

ou un groupe $-SO_2-CH_2-$,
n est égal à 0 ou 1,
$R^5$ représente l'hydrogène, un groupe méthyle ou éthyle,
$R^6$ représente l'hydrogène, un groupe méthyle ou éthyle,
$R^7$ représente un groupe phényle portant le cas échéant 1 à 3 substituants identiques ou différents,
$R^8$ représente un groupe méthyle ou éthyle,
$R^2$ représente l'hydrogène ou un groupe méthyle,
$R^3$ représente un groupe alkyle, alcènyle, alcynyle, alcoxyalkyle, hydroxyalkyle, hydroxyalcoxyalkyle, dialcoxyalkyle, un groupe cycloalkyle, cycloalkylalkyle, aryle, aralkyle, aralcényle, furannylalkyle, tétrahydrofurannylalkyle, tétrahydropyrannylalkyle, dioxolannylalkyle ou dioxannylalkyle, chacun d'eux étant éventuellement substitué et
X représente l'oxygène, le soufre ou un groupe $CH_2$,
leurs sels formés par addition avec des acides, leurs isomères ou mélanges d'isomères.

**8.** Composés aminométhylhétérocycliques substitués selon la revendication 7, pour lesquels, dans la formule Ia
$R^{1-1}$ représente un groupe phényle portant un ou plusieurs substituants identiques ou différents ou un groupe naphtyle portant le cas échéant un ou plusieurs substituants identiques ou différents, les substituants en question étant : des halogènes, des groupes cyano, nitro, des groupes alkyle, alcoxy, alkylthio, halogénoalkyle, halogénoalcoxy, halogénoalkylthio, chacun d'eux à chaîne droite ou ramifiée et contenant chacun 1 à 6 atomes de carbone et le cas échéant 1 à 9 atomes d'halogènes identiques ou différents, des groupes cyclohexyle ou phényle ; ou encore un groupe cycloalkyle ou cycloalcényle portant chacun le cas échéant un ou plusieurs substituants identiques ou différents et contenant chacun 5 à 7 atomes de carbone, un groupe tétrahydronaphtyle ou décahydronaphtyle, les substituants en question étant : des groupes alkyle, halogénoalkyle ou halogénoalcoxy, chacun d'eux à chaîne droite ou ramifiée et contenant chacun 1 à 6 atomes de carbone et le cas échéant 1 à 9 atomes d'halogènes identiques ou différents; ou encore un groupe

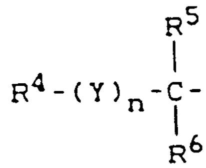

ou

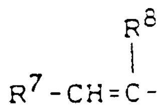

dans lesquels
$R^4$ représente un groupe cyclohexyle, cyclohexényle ou phényle portant chacun le cas échéant 1 à 3 substituants identiques ou différents, les substituants du groupe cyclohexyle et du groupe cyclohexényle étant les suivants : des groupes alkyle, alcoxy ou halogénoalkyle, chacun d'eux à chaîne droite ou ramifiée et contenant chacun 1 à 4 atomes de carbone et le cas échéant 1 à 9 atomes d'halogènes, et les substituants du groupe phényle étant les suivants : les halogènes, les groupes cyano, nitro, les groupes alkyle, alcoxy, alkylthio, halogénoalkyle, halogénoalcoxy ou halogénoalkylthio, chacun d'eux à

chaîne droite ou ramifiée et contenant chacun 1 à 4 atomes de carbone et le cas échéant 1 à 9 atomes d'halogènes identiques ou différents, les groupes cyclohexyle ou phényle,
Y représente l'oxygène, le soufre, un groupe $-CH_2-$, un groupe $-O-CH_2-$, un groupe $-S-CH_2-$, un groupe sulfinyle, un groupe sulfonyle, un groupe

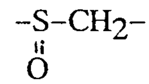

ou un groupe $-SO_2-CH_2-$,
n est égal à 0 ou 1,
$R^5$ représente l'hydrogène, un groupe méthyle ou éthyle,
$R^6$ représente l'hydrogène ou un groupe méthyle,
$R^7$ représente un groupe phényle portant le cas échéant 1 à 3 substituants identiques ou différents, ces substituants étant ceux qui ont été mentionnés en référence à $R^4$,
$R^8$ représente un groupe méthyle ou éthyle,
$R^2$ représente l'hydrogène ou un groupe méthyle,
$R^3$ représente un groupe alkyle en $C_1$-$C_{12}$, alcényle en $C_3$-$C_8$, alcynyle en $C_3$-$C_8$, hydroxyalkyle en $C_2$-$C_6$, alcoxyalkyle ou dialcoxyalkyle contenant chacun 1 à 6 atomes de carbone ou hydroxyalcoxyalkyle contenant 2 à 6 atomes de carbone dans les diverses parties alkyle, chacun de ces groupes à chaîne droite ou ramifiée, ou un groupe cycloalkyle ou cycloalkylalkyle portant éventuellement chacun un ou plusieurs substituants identiques ou différents dans la partie cycloalkyle et contenant chacun 3 à 7 atomes de carbone dans la partie cycloalkyle et le cas échéant 1 à 4 atomes de carbone dans la partie alkyle, les substituants en question étant les suivants : les halogènes et les groupes alkyle, alcoxy, halogénoalkyle ou halogénoalcoxy, chacun d'eux à chaîne droite ou ramifiée et contenant chacun 1 à 4 atomes de carbone et le cas échéant 1 à 9 atomes d'halogènes identiques ou différents ; ou encore un groupe aryle, arylalkyle ou arylalcényle portant chacun le cas échéant un ou plusieurs substituants identiques ou différents dans la partie aryle et contenant chacun 6 à 10 atomes de carbone dans la partie aryle et le cas échéant jusqu'à 6 atomes de carbone dans chacune des parties alkyle et alcényle à chaîne droite ou ramifiée, les substituants de la partie aryle étant les suivants : les halogènes, les groupes cyano, nitro et les groupes alkyle, alcoxy, alkylthio, halogénoalkyle, halogénoalcoxy, halogénoalkylthio, alcoxycarbonyle ou alcoximinoalkyle, chacun d'eux à chaîne droite ou ramifiée et contenant chacun 1 à 4 atomes de carbone dans les diverses parties alkyle et le cas échéant 1 à 9 atomes d'halogènes identiques ou différents, ou bien un groupe furannylalkyle, tétrahydrofurannylalkyle, tétrahydropyrannylalkyle, dioxolannylalkyle ou dioxannylalkyle, chacun d'eux à chaîne droite ou ramifiée et contenant chacun 1 à 4 atomes de carbone dans la partie alkyle et
X représente l'oxygène, le soufre ou un groupe $CH_2$.

**9.** Composés aminométhylhétérocycliques substitués selon la revendication 7, pour lesquels, dans la formule I, $R^{1-1}$ représente un groupe phényle portant 1 à 3 substituants identiques ou différents ou un groupe naphtyle portant le cas échéant 1 à 3 substituants identiques ou différents, les substituants en question étant : le fluor, le chlore, le brome, les groupes méthyle, isopropyle, tert-butyle, trifluorométhyle, trifluorométhoxy, trifluorométhylthio, méthoxy, phényle ou cyclohexyle ; ou encore un groupe cyclohexyle, cyclohexényle, tétrahydronaphtyle ou décahydronaphtyle portant chacun le cas échéant 1 à 3 substituants identiques ou différents, les substituants en question étant : des groupes alkyle à chaîne droite ou ramifiée en $C_1$-$C_4$ ; ou encore un groupe

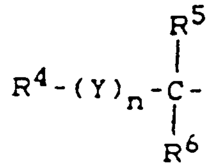

ou

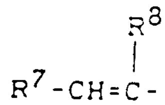

dans lesquels

$R^4$ représente un groupe cyclohexyle ou cyclohexényle portant chacun le cas échéant 1 à 3 substituants identiques ou différents choisis parmi les groupes méthyle, isopropyle, tert-butyle, méthoxy, propoxy, butoxy ou trifluorométhyle, ou un groupe phényle portant éventuellement 1 à 3 substituants identiques ou différents choisis parmi le fluor, le chlore, le brome, les groupes méthyle, isopropyle, tert-butyle, trifluorométhyle, trifluorométhoxy, trifluorométhylthio, méthoxy, phényle ou cyclohexyle,

Y représente l'oxygène, le soufre, un groupe $-CH_2-$, un groupe $-O-CH_2-$ ou un groupe $-S-CH_2-$,

n est égal à 0 ou 1,

$R^6$ représente l'hydrogène ou un groupe méthyle et

$R^7$ représente un groupe phényle portant le cas échéant 1 à 3 substituants identiques ou différents choisis parmi le fluor, le chlore, des groupes méthyle, isopropyle, tert-butyle, trifluorométhyle, trifluorométhoxy, trifluorométhylthio, méthoxy, phényle ou cyclohexyle,

$R^8$ représente un groupe méthyle ou éthyle,

$R^2$ représente l'hydrogène ou un groupe méthyle,

$R^3$ représente un groupe méthyle, éthyle, n- ou iso-propyle, n-, iso-, sec- ou tert-butyle, n- ou iso-pentyle, n- ou iso-hexyle, n- ou iso-heptyle, n- ou iso-octyle, allyle, n- ou iso-butényle, n- ou iso-pentényle, propargyle, n- ou iso-butynyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, un groupe cyclopropyle; cyclopropylméthyle, cyclopropyléthyle, cyclopropylpropyle, cyclopentyle, cyclopentylméthyle, cyclohexyle ou cyclohexylméthyle portant chacun le cas échéant 1 à 5 substituants identiques ou différents choisis parmi le fluor, le chlore, le brome, les groupes méthyle, éthyle, n- ou iso-propyle, n- iso-, sec- ou tert-butyle, ou un groupe phényle, benzyle ou phényléthyle portant chacun le cas échéant 1 à 3 substituants identiques ou différents les substituants en question étant : le fluor, le chlore, le brome, les groupes cyano, nitro, méthyle, éthyle, n-ou iso-propyle, n-, iso-, sec- ou tert-butyle, méthoxy, éthoxy, méthylthio, trifluorométhyle, trifluorométhoxy, triluorométhylthio, méthoxycarbonyle, éthoxycarbonyle, ou méthoxyiminométhyle; ou un groupe furannylméthyle, tétra-hydrofurannylméthyle, tétrahydropyrannylméthyle, dioxolannylméthyle, dioxolannyléthyle, dioxannylméthyle ou dioxannyléthyle et X représente l'oxygène, le soufre ou un groupe $CH_2$.

**10.** Procédé de préparation des composés aminométhylhétérocycliques substitués de formule Ia

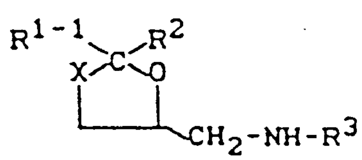

(Ia)

dans laquelle

$R^{1-1}$ représente un groupe phényle substitué, un groupe cycloalkyle, cycloalcényle, tétrahydronaphtyle, décahydronaphtyle ou naphtyle, chacun d'eux éventuellement substitué, ou un groupe

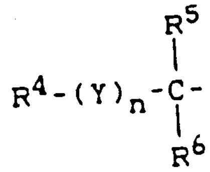

ou

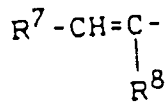

dans lesquels

$R^4$ représente un groupe cyclohexyle, cyclohexényle ou phényle, chacun d'eux éventuellement substitué,

Y représente l'oxygène, le soufre, un groupe $-CH_2-$, $-O-CH_2-$, $-S-CH_2-$, sulfinyle, sulfonyle,

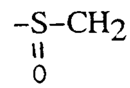

ou $-SO_2-CH_2-$,

n est égal à 0 ou 1,

$R^5$ représente l'hydrogène, un groupe méthyle ou éthyle,

$R^6$ représente l'hydrogène,un groupe méthyle ou éthyle,

$R^7$ représente un groupe phényle portant le cas échéant 1 à 3 substituants identiques ou différents,

$R^8$ représente un groupe méthyle ou éthyle,

$R^2$ représente l'hydrogène ou un groupe méthyle,

$R^3$ représente un groupe alkyle, alcényle, alcynyle, alcoxyalkyle, hydroxyalkyle, hydroxyalcoxyalkyle, dialcoxyalkyle, un groupe cycloalkyle, cycloalkylalkyle, aryle, aralkyle, aralcényle, furannylalkyle, tétrahydrofurannylalkyle, tétrahydropyrannylalkyle, dioxolannylalkyle ou dioxannylalkyle, chacun d'eux éventuellement substitué et

X représente l'oxygène, le soufre ou un groupe $CH_2$,

de leurs sels formés par addition avec des acides, de leurs isomères ou mélanges d'isomères, caractérisé en ce que

(a) on fait réagir des composés hétérocycliques de formule II

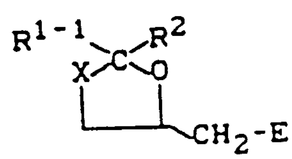

(II)

dans laquelle

$R^{1-1}$ et $R^2$ ont les significations indiquées ci-dessus et

E représente un groupe éliminable accepteur d'électrons, avec des amines de formule III

$H_2N-R^3$ (III)

dans laquelle

$R^3$ a les significations indiquées ci-dessus,

éventuellement en présence d'un diluant et éventuellement en présence d'un accepteur d'acide, ou bien

(b) on hydrogène des composés aminométhylhétérocycliques substitués obtenus par le procédé (a) et répondant à la formule Ia-1

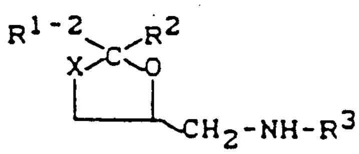

(Ia-1)

dans laquelle

$R^{1-2}$ représente un groupe phényle substitué, un groupe naphtyle éventuellement substitué, un groupe

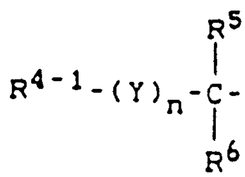

ou

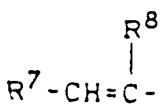

X, $R^2$, $R^3$, $R^5$, $R^6$, $R^7$ et $R^8$ ont les significations indiquées ci-dessus et $R^{4-1}$ représente un groupe phényle éventuellement substitué, en présence d'un catalyseur et le cas échéant en présence d'un diluant.